# EUROPEAN PATENT SPECIFICATION

(11) **EP 3 747 963 B1**
(45) Date of publication and mention of the grant of the patent: **29.05.2024**
(21) Application number: 19748060.1
(22) Date of filing: 31.01.2019
(51) Int. Cl.: C09D 11/328, C09D 11/40, B41J 2/01, C09D 11/32, C09D 11/36, C07D 403/14, C07D 417/12, C07D 417/14, C07D 487/22, C09D 11/38

(54) **INK SET AND INKJET RECORDING METHOD**
TINTENSATZ UND TINTENSTRAHLAUFZEICHNUNGSVERFAHREN
JEU D'ENCRES ET PROCÉDÉ D'ENREGISTREMENT PAR JET D'ENCRE

(30) Priority: 31.01.2018 JP 2018015912
(43) Date of publication of application: 09.12.2020
(73) Proprietor: FUJIFILM Corporation, Tokyo 106-8620 (JP)
(72) Inventor: HAIJIMA, Akimitsu, Ashigarakami-gun, Kanagawa 258-8577 (JP); KONDO, Yoshiaki, Ashigarakami-gun, Kanagawa 258-8577 (JP); NISHIMURA, Mio, Ashigarakami-gun, Kanagawa 258-8577 (JP)
(74) Representative: Klunker IP Patentanwälte PartG mbB
(86) International application number: PCT/JP2019/003421
(87) International publication number: WO 2019/151412

(56) References cited:
- JP-A- 2007 138 124
- JP-A- 2008 101 173
- JP-A- 2009 132 891
- JP-A- 2009 226 642
- JP-A- 2012 072 373
- JP-A- 2012 149 220
- JP-A- 2012 193 329
- JP-A- 2012 193 331
- JP-A- 2016 029 148
- US-A1- 2008 043 079
- US-A1- 2008 145 562
- US-A1- 2010 302 305

## Description

### BACKGROUND OF THE INVENTION

### 1. Field of the Invention

The present disclosure relates to an ink set and an inkjet recording method.

### 2. Description of the Related Art

Conventionally, various methods such as a printing method, an electrophotographic method, and an ink jet method are known as a method for recording an image, and particularly, a recording method using an ink jet method (hereinafter, referred to as an ink jet recording method) is applicable in various fields from the viewpoint that an image can be recorded on various recording media.

In an ink jet recording method, generally, a multicolor image is formed using a plurality of color inks to obtain a desired recorded matter. For recording a multicolor image, three colors of yellow ink, magenta ink, and cyan ink are used and depending on the purpose or case, four colors obtained by adding a black ink to these three color inks may be used.

In addition, for example, depending on the hue of a magenta ink, a plurality of inks having different shades may be used together in the same hue region. In this case, in addition to the above four colors, six colors using light colors, for example, a light cyan ink and a light magenta ink are used. In some cases, seven colors obtained by adding a dark color of a dark yellow ink to these six colors are used.

As described above, an ink set in which two or more kinds of inks are combined is used for ink jet recording.

As a technique using an ink set in which two or more kinds of inks are combined, it is disclosed that an image having good light fastness and ozone resistance can be recorded using an ink set comprising a yellow ink composition, a magenta ink composition, a cyan ink composition, and a black ink composition as inks using dyes as colorants (for example, refer to JP2007-138124A).

Similarly, an ink set comprising a yellow ink composition, a magenta ink composition, a cyan ink composition, and a black ink composition and having excellent ink storage stability is disclosed (for example, refer to JP2012-193330A).

Further, there is disclosed an ink set for ink jet recording including a yellow ink composition, a magenta ink composition, a cyan ink composition, and a black ink composition, in which each ink composition contains a water-soluble organic solvent having a specific structure, and a ratio between the water-soluble organic solvent and water is in a specific range (for example, refer to JP2012-188545A).
US 2010/302305 A1 teaches an ink set capable of forming an image excellent in the light fastness and ozone fastness and reduced in the generation of a bronze phenomenon; an inkjet recording method; and a recorded material. An ink set including at least a yellow ink composition, a magenta ink composition and a cyan ink composition, wherein the yellow ink composition contains, as the yellow colorant, at least one member selected from the group consisting of a compound represented by a specific structure and a salt thereof, each of the yellow colorant, the magenta colorant and the cyan colorant contained in the yellow ink composition, the magenta ink composition and the cyan ink composition, respectively, has at least one ionic hydrophilic group, the counter ion of the ionic hydrophilic group contains a lithium ion, and the lithium ion concentration is 70 mol % or more based on all cations in each ink composition

On the other hand, a recording device that performs recording using an ink jet method comprises a jetting nozzle for jetting an ink composition, and records an image by jetting a desired ink composition from the jetting nozzle according to the image. In a case where the jetting nozzle is clogged with an ink and a jetting failure occurs, the intended ink is not applied, and the reproducibility of an image to be recorded may be impaired. Therefore, for example, when starting the device, a preliminary jetting operation (so-called dummy jetting) may be performed when normally starting the device or the like so as to stably jet the ink from the jetting nozzle.

The dummy-jetted ink composition is stored in an ink disposal unit in the device. In a recording device equipped with a plurality of inks, a plurality of colors of inks are dummy jetted, and thus, the plurality of colors of inks are mixed and stored in the ink disposal unit.

### SUMMARY OF THE INVENTION

As in the above-described related art, the yellow ink composition, the magenta ink composition, the cyan ink composition, and the black ink composition included in the ink set contain a dye, which is an alkali metal salt, in many cases. Among these, lithium salts are widely used as dyes having hues other than a yellow dye since the dye has good solubility and is not easily precipitated. On the other hand, potassium salts are often used than lithium salts since the yellow dye has a relatively high solubility of potassium salts and is not easily precipitated in the ink.

However, in a case where four or more kinds of ink compositions are mixed in dummy jetting as described above, a potassium element included in the dye (for example, a yellow dye) contained in the ink composition is mixed with another dye (for example, a cyan ink composition and a black ink composition), and a lithium element in the other dye (for example, a cyan dye and a black dye) is replaced with the potassium element. As a result, in a case where the ink composition is maintained in a low humidity environment or the like, the viscosity of the ink composition tends to increase.

In a case where the viscosity of the dummy-jetted ink composition increases, the ink composition may be gradually accumulated and piled and come into contact with a recording medium stored and transported in the device. In a case where the recording medium comes into contact with the accumulated ink composition, ink stains are generated in a contact portion of the recording medium and the ink composition, that is, in a side portion that is a back portion or an end surface of the recording medium.

The ink stains impair the quality and appearance of the recorded matter.

JP2007-138124A and JP2012-193330A disclose an ink set using three kinds of inks including a yellow ink composition, a magenta ink composition, and a cyan ink composition or four kinds of ink. However, the present invention focuses on a combination with a dye having a specific structure, and does not focus on the relationship between the kind and amount of the counter ion of the dye and the kind and amount of a solvent used in combination.

Although JP2012-188545A discloses an ink set similar to the ink set in JP2007-138124A and JP2012-193330A, the kind and amount of the counter ion of the dye are not mentioned and the amount of the solvent used in combination is different from the scope of the present disclosure.

The present disclosure has been made in view of the above.

An object to be achieved by one embodiment of the present invention is to provide an ink set in which the generation of ink stains (for example, ink stains on a portion (at least a part of a side portion and a back portion) other than a recording surface) on a recording medium (for example, a sheet of recording paper) is suppressed.

Another object to be achieved by another embodiment of the present invention is to provide an ink jet recording method in which the generation of ink stains (for example, ink stains on a portion (at least a part of a side portion and a back portion) other than a recording surface) on a recording medium (for example, a sheet of recording paper) is suppressed.

Specific units for achieving the objects include the following aspects.
<1> An ink set comprising, at least:
   a yellow ink composition containing at least one compound selected from the group consisting of a compound represented by Formula (Y-1) or (Y-2) below and a salt thereof;
   a magenta ink composition containing at least one compound selected from the group consisting of a compound represented by Formula (M) or (MM) below and a salt thereof;
   a cyan ink composition containing at least one compound selected from the group consisting of a compound represented by Formula (C) below and a salt thereof; and
   a black ink composition containing at least one compound selected from the group consisting of a compound represented by Formula (B) below and a salt thereof,
   in which the yellow ink composition, the magenta ink composition, the cyan ink composition, and the black ink composition each contains, with respect to a total mass of each ink composition, 0.5% by mass to 5.0% by mass of a solvent A containing no nitrogen atom and having a solubility parameter value, i.e. an SP value of 22 MPa^{1/2} to 26 MPa^{1/2}, 0.1% by mass to 5.0% by mass of a solvent B containing a nitrogen atom and having an SP value of 24 MPa^{1/2} to 29 MPa^{1/2}, 10% by mass to 50% by mass of a solvent C containing no nitrogen atom and having an SP value of 29 MPa^{1/2} to 31 MPa^{1/2}, 5% by mass to 30% by mass of a solvent D containing no nitrogen atom and having an SP value of 32 MPa^{1/2} to 34 MPa^{1/2}, and 30% by mass to 60% by mass of water, and
   a ratio of a total mass of lithium with respect to a total mass of alkali metals included in an entire ink provided by mixing each of the yellow ink composition, the magenta ink composition, the cyan ink composition, and the black ink composition at an equal mass ratio is 70% by mass or more.

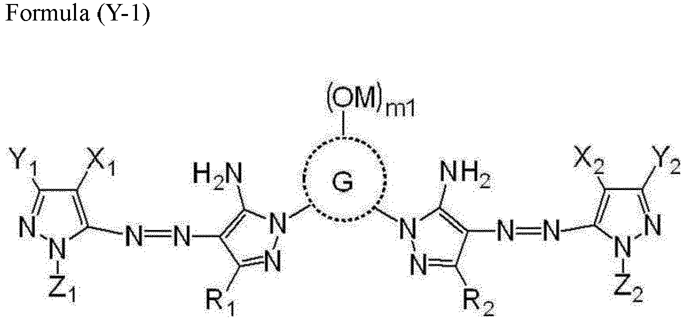

In Formula (Y-1), R₁, R₂, X₁, X₂, Y₁, Y₂, Z₁, and Z₂ each independently represent a hydrogen atom, a halogen atom, an alkyl group, an aryl group, a heterocyclic group, a cyano group, an alkoxy group, an amide group, a ureido group, an alkylsulfonylamino group, an arylsulfonylamino group, a sulfamoyl group, an alkylsulfonyl group, an arylsulfonyl group, a carbamoyl group, or an alkoxycarbonyl group. G represents an atom group forming a 5-membered to 8-membered nitrogen-containing heterocyclic ring, M represents a hydrogen atom or a cation. m1 represents an integer of 0 to 3.

The R₁, R₂, X₁, X₂, Y₁, Y₂, Z₁, and Z₂ may each independently have a linear or branched alkyl group having 1 to 12 carbon atoms, a linear or branched aralkyl group having 7 to 18 carbon atoms, a linear or branched alkenyl group having 2 to 12 carbon atoms, a linear or branched alkynyl group having 2 to 12 carbon atoms, a cycloalkyl group having 3 to 12 carbon atoms, a cycloalkenyl group having 3 to 12 carbon atoms, a halogen atom, an aryl group, a heterocyclic group, cyano group, a hydroxy group, a nitro group, a carboxy group, an amino group, an alkyloxy group, an aryloxy group, an acylamino group, an alkylamino group, an anilino group, a ureido group, a sulfamoylamino group, an alkylthio group, an arylthio group, an alkyloxycarbonylamino group, an alkylsulfonylamino group and an arylsulfonylamino group, a carbamoyl group, a sulfamoyl group, a sulfonyl group, an alkyloxycarbonyl group, a heterocyclic oxy group, an azo group, an acyloxy group, a carbamoyloxy group, a silyloxy group, an aryloxycarbonylamino group, an imide group, a heterocyclic thio group, a sulfinyl group, a phosphonyl group, an aryloxycarbonyl group, an acyl group, or an ionic hydrophilic group as a substituent.

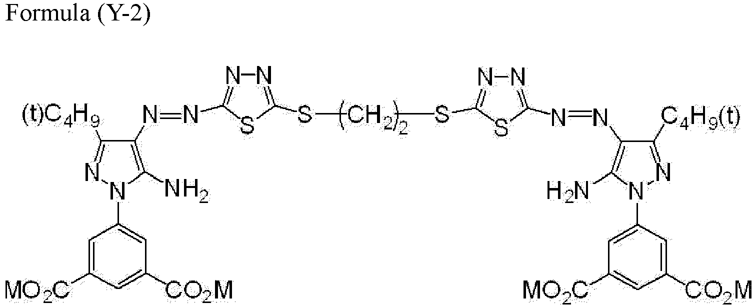

In Formula (Y-2), M's each independently represents a hydrogen atom or a cation.

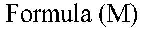

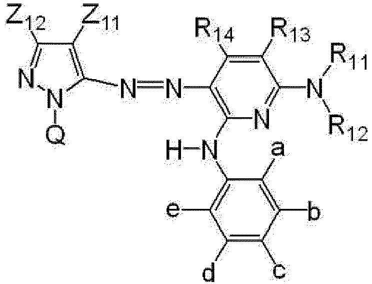

In Formula (M), Z₁₁ represents an electron-withdrawing group having a Hammett's substituent constant σₚ value of 0.2 or more. Z₁₂ represents a hydrogen atom, an alkyl group, an alkenyl group, an alkynyl group, an aralkyl group, an aromatic group, a heterocyclic group, or an acyl group. R₁₁ and R₁₂ each independently represent a hydrogen atom, an alkyl group, a cycloalkyl group, an alkenyl group, an alkynyl group, an aralkyl group, an aryl group, a heterocyclic group, an acyl group, an alkoxycarbonyl group, an aryloxycarbonyl group, a carbamoyl group, an alkylsulfonyl group, an arylsulfonyl group, or a sulfamoyl group. R₁₁ and R₁₂ do not represent a hydrogen atom at the same time. R₁₃, R₁₄, b, c, and d each independently represent a hydrogen atom, a halogen atom, an alkyl group, an alkenyl group, an alkynyl group, an aralkyl group, an aryl group, a heterocyclic group, a cyano group, a carboxy group, a carbamoyl group, an alkoxycarbonyl group, an aryloxycarbonyl group, an acyl group, a hydroxy group, an alkoxy group, an aryloxy group, a silyloxy group, an acyloxy group, a carbamoyloxy group, a heterocyclic oxy group, an alkoxycarbonyloxy group, an aryloxycarbonyloxy group, an amino group substituted with an alkyl group, an aryl group or a heterocyclic group, an acylamino group, a ureido group, a sulfamoylamino group, an alkoxycarbonylamino group, an aryloxycarbonylamino group, an alkylsulfonylamino group, an arylsulfonylamino group, a nitro group, an alkylthio group, an arylthio group, an alkylsulfonyl group, an arylsulfonyl group, an alkylsulfinyl group, an arylsulfinyl group, a sulfamoyl group, a heterocyclic thio group, or an ionic hydrophilic group. R₁₃ and R₁₁, or R₁₁ and R₁₂ may be bonded to each other to form a 5-membered or 6-membered ring. a and e each independently represent an alkyl group, an alkoxy group, or a halogen atom, and in a case where a and e both represent an alkyl group, a total carbon number of the alkyl group is 3 or more. a and b, or e and d may be bonded to each other to form a ring. Q represents a hydrogen atom, an alkyl group, an alkenyl group, an alkynyl group, an aralkyl group, an aromatic group, or a heterocyclic group. However, Formula (M) has at least one ionic hydrophilic group.

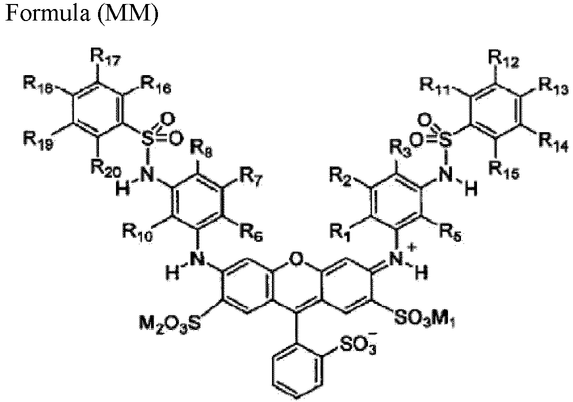

In Formula (MM), R₁, R₅, R₆, and R₁₀ each independently represent an alkyl group. R₂, R₃, R₇, R₈, R₁₁, R₁₂, R₁₃, R₁₄, R₁₅, R₁₆, R₁₇, R₁₈, R₁₉, and R₂₀ each independently represent a hydrogen atom or a substituent. M₁ and M₂ each independently represent a hydrogen atom, an alkali metal ion, or an ammonium ion.

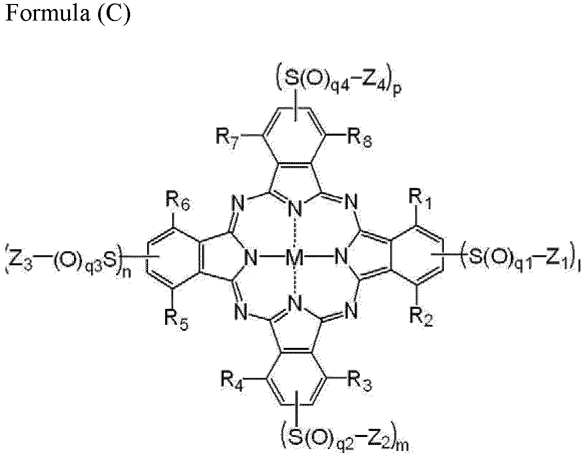

In Formula (C), R₁, R₂, R₃, R₄, R₅, R₆, R₇, and R₈ each independently represent a hydrogen atom, a halogen atom, an alkyl group, a cycloalkyl group, an alkenyl group, an aralkyl group, an aryl group, a heterocyclic group, a cyano group, a hydroxy group, a nitro group, an amino group, an alkylamino group, an alkoxy group, an aryloxy group, an amide group, an arylamino group, a ureido group, a sulfamoylamino group, an alkylthio group, an arylthio group, an alkoxycarbonylamino group, a sulfonamide group, a carbamoyl group, a sulfamoyl group, a sulfinyl group, a sulfonyl group, an alkoxycarbonyl group, a heterocyclic oxy group, an azo group, an acyloxy group, a carbamoyloxy group, a silyloxy group, an aryloxycarbonyl group, an aryloxycarbonylamino group, an imide group, a heterocyclic thio group, a phosphoryl group, an acyl group, or an ionic hydrophilic group. Z₁, Z₂, Z₃, and Z₄ each independently represent an alkyl group, a cycloalkyl group, an alkenyl group, an alkynyl group, an aralkyl group, an aryl group, or a heterocyclic group, and at least one of Z₁, Z₂, Z₃, or Z₄ has an ionic hydrophilic group as a substituent. l, m, n, p, q1, q2, q3, and q4 each independently represent an integer of 1 or 2. M represents a metal atom or an oxide, hydroxide, or halide of a metal.

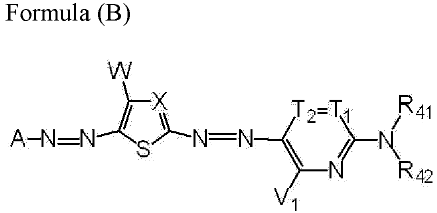

In Formula (B), A represents an aromatic group or a heterocyclic group. X represents a nitrogen atom or =C(W₁)-, and W₁ represents an electron-withdrawing group having a Hammett's substituent constant σₚ value of 0.20 or more. T₁ and T₂ each represent =C(R₄₃)- and -C(R₄₄)=, or one of T₁ and T₂ represents a nitrogen atom, and the other represents =C(R₄₃)- or -C(R₄₄)=. V₁, W, R₄₃, and R₄₄ each independently represent a hydrogen atom, a halogen atom, an alkyl group, an alkenyl group, an alkynyl group, an aralkyl group, an aryl group, a heterocyclic group, a cyano group, a carboxy group, a carbamoyl group, or an alkoxycarbonyl group, an aryloxycarbonyl group, a heterocyclic oxycarbonyl group, an acyl group, a hydroxy group, an alkoxy group, an aryloxy group, a heterocyclic oxy group, a silyloxy group, an acyloxy group, a carbamoyloxy group, an alkoxycarbonyloxy group, an aryloxycarbonyloxy group, an amino group (including an alkylamino group, an arylamino group, and a heterocyclic amino group), an acylamino group, a ureido group, a sulfamoylamino group, an alkoxycarbonylamino group, an aryloxycarbonylamino group, an alkylsulfonylamino group, an arylsulfonylamino group, a heterocyclic sulfonylamino group, a nitro group, an alkylarylthio group, an arylthio group, a heterocyclic thio group, an alkylsulfonyl group, an arylsulfonyl group, a heterocyclic sulfonyl group, an alkylsulfinyl group, an arylsulfinyl group, a heterocyclic sulfinyl group, a sulfamoyl group, or a sulfo group. R₄₁ and R₄₂ each independently represent a hydrogen atom, an alkyl group, an alkenyl group, an alkynyl group, an aralkyl group, an aryl group, a heterocyclic group, an acyl group, an alkoxycarbonyl group, an aryloxycarbonyl group, a carbamoyl group, an alkylsulfonyl group, an arylsulfonyl group, or a sulfamoyl group, and R₄₁ and R₄₂ do not represent a hydrogen atom at the same time. R₄₃ and R₄₁, or R₄₁ and R₄₂ may be bonded to each other to form a 5-membered or 6-membered ring.
<2> The ink set according to <1>, in which the ratio of the total mass of lithium with respect to the total mass of alkali metals included in the entire ink provided by mixing each of the yellow ink composition, the magenta ink composition, the cyan ink composition, and the black ink composition at an equal mass ratio is 80% by mass or more.
<3> The ink set according to <1> or <2>, in which the ratio of the total mass of lithium with respect to the total mass of alkali metals included in the entire ink provided by mixing each of the yellow ink composition, the magenta ink composition, the cyan ink composition, and the black ink composition at an equal mass ratio is 85% by mass or more.
<4> The ink set according to any one of <1> to <3>, in which the ratio of the mass of lithium with respect to the total mass of alkali metals in each of the yellow ink composition, the magenta ink composition, the cyan ink composition, and the black ink composition is 70% by mass or more.
<5> The ink set according to any one of <1> to <4>, in which the black ink composition further contains a compound represented by Formula (BA) below.

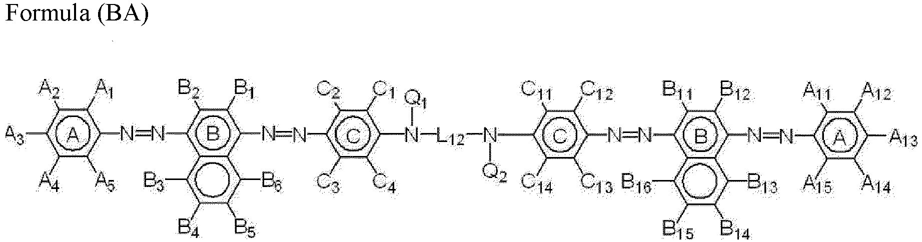

In Formula (BA), ring A, ring B, and ring C each independently represent an aryl group or a heterocyclic group. A₁, A₂, A₃, A₄, A₅, A₁₁, A₁₂, A₁₃, A₁₄, A₁₅, B₁, B₂, B₃, B₄, B₅, B₆, B₁₁, B₁₂, B₁₃, B₁₄, B₁₅, B₁₆, C₁, C₂, C₃, C₄, C₁₁, C₁₂, C₁₃, and C₁₄ each independently represent a hydrogen atom or a substituent. Q₁ and Q₂ each independently represent a hydrogen atom or a substituent. L₁₂ represents a divalent linking group. However, at least one of A₁ to A₅, A₁₁ to A₁₅, B₁ to B₆, B₁₁ to B₁₆, C₁ to C₄, C₁₁ to C₁₄, Q₁, Q₂, or L₁₂ has at least one ionic hydrophilic group.
<6> The ink set according to any one of <1> to <5>, in which the solvent A is 2-ethyl-1,3-hexanediol, the solvent B is 2-pyrrolidone, the solvent C is 1,3-butanediol, and the solvent D is glycerin.
<7> The ink set according to any one of <1> to <6>, in which a content of a surfactant in each of the yellow ink composition, the magenta ink composition, the cyan ink composition, and the black ink composition is less than 0.1% by mass with respect to the total mass of each ink composition.
<8> An ink jet recording method for recording an image using the ink set according to any one of <1> to <7>.

According to an embodiment of the present invention, there is provided an ink set in which the generation of ink stains (for example, ink stains on a portion (at least a part of a side portion and a back portion) other than a recording surface) on a recording medium (for example, a sheet of recording paper) is suppressed.

According to another embodiment of the present invention, there is provided an ink jet recording method in which the generation of ink stains (for example, ink stains on a portion (at least a part of a side portion and a back portion) other than a recording surface) on a recording medium (for example, a sheet of recording paper) is suppressed.

### DESCRIPTION OF THE PREFERRED EMBODIMENTS

Hereinafter, an ink set and an ink jet recording method using the ink set according to the present disclosure will be described in detail.

In this specification, the "back portion" refers to a surface of a recording medium on the opposite side (back side) to the front side on which recording is performed, and the "side portion" refers to an end portion that connects the front side surface and the back side surface and has an area corresponding to the thickness of the recording medium.

In the present specification, a numerical range indicated using "to" indicates a range including numerical values given before and after "to" as a minimum value and a maximum value, respectively. In the numerical ranges described stepwise in the present disclosure, the upper limit or the lower limit described in a certain numerical range may be replaced with the upper limit or the lower limit of another numerical range described in a stepwise manner. In addition, in the numerical ranges described in the present disclosure, the upper limit or the lower limit described in a certain numerical range may be replaced with the value shown in the embodiment.

In the present specification, "% by mass" and "% by weight" have the same meaning, and "parts by mass" and "parts by weight" have the same meaning. In the present specification, a combination of two or more preferable embodiments is a more preferable embodiment.

In the present specification, regarding the expression of groups in compounds represented by formulae, in a case where there are no descriptions of whether or not the groups are substituted or not substituted and in a case where the groups are capable of further having a substituent, unless otherwise specified, the groups may be not only unsubstituted groups but also groups having a substituent. For example, in a formula, in a case where there is a description that "R represents an alkyl group, an aryl group, or a heterocyclic group", it means that "R represents an unsubstituted alkyl group, a substituted alkyl group, an unsubstituted aryl group, a substituted aryl group, an unsubstituted heterocyclic group, or a substituted heterocyclic group".

### <Ink Set>

An ink set of the present disclosure includes ink compositions of at least the following four kinds of hues, and may further include ink compositions of other hues other than the following four kinds as necessary.

In the present specification, the "ink composition" is also simply referred to as "ink".

### (1) Yellow Ink Composition

Ink composition containing at least one compound selected from the group consisting of a compound represented by Formula (Y-1) or (Y-2) and a salt thereof

### (2) Magenta ink composition

Ink composition containing at least one compound selected from the group consisting of a compound represented by Formula (M) or Formula (MM) below and a salt thereof

### (3) Cyan ink composition

Ink composition containing at least one compound selected from the group consisting of a compound represented by Formula (C) and a salt thereof

### (4) Black ink composition

Ink composition containing at least one compound selected from the group consisting of a compound represented by Formula (B) and a salt thereof

The ink compositions in the ink set of the present disclosure are not limited to a case where each ink composition indicates only a single color, and may include a plurality of colors in a range of each hue system depending on the shade, hue, or the like. For example, in a case of a magenta ink composition, the ink set may include only a single color magenta ink composition, or may include, for example, two colors of a light magenta ink composition and a dark magenta ink composition. In addition, in a case of the magenta ink composition, for example, a composition including two colors of a magenta ink composition having a hue close to red and a magenta ink composition having a hue close to pink is also included.

In the ink set according to the present disclosure, the yellow ink composition, the magenta ink composition, the cyan ink composition, and the black ink composition each further contain, in addition to the colorants, a solvent A containing no nitrogen atom and having an SP value of 22 MPa^{1/2} to 26 MPa^{1/2} (hereinafter, also referred to as a solvent A) in an amount of 0.5% by mass to 5.0% by mass with respect to a total mass of each ink composition, a solvent B containing a nitrogen atom and having an SP value of 24 MPa^{1/2} to 29 MPa^{1/2} (hereinafter, also referred to as a solvent B) in an amount of 0.1% by mass to 5.0% by mass with respect to the total mass of each ink composition, a solvent C containing no nitrogen atom and having an SP value of 29 MPa^{1/2} to 31 MPa^{1/2} (hereinafter, also referred to as a solvent C) in an amount of 10% by mass to 50% by mass with respect to the total mass of each ink composition, a solvent D containing no nitrogen atom and having an SP value of 32 MPa^{1/2} to 34 MPa^{1/2} (hereinafter, also referred to as a solvent D) in an amount of 5% by mass to 30% by mass with respect to the total mass of each ink composition, and water in an amount of 30% by mass to 60% by mass with respect to the total mass of each ink composition, and
a ratio of a total mass of lithium with respect to a total mass of alkali metals included in the entire ink provided by mixing each of the yellow ink composition, the magenta ink composition, the cyan ink composition, and the black ink composition at an equal mass ratio is 70% by mass or more.

Conventionally, a technique using an ink set in which two or more kinds of inks are combined has been used, and recording a multicolor image by an ink jet method has been widely performed. In a case where an image is recorded by an ink jet method, an image is recorded by jetting an ink composition from a jetting nozzle provided for each color. However, in a case where there is a period in which an ink is not jetted from the jetting nozzle or the like, the ink may be increased in viscosity or solidified in the distal end portion or inside thereof to cause ink clogging. In a case where there is a jetting nozzle from which the ink composition is not jetted, the quality of the recorded image is impaired, and a preliminary jetting operation (so-called dummy jetting) is usually performed at the time of starting the device. The preliminarily jetted ink composition is not used for recording an image, and thus the jetted ink composition is stored in a disposal unit. In the disposal unit, the ink compositions of a plurality of colors are mixed.

Generally, while a lithium salt is widely used as a dye included in an ink composition (for example, a dye contained in an ink composition having a hue other than yellow), due to the fact that depending on cases, the solubility of a potassium salt is as high as the solubility of a lithium salt, and precipitation in an ink does not easily occur, a potassium salt may be used as a dye (for example, a yellow dye). In a case where a plurality of ink compositions are mixed in the disposal unit (for example, a yellow ink composition and an ink composition having a hue other than yellow are mixed), a potassium element included in a dye (for example, a yellow dye) is replaced with a lithium element in another dye other than a potassium salt (for example, a dye having a hue other than yellow (for example, a cyan dye and a black dye)) to cause an increase in viscosity of the ink composition. In a case where the viscosity of the dummy-jetted ink composition increases, the ink composition may be gradually accumulated and piled in the device and come into contact with a recording medium stored and transported in the device. In a case where the recording medium comes into contact with the accumulated ink composition, even in a case where the ink composition does not come into contact with a recording surface, the ink is attached to a contact portion of the recording medium other than the recording surface and the ink composition, that is, the side portion or back portion corresponding to the thickness in the recording medium, and ink stains are generated. The ink stains impair the quality and appearance of the recorded matter.

In view of the above description, according to the present disclosure, a composition in which each of a yellow ink composition, a magenta ink composition, a cyan ink composition, and a black ink composition contains a compound having a structure suitable for each color as a colorant, a ratio of the total mass of lithium with respect to the total mass of alkali metals is 70% by mass or more, and further, four kinds of solvents having different SP values are contained in combination at a specific ratio is provided. Accordingly, for example, it is possible to suppress the generation of ink stains on a portion of a sheet recording medium (at least a part of the side portion and the back portion) other than the recording surface.

The ink set according to the present disclosure includes in a case where four colors of a yellow ink composition, a magenta ink composition, a cyan ink composition, and a black ink composition, and the four colors of ink compositions are mixed at an equal mass ratio, a ratio (M^{Li}/M^{I}) of the total mass of lithium (M^{Li}) with respect to the total mass of alkali metals (M^{I}) included in the entire ink of the ink set is 70% by mass or more.

The fact that the ratio represented by M^{Li}/M^{I} is 70% by mass or more means that the amount (content ratio) of the total lithium metal contained in the yellow ink composition, the magenta ink composition, the cyan ink composition, and the black ink composition is large and the viscosity increase phenomenon of the ink composition, which tends to occur due to the incorporation of an alkali metal (for example, potassium) other than lithium, does not easily occur.

In the above description, the ratio of the total mass of lithium with respect to the total mass of alkali metals included in the entire ink of the ink set (the ratio represented by M^{Li}/M^{I}) is preferably 80% by mass or more and more preferably 85% by mass or more for the same reason as described above.

Next, components included in each ink composition included in the ink set according to the present disclosure will be described. In the following description, components included in the yellow ink composition will be described first, and then components included in the magenta ink composition, the cyan ink composition, and the black ink composition will be described.

Here, among the components included in the magenta ink composition, the cyan ink composition, and the black ink composition, the detailed description of the same components as those included in the yellow ink composition will be omitted.

### (1) Yellow Ink Composition

The yellow ink composition in the present disclosure contains, as a colorant, at least one compound selected from the group consisting of a compound represented by Formula (Y-1) or (Y-2) and a salt thereof, a solvent A, a solvent B, a solvent C, a solvent D, and water, and may further contain other components such as a surfactant and an additive as necessary.

### -Solvent-

First, the solvents (solvents A to D) included in the yellow ink composition will be described.

Hereinafter, the numerical value in parentheses following the compound name indicates the SP value (unit: MPa^{1/2}) of the solvent, and the unit is omitted. In addition, the SP value in parentheses indicates a value obtained by rounding off the first decimal point.

The SP value (solubility parameter/unit: MPa^{1/2}) of a solvent in the present disclosure is a value expressed as the square root of molecular cohesion energy, and can be calculated by the method described in R. F. Fedors, Polymer Engineering Science, 14, pp. 147 to 154 (1974), and this numerical value is adopted in the present disclosure.

### [Solvent A Containing No Nitrogen Atom and Having SP Value of 22 MPa^{1/2} to 26 MPa^{1/2}]

The yellow ink composition contains a solvent A containing no nitrogen atom and having an SP value of 22 MPa^{1/2} to 26 MPa^{1/2}, and the content of the solvent A is in a range of 0.5% by mass to 5.0% by mass with respect to the total mass of the yellow ink composition.

By containing the solvent A, the surface tension of the ink can be reduced, and the jettability of the ink can be improved. From such a viewpoint, the content of the solvent A is set to a range of 0.5% by mass or more. However, in a case where the content of the solvent A is excessively large, the dye is easily precipitated, and thus, the content of the solvent A is set to 5.0% by mass or less. The content of the solvent A is preferably in a range of 1.0% by mass to 3.0% by mass with respect to the total mass of the yellow ink composition.

In a case where the SP value of the solvent A is 22 MPa^{1/2} or more, the solubility of the dye is easily maintained satisfactorily, and the separation of the dye or the precipitation of the dye in a low temperature environment does not easily occur. In addition, in a case where the SP value is 26 MPa^{1/2} or less, the effect of reducing the surface tension of the ink is high, and the jettability is improved.

The SP value of the solvent A is preferably 24 MPa^{1/2} to 25 MPa^{1/2} for the same reason as described above.

Specific examples of the solvent A include 2-ethyl-1,3-hexanediol (25), polyethylene glycol 200 (26), polypropylene glycol 200 (25), GP250 (22), diethylene glycol monobutyl ether (22), triethylene glycol monomethyl ether (22), triethylene glycol monoethyl ether (22), 2-ethyl-2-butyl-1,3-propanediol (25), and 1,2-hexanediol (24).

Among these, 2-ethyl-1,3-hexanediol (25 MPa^{1/2}) is more preferable.

In a case where the content of the solvent A is 0.5% by mass or more, the effect of reducing the surface tension of the ink is high, and the jettability is improved. In a case where the content of the solvent A is 5.0% by mass or less, the solubility of the dye is easily maintained satisfactorily, and the precipitation of the dye is effectively suppressed.

The content of the solvent A is preferably 1.0% by mass to 3.0% by mass with respect to the total mass of the yellow ink composition.

### [Solvent B Containing Nitrogen Atom and Having SP Value of 24 MPa^{1/2} to 29 MPa^{1/2}]

The yellow ink composition contains a solvent B containing a nitrogen atom and having an SP value of 24 MPa^{1/2} to 29 MPa^{1/2}, and the content of the solvent B is in a range of 0.1% by mass to 5.0% by mass with respect to the total mass of the yellow ink composition.

In a case where the solvent B is contained, there is an effect that the jetting stability is improved. From such a viewpoint, the content of the solvent B is in a range of 0.1% by mass or more.

In a case where the SP value of the solvent B is 24 MPa^{1/2} or more, the precipitation of the dye is effectively suppressed. In addition in a case where the SP value of the solvent B is 29 MPa^{1/2} or less, the jetting stability of the ink is satisfactory.

The SP value of the solvent B is preferably 25 MPa^{1/2} to 26 MPa^{1/2} for the same reason as described above.

Specific examples of the solvent B include 2-pyrrolidone (26), N-methyl-pyrrolidone (24), N-hydroxyethyl-pyrrolidone (29), 2-amino-2-ethyl-1,3-propanediol (28), ethylene urea (29), a compound (25) represented by Structural Formula (1)-1 below, and a compound (24) represented by Structural Formula (1)-2 below.

Among these, 2-pyrrolidone is more referable.

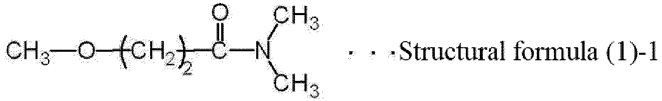

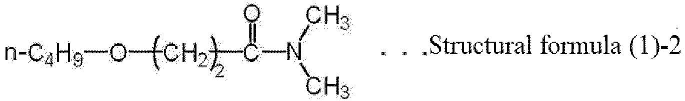

The content of the solvent B is in a range of 0.1% by mass to 5.0% by mass with respect to the total mass of the yellow ink composition. A case where the content of the solvent B is 5.0% by mass or less is advantageous in that the solubility of the dye and the compatibility with other solvents are enhanced.

The content of the solvent B is preferably 0.3% by mass to 3.0% by mass, more preferably 0.5% by mass to 2.0% by mass with respect to the total mass of the yellow ink composition for the same reason as described above.

### [Solvent C Containing No Nitrogen Atom and Having SP Value of 29 MPa^{1/2} to 31 MPa^{1/2}]

The yellow ink composition contains a solvent C containing no nitrogen atom and having an SP value of 29 MPa^{1/2} to 31 MPa^{1/2}, and the content of the solvent C is 10% by mass to 50% by mass with respect to the total mass of the yellow ink composition.

The solvent C increases the solubility of the dye most among the solvents A to D. Therefore, in a case where the solvent C is contained, the effect of preventing the precipitation of the dye is high. In addition, the solvent C functions as a dissolution aid for a solvent having an SP value of less than 29 MPa^{1/2}, and particularly suppresses separation and precipitation of "the solvent A containing no nitrogen atom and having an SP value of 22 MPa^{1/2} to 26 MPa^{1/2}" in the ink composition. From such a viewpoint, the content of the solvent C is in a range of 10% by mass or more.

In a case where the SP value of the solvent C is 29 MPa^{1/2} or more, the function as a solvent aid is strong, and the effect of improving the solubility of the dye is high. In addition, a case where the SP value is 31 MPa^{1/2} or less is advantageous in terms of the solubility of the solvent C itself.

The SP value of the solvent C is more preferably in a range of 30 MPa^{1/2} to 31 MPa^{1/2} for the same reason as described above.

Specific examples of the solvent C include 1,3-butanediol (30), diethylene glycol (31), 1,5-pentanediol (29).

Among these, 1,3-butanediol is more preferable.

In a case where the content of the solvent C is 10% by mass or more, the solubility of the dye can be effectively increased. In a case where the content of the solvent C is 50% by mass or less, a phase separation or the like does not occur in the ink, and this case is advantageous in terms of liquid stability.

The content of the solvent C is preferably 15% by mass to 45% by mass and more preferably 20% by mass to 35% by mass with respect to the total mass of the yellow ink composition for the same reason as described above.

### [Solvent D Containing No Nitrogen Atom and Having SP Value of 32 MPa^{1/2} to 34 MPa^{1/2}]

The yellow ink composition contains a solvent D containing no nitrogen atom and having a SP value of 32 MPa^{1/2} to 34 MPa^{1/2}, and the content of the solvent D is 5% by mass to 30% by mass with respect to the total mass of the yellow ink composition.

The solvent D most enhances the moisturizing properties of the ink composition among the solvents A to D. Therefore, by containing the solvent D, the ink composition is prevented from being dried at a jetting port of an ink jet head and at a place where the dummy-jetted and discarded ink composition is stored. Thus, the precipitation of the dye in the ink composition is suppressed, and the jetting stability and ink stains on an output recording medium can be prevented. From such a viewpoint, the content of the solvent D is in a range of 5% by mass or more.

In a case where the SP value of the solvent D is 32 MPa^{1/2} or more, the moisturizing effect of the ink composition is high, and the drying of the ink composition is suppressed. As a result, the precipitation of the dye in the ink composition is suppressed. Further, a case where the SP value is 34 MPa^{1/2} or less is advantageous in that the solubility in water is high and the compatibility with the solvents A, B, and C is high.

The SP value of the solvent D is more preferably in a range of 33 MPa^{1/2} to 34 MPa^{1/2} for the same reason as described above.

Specific examples of the solvent C include glycerin (34), propylene glycol (33), 1,3-propanediol (33), and diglycerin (32).

Among these, glycerin is more preferable.

In a case where the content of the solvent D is 5% by mass or more, the effect of preventing the drying of the ink composition is excellent, and the precipitation of the dye in the ink composition is suppressed. In a case where the content of the solvent D is 30% by mass or less, a phase separation or the like does not occur in the ink, and this case is advantageous in terms of liquid stability.

The content of the solvent D is preferably 8% by mass to 28% by mass and more preferably 10% by mass to 25% by mass with respect to the total mass of the yellow ink composition for the same reason as described above.

As preferable solvents in the yellow ink composition, from the viewpoint that the effect by the ink set according to the present disclosure is more effectively exhibited, it is preferable that the solvent A is a solvent containing no nitrogen atom, having an SP value of 24 MPa^{1/2} to 25 MPa^{1/2}, and contained at a content of 1.0% by mass to 3.0% by mass with respect to the total mass of the yellow ink composition, the solvent B is a solvent containing a nitrogen atom, having an SP value of 25 MPa^{1/2} to 26 MPa^{1/2}, and contained at a content of 0.5% by mass to 2.0% by mass with respect to the total mass of the yellow ink composition, the solvent C is a solvent containing no nitrogen atom, having an SP value of 30 MPa^{1/2} to 31 MPa^{1/2}, and contained at a content of 20% by mass to 30% by mass with respect to the total mass of the yellow ink composition, and the solvent D is a solvent containing no nitrogen atom, having an SP value of 33 MPa^{1/2} to 34 MPa^{1/2}, and contained at a content of 10% by mass to 25% by mass with respect to the total mass of the yellow ink composition.

Among these, as preferable solvents in the yellow ink composition, from the same viewpoint as above, it is preferable that the solvent A is 2-ethyl-1,3-hexanediol, the solvent B is 2-pyrrolidone, the solvent C is 1,3-butanediol, and the solvent D is glycerin.

In addition, the yellow ink composition of the present disclosure may further contain a solvent other than the solvents A to D as long as the effect of the ink set of the present disclosure is not significantly impaired.

The details and preferable aspects of the solvent A, the solvent B, the solvent C, and the solvent D are the same not only in the yellow ink composition but also in the magenta ink composition, the cyan ink composition, and the black ink composition described later.

In the yellow ink composition of the present disclosure, the magenta ink composition, the cyan ink composition, and the black ink composition described later, the content of water is 30% by mass to 60% by mass, and preferably 40% by mass to 55% by mass. In a case where the content of water is 30% by mass or more, the water-soluble dye can be stably dissolved. In a case where the content of water is 60% by mass or less, the compatibility with the solvent A, the solvent B, the solvent C, and the solvent D can be satisfied.

### -Colorant (Dye)-

Next, the colorant contained in the yellow ink composition will be described.

The yellow ink composition in the present disclosure contains at least one compound selected from the group consisting of a compound represented by Formula (Y-1) or (Y-2) and a salt thereof (also referred to as a yellow dye).

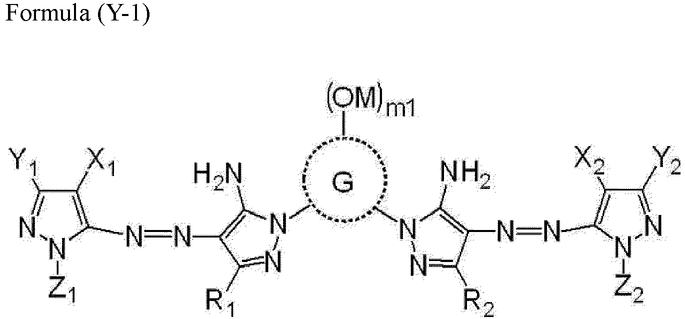

For the details of Formula (Y-1), the description of paragraphs 0235 to 0257 and paragraphs 0160 to 0163 of JP2007-138124A can be referred to. However, none of the documents cited herein is incorporated by reference.

In Formula (Y-1), R₁, R₂, X₁, X₂, Y₁, Y₂, Z₁, and Z₂ each independently represent a hydrogen atom, a halogen atom, an alkyl group, an aryl group, a heterocyclic group, a cyano group, an alkoxy group, an amide group, a ureido group, an alkylsulfonylamino group, an arylsulfonylamino group, a sulfamoyl group, an alkylsulfonyl group, an arylsulfonyl group, a carbamoyl group, or an alkoxycarbonyl group.

R₁, R₂, X₁, X₂, Y₁, Y₂, Z₁, and Z₂ may be unsubstituted or substituted with a substituent. The substituent will be described later.

As R₁, R₂, X₁, X₂, Y₁, Y₂, Z₁, and Z₂, a hydrogen atom, a halogen atom, an alkyl group, an aryl group, a cyano group, an alkylsulfonyl group, an arylsulfonyl group, or a heterocyclic group is preferable, and a hydrogen atom, an alkyl group, an aryl group, a cyano group, or an alkylsulfonyl group is most preferable.

Among these, as R₁ and R₂, a substituted or unsubstituted alkyl group having 1 to 12 carbon atoms in total, a substituted or unsubstituted aryl group having 6 to 18 carbon atoms in total, or a substituted or unsubstituted heterocyclic group having 4 to 12 carbon atoms in total is preferable, a linear alkyl group or a branched alkyl group having 1 to 8 carbon atoms in total is more preferable, a secondary or tertiary alkyl group is particularly preferable, and a t-butyl group is most preferable.

In addition, as X₁ and X₂, a cyano group, an alkylsulfonyl group having 1 to 12 carbon atoms, an arylsulfonyl group having 6 to 18 carbon atoms, or a sulfamoyl group having 0 to 12 carbon atoms is preferable, a cyano group, an alkylsulfonyl group having 1 to 12 carbon atoms, or a sulfamoyl group having 0 to 12 carbon atoms is more preferable, and a cyano group or an alkylsulfonyl group having 1 to 12 carbon atoms is even more preferable.

As Y₁ and Y₂, a hydrogen atom, a substituted or unsubstituted alkyl group having 1 to 12 carbon atoms in total, a substituted or unsubstituted aryl group having 6 to 18 carbon atoms in total, or a substituted or unsubstituted heterocyclic group having 4 to 12 carbon atoms in total is preferable. Among these, a hydrogen atom, or a linear alkyl group and/or a branched alkyl group having 1 to 8 carbon atoms in total is preferable, a hydrogen atom or an alkyl group having 1 to 8 is more preferable, and a hydrogen atom is most preferable.

As Z₁ and Z₂, a hydrogen atom, a substituted or unsubstituted alkyl group, a substituted or unsubstituted cycloalkyl group, a substituted or unsubstituted alkenyl group, a substituted or unsubstituted alkynyl group, a substituted or unsubstituted aralkyl group, a substituted or unsubstituted aryl group, or a substituted or unsubstituted heterocyclic group is preferable, a substituted aryl group, or a substituted heterocyclic group is more preferable, and a substituted aryl group is even more preferable.

In addition, R₁, R₂, X₁, X₂, Y₁, Y₂, Z₁, and Z₂ each independently preferably have a linear or branched alkyl group having 1 to 12 carbon atoms, a linear or branched aralkyl group having 7 to 18 carbon atoms, a linear or branched alkenyl group having 2 to 12 carbon atoms, a linear or branched alkynyl group having 2 to 12 carbon atoms, a cycloalkyl group having 3 to 12 carbon atoms, a cycloalkenyl group having 3 to 12 carbon atoms (each of the above groups has a branched chain) as a substituent for the reason that the stability of ink is improved, and a case of have a group having an asymmetric carbon is particularly preferable. Examples of the substituents that R₁, R₂, X₁, X₂, Y₁, Y₂, Z₁, and Z₂ may have include methyl, ethyl, propyl, isopropyl, sec-butyl, t-butyl, 2-ethylhexyl, 2-methylsulfonylethyl, 3-phenoxypropyl, trifluoromethyl, cyclopentyl, a halogen atom (for example, a chlorine atom, and a bromine atom), an aryl group (for example, phenyl, 4-t-butylphenyl, 2,4-di-t-amylphenyl, hetero 2-pyrimidinyl, 2-benzothiazolyl), a cyano group, a hydroxy group, a nitro group, a carboxy group, an amino group, an alkyloxy group (for example, methoxy, ethoxy, 2-methoxyethoxy, and 2-methylsulfonylethoxy), an aryloxy group (for example, phenoxy, 2-methylphenoxy, 4-t-butylphenoxy, 3-nitrophenoxy, 3-t-butyloxycarbamoylphenoxy, and 3-methoxycarbonylphenyloxy), an acylamino group (for example, acetamido, benzamido, and 4-(3-t-butyl-4-hydroxyphenoxy)butanamide), an alkylamino group (for example, methylamino, butylamino, diethylamino, and methylbutylamino), an anilino group (for example, phenylamino, and 2-chloroanilino), a ureido group (for example, phenylureido, methylureide, N,N-dibutylureido), a sulfamoylamino group (for example, N,N-dipropylsulfamoylamino), an alkylthio group (for example, methylthio, octylthio, and 2-phenoxyethylthio), an arylthio group (for example, phenylthio, 2-butoxy-5-t-octylphenylthio, and 2-carboxyphenylthio), an alkyloxycarbonylamino group (for example, methoxycarbonylamino), an alkylsulfonylamino group and an arylsulfonylamino group (for example, methylsulfonylamino, phenylsulfonylamino, and p-toluenesulfonylamino), a carbamoyl group (for example, N-ethylcarbamoyl, and N,N-dibutylcarbamoyl), a sulfamoyl group (for example, N-ethylsulfamoyl, N,N-dipropylsulfamoyl, and N-phenylsulfamoyl), a sulfonyl group (for example, methylsulfonyl, octylsulfonyl, phenylsulfonyl, and p-toluenesulfonyl), an alkyloxycarbonyl group (for example, methoxycarbonyl, and butyloxycarbonyl), a heterocyclic oxy group (for example, 1-phenyltetrazole-5-oxy, and 2-tetrahydropyranyloxy), an azo group (for example, phenylazo, 4-methoxyphenylazo, 4-pivaloylaminophenylazo, and 2-hydroxy-4-propanoylphenylazo), an acyloxy group (for example, acetoxy), a carbamoyloxy group (for example, N-methylcarbamoyloxy, and N-phenylcarbamoyloxy), a silyloxy group (for example, trimethylsilyloxy, and dibutylmethylsilyloxy), aryloxycarbonylamino groups (for example, phenoxycarbonylamino), an imide group (for example, N-succinimido, and N-phthalimido), a heterocyclic thio group (for example, 2-benzothiazolylthio, 2,4-di-phenoxy-1,3,5-triazole-6-thio, and 2-pyridylthio), a sulfinyl group (for example, 3-phenoxypropylsulfinyl), a phosphonyl group (for example, phenoxyphosphonyl, octyloxyphosphonyl, and phenylphosphonyl), an aryloxycarbonyl group (for example, phenoxycarbonyl), an acyl group (for example, acetyl, 3-phenylpropanoyl, and benzoyl), and an ionic hydrophilic group (for example, a carboxylate, a sulfonic acid or a sulfonate, a phosphonic acid or a phosphonate, or a quaternary ammonium group).

G represents an atomic group forming a 5-membered to 8-membered nitrogen-containing heterocyclic ring.

Preferable examples of the 5-membered to 8-membered nitrogen-containing heterocyclic ring in G include an S-triazine ring, a pyrimidine ring, a pyridazine ring, a pyrazine ring, a pyridine ring, an imidazole ring, a pyrazole ring, and a pyrrole ring. Among these, an S-triazine ring, a pyrimidine ring, a pyridazine ring or a pyrazine ring is more preferable, and an S-triazine ring is even more preferable.

M represents a hydrogen atom or a cation.

The cation in M is preferably an alkali metal ion, an ammonium ion (NH₄⁺), or a quaternary ammonium ion (NR₄⁺), and more preferably a Li⁺ ion, a Na⁺ ion, a K⁺ ion, a NH₄⁺ ion, or a NR₄⁺ ion. Here, R represents an alkyl group or an aryl group, and is the same as the examples of the alkyl group and the aryl group represented by R₁ and the like and Y₁ and the like. Among these, M is preferably a Li⁺ ion, a Na⁺ ion, a K⁺ ion, or a NH₄⁺ ion, more preferably a Li⁺ ion, a Na⁺ ion, or K⁺ ion, and most preferably a Li⁺ ion.

m1 represents an integer of 0 to 3.

In a case where the structure of a preferable example of the 5-membered to 8-membered nitrogen-containing heterocyclic ring in G can be substituted with an -OM group, m1 is preferably 0 to 2. Among these, m1 is more preferably 0 or 1, and m1 = 1 is even more preferable.

In Formula (Y-1), combinations including the following (A) to (G) are particularly preferable.
(A) R₁ and R₂ may be the same as or different from each other, a substituted or unsubstituted alkyl group having 1 to 12 carbon atoms in total, a substituted or unsubstituted aryl group having 6 to 18 carbon atoms in total, or a substituted or unsubstituted heterocyclic group having 4 to 12 carbon atoms in total is preferable, a linear alkyl group or a branched alkyl group having 1 to 8 carbon atoms in total or an amino group is more preferable, a secondary or tertiary alkyl group or amino group is even more preferable, and a t-butyl and amino group is particularly preferable.
(B) X₁ and X₂ may be the same as or different from each other, a cyano group, an alkylsulfonyl group having 1 to 12 carbon atoms, an arylsulfonyl group having 6 to 18 carbon atoms, or a sulfamoyl group having 0 to 12 carbon atoms is more preferable, and a cyano group or an alkylsulfonyl group having 1 to 12 carbon atoms is even more preferable.
(C) Y₁ and Y₂ may be the same as or different from each other, a hydrogen atom, a substituted or unsubstituted alkyl group having 1 to 12 carbon atoms, a substituted or unsubstituted aryl group having 6 to 18 carbon atoms, or a substituted or unsubstituted heterocyclic group having 4 to 12 carbon atoms in total is preferable, a hydrogen atom or a substituted or unsubstituted alkyl group is more preferable, and a hydrogen atom is even more preferable.
(D) Z₁ and Z₂ may be the same as or different from each other, a substituted or unsubstituted alkyl group having 1 to 12 carbon atoms in total, a substituted or unsubstituted aryl group having 6 to 18 carbon atoms in total, or a substituted or unsubstituted heterocyclic group having 4 to 12 carbon atoms in total is preferable, a substituted or unsubstituted aryl group, or a substituted or unsubstituted heterocyclic group is more preferable, and a substituted aryl group is even more preferable.
   The substituted alkyl group, the substituted aryl group and the substituted heterocyclic group are preferably those substituted with an ionic hydrophilic group (for example, a carboxylate (preferably an alkali metal salt), a sulfonic acid or a sulfonate (preferably an alkali metal salt), and a phosphonic acid or phosphonate (preferably an alkali metal salt)) as a substituent.
(E) G represents an atomic group forming a 5-membered to 8-membered nitrogen-containing heterocyclic ring, and preferable examples of the 5-membered to 8-membered nitrogen-containing heterocyclic ring include an S-triazine ring, a pyrimidine ring, a pyridazine ring, a pyrazine ring, a pyridine ring, an imidazole ring, a pyrazole ring, and a pyrrole ring. Among these, an S-triazine ring, a pyrimidine ring, a pyridazine ring or a pyrazine ring is preferable, and an S-triazine ring is most preferable.
(F) m1 represents an integer of 0 to 3. In a case where the structure of the preferred example of the 5-membered to 8-membered nitrogen-containing heterocyclic ring in G can be substituted with an -OM group, m1 is preferably 0 to 2, and more preferably 0 or 1, and m1 = 1 is even more preferable.
(G) M is preferably a hydrogen atom or a cation, more preferably a hydrogen atom, an alkali metal ion, a ammonium or quaternary ammonium cation, even more preferably a Li⁺ ion, a Na⁺ ion, a K⁺ ion, or an NH₄⁺ ion, and most preferably a Li⁺ ion.

Among the compounds represented by Formula (Y-1), a compound represented by Formula (Y-1-1) below is preferable.

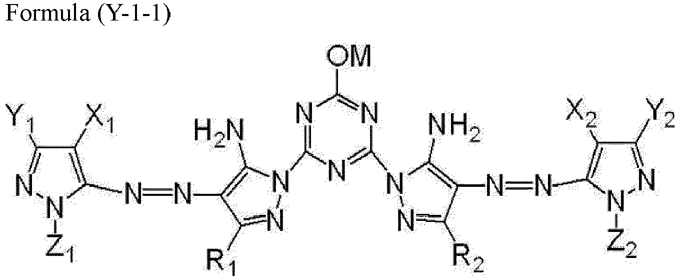

In Formula (Y-1-1), R₁, R₂, Y₁, and Y₂ are each independently the same as R₁, R₂, Y₁, and Y₂ in Formula (Y-1), and the preferable aspects are also the same.

X₁ and X₂ each independently represent a cyano group, an alkylsulfonyl group having 1 to 12 carbon atoms, an arylsulfonyl group having 6 to 18 carbon atoms, or a sulfamoyl group having 0 to 12 carbon atoms, and a cyano group, or an alkylsulfonyl group having 1 to 12 carbon atoms is preferable.

Z₁ and Z₂ each independently represent a hydrogen atom, a substituted or unsubstituted alkyl group, a substituted or unsubstituted alkenyl group, a substituted or unsubstituted alkynyl group, a substituted or unsubstituted aralkyl group, a substituted or unsubstituted aryl group, or a substituted or unsubstituted heterocyclic group. Among these, Z₁ and Z₂ each represent a substituted or unsubstituted alkyl group having 1 to 12 carbon atoms, a substituted or unsubstituted aryl group having 6 to 18 carbon atoms, or a substituted or unsubstituted heterocyclic group having 4 to 12 carbon atoms is preferable, a substituted or unsubstituted aryl group or a substituted or unsubstituted heterocyclic group is more preferable, and a substituted aryl group is even more preferable.

The substituents that Z₁ and Z₂ may be the same as the substituents that R₁, R₂, X₁, X₂, Y₁, Y₂, Z₁, and Z₂ in Formula (Y-1) may have and the preferable aspects are also the same.

M represents a hydrogen atom or a cation, a hydrogen atom, an alkali metal ion, an ammonium ion, or a quaternary ammonium ion is preferable, a Li⁺ ion, a Na⁺ ion, a K⁺ ion, or an NH₄⁺ ion is more preferable, and a Li⁺ ion is more preferable.

In the present disclosure, in a case where it is preferable that the compounds represented by Formulae (Y-1) and (Y-1-1) have hydrophilicity, the compounds preferably have two or more ionic hydrophilic groups in the molecule, more preferably have 2 to 10 ionic hydrophilic groups, and even more preferably have 3 to 6 ionic hydrophilic groups.

Specific examples of the compound represented by Formula (Y-1) and a salt thereof are shown below. However, the present disclosure is not limited to the following specific examples.

(YELLOW-1) 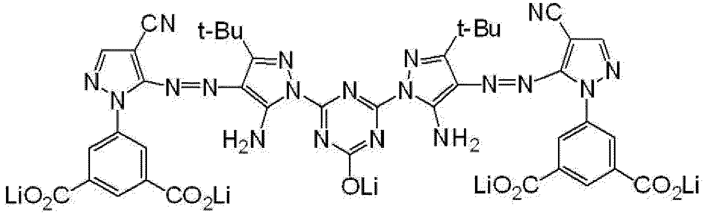
(YELLOW-2) 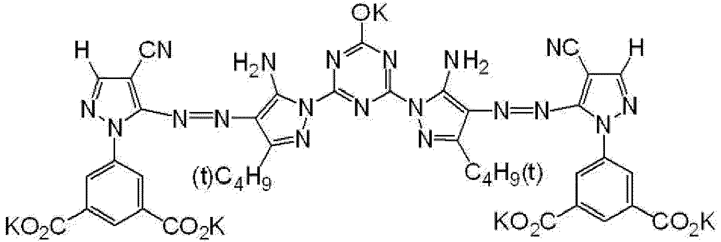
(YELLOW-3)

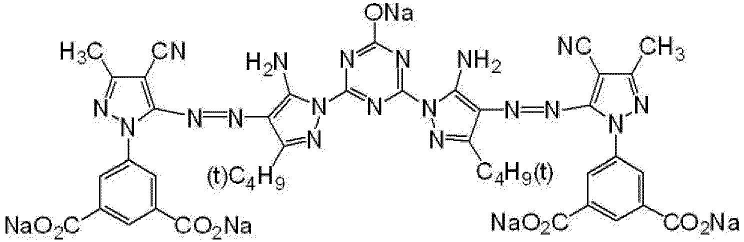

Next, the compound represented by Formula (Y-2) and a salt thereof will be described.

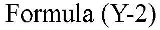

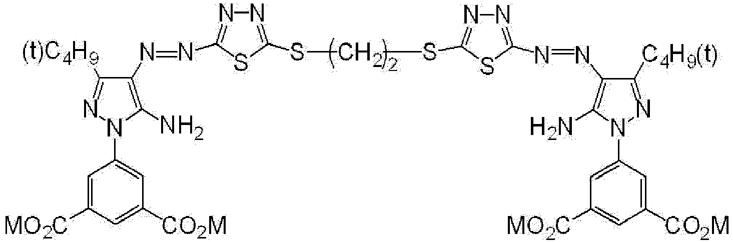

For the details of Formula (Y-2), the description of paragraphs 0305 to 0308 of JP2012-193330A can be referred to.

In Formula (Y-2), a plurality of M's each independently represent a hydrogen atom or a cation.

Examples of the cation in M include a Li⁺ ion, a Na⁺ ion, a K⁺ ion, or an NH₄⁺ ion.

The cation is preferably a Na⁺ ion, a K⁺ ion, or an NH₄⁺ ion, more preferably a Na⁺ ion, a K⁺ ion, and even more preferably a Li⁺ ion. In the dye represented by Formula (Y-2), it is preferable that the main component of M is a Li⁺ ion, and it is more preferable that all M's are Li⁺ ions. The main component means that the amount of the component is 70% by mass or more of all of M's.

It is most preferable that all M's in the dye represented by Formula (Y-2) included in the ink composition are K⁺ ions besides mixed salts. Since all M's are K⁺ ions, in a molecular dispersion state in a case of being dissolved in an aqueous solution or an ink solution, cation species are exchanged in an ionized state where a carboxy group, which is an ionic hydrophilic group, or a salt thereof (-CO₂M) is dissociated into -CO₂- and M⁺. Therefore, it is possible to obtain an effect that a salt having lower solubility in an aqueous solution or an ink solution is formed, thereby easily suppressing precipitation in a state of a salt of a colorant.

Specific examples of the compound represented by Formula (Y-2) and a salt thereof are shown below. However, the present disclosure is not limited to the following specific examples.

Among the compounds represented by Formula (Y-2), YELLOW-4 is particularly preferable.

(YELLOW-4) 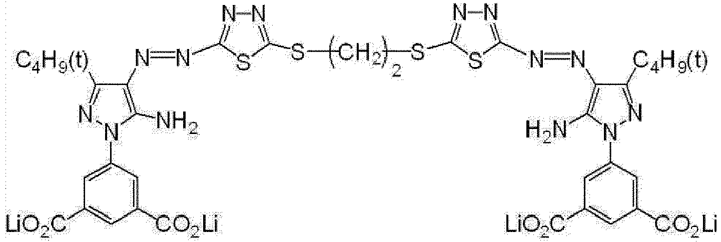
(YELLOW-5)

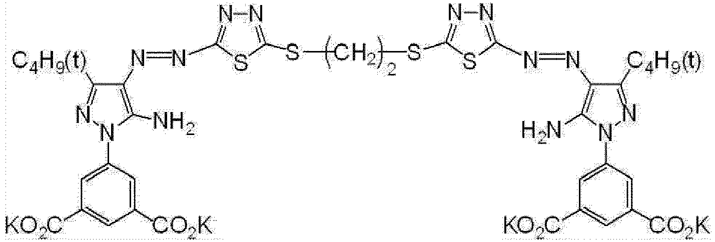

In the yellow ink composition, the ratio of the mass of lithium with respect to the total mass of alkali metals is preferably 70% by mass or more, more preferably 75% by mass or more, even more preferably 80% by mass or more, and particularly preferably 90% by mass or more.

In a case where the mass ratio of lithium is 70% by mass or more, the content of alkali metals (particularly potassium) other than lithium included in the ink composition is small, and thus, a viscosity increase phenomenon in a case of being mixed with an ink composition other than the yellow ink composition is effectively suppressed. Accordingly, it is possible to suppress ink stains on the recording medium caused by the accumulation of the ink composition.

### -Other Components-

The yellow ink composition in the present disclosure may further contain, in addition to colorants and the solvents, other components such as additives such as a surfactant, colloidal silica, a water-soluble polymer agent, an antifoaming agent, a pH adjusting agent, a preservative, a polymerization inhibitor, an anti-drying agent (wetting agent), an antifading agent, an emulsification stabilizer, a penetration enhancer, an ultraviolet absorber, a fungicide, a viscosity adjuster, a dispersion stabilizer, a rust inhibitor and a chelating agent. These other components are the same not only in the yellow ink composition but also in the magenta ink composition, the cyan ink composition, and the black ink composition described later.

### (Surfactant)

The yellow ink composition according to the present disclosure may contain a surfactant in a range that does not impair the effects according to the embodiment of the present invention. By containing a surfactant, it is possible to lower the surface tension and improve the j ettability.

However, it is preferable that the surfactant is not substantially contained from the viewpoint of avoiding adverse effects (such as abnormal jetting due to bubbles near the jetting port) caused by containing the surfactant in the ink composition. Therefore, in the present disclosure, the content of the surfactant in the yellow ink composition is preferably less than 0.1% by mass with respect to the total mass of the ink composition.

Here, the fact that the content of the surfactant is less than 0.1% by mass with respect to the total mass of the ink composition means that the ink composition does not substantially contain the surfactant, that is, the content of the surfactant is such an extent that the content is such that the surface activity is not exhibited.

In the ink set according to the present disclosure, a composition in which the surface tension is reduced by containing a solvent having a low SP value without containing a surfactant, that is, the aforementioned "solvent containing no nitrogen atom and having an SP value of 22 to 26" to improve the jettability is adopted. This composition is preferable from the viewpoint that in particular, the effect of lowering the dynamic surface tension in a short period of time is greater than that of the surfactant, and various adverse effects caused by bubbles easily generated due to the use of the surfactant are avoided.

As the surfactant, a compound having a structure having both a hydrophilic portion and a hydrophobic portion in a molecule can be used, and an anionic surfactant, a cationic surfactant, an amphoteric surfactant, a nonionic surfactant, and a betaine-based surfactant can be used.

As the surfactant, a nonionic surfactant is preferable, and an acetylene glycol derivative (acetylene glycol-based surfactant) is more preferable.

Examples of the acetylene glycol-based surfactant include 2,4,7,9-tetramethyl-5-decyne-4,7-diol and an alkylene oxide adduct of 2,4,7,9-tetramethyl-5-decyne-4,7-diol. As the surfactant, commercially available products may be used, and examples of commercially available products include E series (for example, OLFINE E1010) manufactured by Nissin Chemical Industry Co., Ltd.

### (Colloidal Silica)

The yellow ink composition in the present disclosure may contain colloidal silica. By containing the colloidal silica, the stability during continuous jetting of the ink composition can be further improved.

The colloidal silica is a colloid consisting of fine particles of an inorganic oxide including silicon having an average particle diameter of several 100 nm or less. The colloidal silica may include silicon dioxide (including a hydrate thereof) as a main component, and may contain aluminate (sodium aluminate, potassium aluminate, or the like) as a minor component.

The colloidal silica may contain an inorganic salt such as sodium hydroxide, potassium hydroxide, lithium hydroxide, or ammonium hydroxide, and an organic salt such as tetramethylammonium hydroxide. These inorganic salt and organic salt function as, for example, a stabilizing agent for colloid.

For the colloidal silica, for example, the description in paragraphs 0043 to 0050 of JP2011-202117A can be appropriately referred to.

In addition, the ink composition in the present disclosure may contain an alkali metal silicate in place of colloidal silica or in addition to colloidal silica, as necessary. For the alkali metal silicate, the description of paragraphs 0052 to 0056 of JP2011-202117A can be appropriately referred to.

In a case where the yellow ink composition in the present disclosure includes the colloidal silica, the content of the colloidal silica is preferably 0.0001% by mass to 10% by mass, more preferably 0.01% by mass to 3% by mass, even more preferably 0.02% by mass to 0.5% by mass, and particularly preferably 0.03% by mass to 0.3% by mass with respect to the total amount of the ink composition.

### (Water-Soluble Polymer Compound)

The yellow ink composition according to the present disclosure may contain a water-soluble polymer compound.

The water-soluble polymer compound is not particularly limited, and a known water-soluble polymer compound such as polyvinyl alcohol, polyacrylamide, polyvinyl pyrrolidone, and polyethylene glycol can be used. In addition, as the water-soluble polymer compound, a specific polymer compound which may be contained in a treatment liquid described later, and water-soluble polymer compounds described in paragraphs 0026 to 0080 of JP2013-001854A are also preferable.

In a case where the yellow ink composition in the present disclosure contains a water-soluble polymer compound, the content of the water-soluble polymer compound is preferably 0.0001% by mass to 10% by mass, more preferably 0.01% by mass to 3% by mass, even more preferably 0.02% by mass to 0.5% by mass, and particularly preferably 0.03% by mass to 0.3% by mass with respect to the total amount of the ink composition.

### (Antifoaming Agent)

The yellow ink composition in the present disclosure may contain an antifoaming agent.

Examples of the antifoaming agent include a silicone-based compound (silicone-based antifoaming agent) and a pluronic-based compound (pluronic-based antifoaming agent). Among these, a silicone-based antifoaming agent is preferable. As the silicone-based antifoaming agent, a silicone-based antifoaming agent having a polysiloxane structure is preferable.

Commercially available products can be used as the antifoaming agent. Examples of commercially available products include BYK-012, 017, 021, 022, 024, 025, 038, and 094 (all manufactured by BYK Chemie Japan KK), KS-537, KS-604, and KM-72F (all manufactured by Shin-Etsu Chemical Co., Ltd.), TSA-739 (manufactured by Momentive Performance Materials Japan LLC), and OLFIN AF104 (manufactured by Nissin Chemical Industry Co., Ltd.).

Among these, BYK-017, 021, 022, 024, 025, and 094, KS-537, KS-604, KM-72F, and TSA-739, which are silicone-based antifoaming agents, are preferable, and among these, BYK-024 is most preferable from the viewpoint of jetting stability of the ink.

In a case where the yellow ink composition in the present disclosure contains an antifoaming agent, the content of the antifoaming agent is preferably 0.0001% by mass to 1% by mass, and more preferably 0.001% by mass to 0.1% by mass with respect to the total amount of the ink composition.

### (pH Adjusting Agent)

The yellow ink composition in the present disclosure may contain a pH adjusting agent.

The pH adjusting agent is not particularly limited as long as the pH can be adjusted to be a desired value without adversely affecting the prepared ink composition, and can be appropriately selected depending on the purpose. Examples thereof include alcohol amines (for example, diethanolamine, triethanolamine, and 2-amino-2-ethyl-1,3-propanediol), alkali metal hydroxides (for example, lithium hydroxide, sodium hydroxide, and potassium hydroxide), ammonium hydroxide (for example, ammonium hydroxide, and quaternary ammonium hydroxide), phosphonium hydroxide, and alkali metal carbonate.

In a case where the yellow ink composition in the present disclosure contains the pH adjusting agent, the content of the pH adjusting agent is preferably such that the pH of the ink composition becomes 5 to 10 (more preferably 7.0 to 9.5).

The yellow ink composition in the present disclosure may contain an inorganic salt as a pH buffer. By containing an inorganic salt, fluctuations in pH can be suppressed and jetting can be stabilized.

Examples of the inorganic salt as the pH buffer include carbonate, and phosphate, and carbonate is preferable from the viewpoint of member resistance. Examples of the carbonate include potassium carbonate, sodium carbonate, lithium carbonate, ammonium carbonate, potassium hydrogen carbonate, sodium hydrogen carbonate, and lithium hydrogen carbonate, and from the viewpoint of not lowering the solubility of the dye, lithium carbonate or lithium hydrogen carbonate is particularly preferable.

The inorganic salts can be used alone or in combination of two or more.

In a case where the yellow ink composition in the present disclosure contains an inorganic salt, the content of the inorganic salt in the ink composition (in a case of two or more kinds, the total content) is not particularly limited, but the content is preferably 0.001% by mass to 1% by mass, more preferably 0.01% by mass to 0.5% by mass, and particularly preferably 0.03% by mass to 0.1% by mass with respect to the total amount of the ink composition.

### (2) Magenta Ink Composition

The magenta ink composition in the present disclosure contains at least one compound (also referred to as a magenta dye) selected from the group consisting of a compound represented by Formula (M) or (MM) and a salt thereof, a solvent A, a solvent B, a solvent C, a solvent D, and water, and may further contain other components such as a surfactant and an additive, as necessary.

The solvent A, the solvent B, the solvent C, the solvent D, the water, and other components in the magenta ink composition are the same as those in the above-described yellow ink composition, and the preferable aspects are also the same. Therefore, the detailed description in this section is omitted.

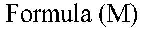

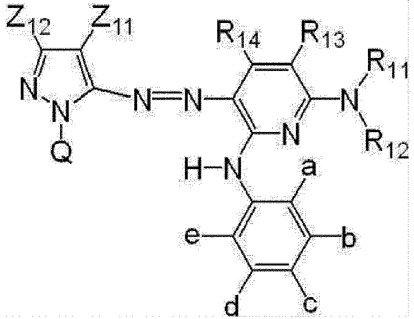

For the details of Formula (M), the descriptions of paragraphs 0520 to 0563, paragraph 0496, paragraph 0500, paragraph 0507, paragraph 0514, and paragraph 0553 of JP2007-138124A can be referred to.

In Formula (M), Z₁₁ represents an electron-withdrawing group having a Hammett's substituent constant σₚ value of 0.2 or more. The electron-withdrawing group for Z₁₁ is an electron-withdrawing group having a Hammett's substituent constant σₚ value of 0.20 or more and preferably 0.30 or more. The upper limit of the σₚ value is preferably 1.0 or less.

Specific examples of the electron-withdrawing group having a σₚ value of 0.20 or more include an acyl group, an acyloxy group, a carbamoyl group, an alkyloxycarbonyl group, an aryloxycarbonyl group, a cyano group, a nitro group, a dialkylphosphono group, and a diarylphospho group, a diarylphosphinyl group, alkylsulfinyl, an arylsulfinyl group, an alkylsulfonyl group, an arylsulfonyl group, a sulfonyloxy group, an acylthio group, a sulfamoyl group, a thiocyanate group, a thiocarbonyl group, a halogenated alkyl group, a halogenated alkoxy group, a halogenated aryloxy group, a halogenated alkylamino group, a halogenated alkylthio group, a heterocyclic group, a halogen atom, an azo group, a selenocyanate group, and an aryl group substituted with other electron-withdrawing group having σₚ value of 0.20 or more.

Z₁₁ preferably represents a cyano group, an alkylsulfonyl group, an arylsulfonyl group, a nitro group, or a halogen atom, more preferably represents a cyano group, an alkylsulfonyl group, or an arylsulfonyl group, and most preferably represents a cyano group. The substituent at Z₁₁ may be further substituted.

Z₁₂ represents a hydrogen atom, an alkyl group, an alkenyl group, an alkynyl group, an aralkyl group, an aromatic group, a heterocyclic group, or an acyl group. Among these, Z₁₂ is more preferably an alkyl group. The substituent at Z₁₂ may be further substituted.

R₁₁ and R₁₂ each independently represent a hydrogen atom, an alkyl group, a cycloalkyl group, an alkenyl group, an alkynyl group, an aralkyl group, an aryl group, a heterocyclic group, an acyl group, an alkoxycarbonyl group, an aryloxycarbonyl group, a carbamoyl group, an alkylsulfonyl group, an arylsulfonyl group, or a sulfamoyl group. Each group may further have a substituent.

However, R₁₁ and R₁₂ do not represent a hydrogen atom at the same time.

Among these, R₁₁ and R₁₂ each independently preferably represent a hydrogen atom, a substituted or unsubstituted cycloalkyl group, a substituted or unsubstituted aryl group, a substituted or unsubstituted heterocyclic group, a substituted or unsubstituted acyl group, a substituted or unsubstituted alkylsulfonyl group, and a substituted or unsubstituted arylsulfonyl group, and more preferably represent a hydrogen atom, a substituted aryl group, and a substituted heterocyclic group, and among these, a substituted aryl group and a substituted heterocyclic group are particularly preferable.

In a case where the group is substituted with a substituent, the substituent is preferably a linear or branched alkyl group having 1 to 12 carbon atoms, a linear or branched aralkyl group having 7 to 18 carbon atoms, a linear or branched alkenyl group having 2 to 12 carbon atoms, a linear or branched alkynyl group having 2 to 12 carbon atoms, a cycloalkyl group having 3 to 12 carbon atoms, a cycloalkenyl group having 3 to 12 carbon atoms (for example, methyl, ethyl, propyl, isopropyl, sec-butyl, t-butyl, 2-ethylhexyl, 2-methylsulfonylethyl, 3-phenoxypropyl, trifluoromethyl, and cyclopentyl), or an ionic hydrophilic group (for example, a carboxylate (preferably an alkali metal salt), a sulfonic acid or sulfonic acid salt (preferably an alkali metal salt), or a phosphonic acid or phosphonate (preferably an alkali metal salt)).

However, R₁₁ and R₁₂ do not represent hydrogen atoms at the same time.

R₁₃, R₁₄, b, c, and d each independently represent a hydrogen atom, a halogen atom, an alkyl group, an alkenyl group, an alkynyl group, an aralkyl group, an aryl group, a heterocyclic group, a cyano group, a carboxy group, a carbamoyl group, an alkoxycarbonyl group, an aryloxycarbonyl group, an acyl group, a hydroxy group, an alkoxy group, an aryloxy group, a silyloxy group, an acyloxy group, a carbamoyloxy group, a heterocyclic oxy group, an alkoxycarbonyloxy group, an aryloxycarbonyloxy group, an amino group substituted with an alkyl group, an aryl group or a heterocyclic group, an acylamino group, a ureido group, a sulfamoylamino group, an alkoxycarbonylamino group, an aryloxycarbonylamino group, an alkylsulfonylamino group, an arylsulfonylamino group, a nitro group, an alkylthio group, an arylthio group, an alkylsulfonyl group, an arylsulfonyl group, an alkylsulfinyl group, an arylsulfinyl group, a sulfamoyl group, a heterocyclic thio group, or an ionic hydrophilic group, and each group may be substituted with these groups.

R₁₃ and R₁₄ each independently preferably represent a hydrogen atom, an alkyl group, a cyano group, a carboxy group, a carbamoyl group, or an alkoxycarbonyl group, and more preferably represent a hydrogen atom, an alkyl group, a cyano group, or a carboxy group. Each group may be further substituted, and similar to R₁₁ and R₁₂, the substituent is preferably a linear or branched alkyl group having 1 to 12 carbon atoms, a linear or branched aralkyl group having 7 to 18 carbon atoms, a linear or branched alkenyl group having 2 to 12 carbon atoms, a linear or branched alkynyl group having 2 to 12 carbon atoms, a cycloalkyl group having 3 to 12 carbon atoms, a cycloalkenyl group having 3 to 12 carbon atoms, or an ionic hydrophilic group.

Among these, it is preferable that R₁₃ represents a hydrogen atom and R₁₄ represents an alkyl group having 1 to 6 carbon atoms, and it is more preferable that R₁₃ represents a hydrogen atom and R₁₄ represents a methyl group.

In addition, b, c, and d each independently preferably represent a hydrogen atom, an alkyl group, a cyano group, a carboxy group, a carbamoyl group, an alkoxycarbonyl group, an ionic hydrophilic group, and more preferably represent a hydrogen atom, an alkyl group, a cyano group, or an ionic hydrophilic group. Each group may be further substituted, and similar to R₁₁ and R₁₂, the substituent is preferably a linear or branched alkyl group having 1 to 12 carbon atoms, a linear or branched aralkyl group having 7 to 18 carbon atoms, a linear or branched alkenyl group having 2 to 12 carbon atoms, a linear or branched alkynyl group having 2 to 12 carbon atoms, a cycloalkyl group having 3 to 12 carbon atoms, a cycloalkenyl group having 3 to 12 carbon atoms, or an ionic hydrophilic group.

Among these, c more preferably represents a hydrogen atom or an alkyl group having 1 to 6 carbon atoms, and even more preferably represents a hydrogen atom or a methyl group.

In addition, b and d each independently more preferably represent a hydrogen atom or an ionic hydrophilic group, and even more preferably represent a hydrogen atom, a sulfonic acid or sulfonate (preferably an alkali metal salt), or a carboxylic acid or carboxylate (preferably a alkali metal salt), and a combination of b and d is particularly preferably a combination of a hydrogen atom and a sulfonic acid or sulfonate.

a and e each independently represent an alkyl group, an alkoxy group, or a halogen atom, and in a case where a and e both represent an alkyl group, the total number of carbon atoms in the alkyl group is 3 or more, and the alkyl groups in a and e may be further substituted.

R₁₃ and R₁₁, or R₁₁ and R₁₂ may be bonded to each other to form a 5-membered- or 6-membered ring.
a and b, or e and d may be bonded to each other to form a ring.

Q represents a hydrogen atom, an alkyl group, an alkenyl group, an alkynyl group, an aralkyl group, an aromatic group, or a heterocyclic group. The group in Q may have a substituent.

Q is preferably an aryl group substituted with an electron-withdrawing group or a heterocyclic group substituted with an electron-withdrawing group. The electron-withdrawing group which is a substituent of Q is preferably a group having a Hammett's substituent constant σₚ value of 0.20 or more, and more preferably a group having a Hammett's substituent constant σₚ value of 0.30 or more. The upper limit of the σₚ value is preferably 1.0 or less. Specific examples of the electron-withdrawing group having a σₚ value of 0.20 or more are the same as described in Z₁₁ in Formula (M), and a sulfonic acid or sulfonate (preferably an alkali metal salt), or a carboxylic acid or carboxylate (preferably an alkali metal salt) are preferable.

In Formula (M), a combination including the following (A) to (F) is particularly preferable.
(A) Z₁₁ is an electron-withdrawing group having a Hammett's substituent constant σₚ value of 0.20 or more and preferably 0.30 or more. The upper limit of the σₚ value is preferably 1.0 or less. As Z₁₁, a cyano group, an alkylsulfonyl group, an arylsulfonyl group, a nitro group or a halogen atom is more preferable, a cyano group, an alkylsulfonyl group or an arylsulfonyl group is even more preferable, and a cyano group is most preferable.
(B) As Z₁₂, a hydrogen atom, an alkyl group, a cycloalkyl group, an aralkyl group, an aryl group, a heterocyclic group, or an acyl group is preferable, and an alkyl group is more preferable. Each substituent may be further substituted. More specifically, the alkyl group as Z₁₂ includes an alkyl group having a substituent and an unsubstituted alkyl group. The alkyl group is preferably an alkyl group having 1 to 12 carbon atoms excluding the carbon atom of the substituent, and more preferably an alkyl group having 1 to 6 carbon atoms. Examples of the substituent include a hydroxy group, an alkoxy group, a cyano group, a halogen atom, and an ionic hydrophilic group. Among these, methyl, ethyl, butyl, isopropyl, t-butyl, hydroxyethyl, methoxyethyl, cyanoethyl, trifluoromethyl, 3-sulfopropyl and 4-sulfobutyl are preferable, isopropyl and t-butyl are particularly preferable, and t-butyl is more preferable.
(C) Q represents a hydrogen atom, an alkyl group, an alkenyl group, an alkynyl group, an aralkyl group, an aromatic group or a heterocyclic group. Each of these substituents may be further substituted. Further, Q is preferably an aryl group or a heterocyclic group substituted with an electron-withdrawing group. The electron-withdrawing group functions as a substituent of Q is an electron-withdrawing group having a Hammett's substituent constant σₚ value of 0.20 or more and preferably 0.30 or more. The upper limit of the σₚ value is preferably 1.0 or less. More specifically, a heterocyclic group substituted with an electron-withdrawing group is preferable, and a sulfo group, a substituted or unsubstituted carbamoyl group, a benzoxazole ring substituted with a substituted or unsubstituted sulfamoyl group, and a benzothiazole ring are preferable, a sulfo group, and a benzothiazole ring substituted with a substituted sulfamoyl group are particularly preferable.
(D) a and e are preferably an alkyl group or a halogen atom, and a case where a and e are unsubstituted alkyl groups in a case where a and e are both alkyl groups, the total number of carbon atoms of a and e is 3 or more (preferably 5 or less), and a, b, c, and d each independently represent a hydrogen atom, a halogen atom, an alkyl group, or an ionic hydrophilic group (each preferably represents a hydrogen atom, an alkyl group having 1 to 4 carbon atoms, and an ionic hydrophilic group) is preferable. More preferably, a and e are each independently preferably a methyl group, an ethyl group, or an isopropyl group, more preferably an ethyl group or an isopropyl group, and even more preferably a = b = ethyl group or an isopropyl group.
   c is preferably a hydrogen atom or an alkyl group, and more preferably a hydrogen atom or a methyl group.
   b and d are each independently preferably a hydrogen atom or an ionic hydrophilic group and more preferably a hydrogen atom, a sulfo group, or a carboxy group, and even more preferably, a combination of b and d is a hydrogen atom and a sulfo group.
(E) R₁₃ is a hydrogen atom, and R₁₄ is an alkyl group (particularly a methyl group).
(F) R₁₁ and R₁₂ are each independently a hydrogen atom, a substituted aryl group, or a substituted heterocyclic group.

The compound represented by Formula (M) has at least one (preferably 3 or more and 6 or less) ionic hydrophilic group in the molecule. That is, in Formula (M), at least one of Z₁₁, Z₁₂, R₁₁, R₁₂, R₁₃, R₁₄, a, b, c, d, e, or Q has at least one ionic hydrophilic group.

The ionic hydrophilic group includes a sulfonic acid group (sulfo group), a carboxylic acid group (carboxy group), a phosphonic acid group (phosphono group), a quaternary ammonium group, and the like. As the ionic hydrophilic group, a carboxy group, a phosphono group, and a sulfo group are preferable, and a carboxy group and a sulfo group are particularly preferable. Particularly, at least one is most preferably a sulfo group. The carboxy group, the phosphono group and the sulfo group may be in the form of a salt, and examples of counter ions forming a salt include an ammonium ion, an alkali metal ion (for example, a lithium ion, a sodium ion, and a potassium ion), and an organic cation (for example, a tetramethylammonium ion, a tetramethylguanidinium ion, and a tetramethylphosphonium). Among the counter ions, alkali metal salts are preferable. Among the alkali metal salts, a potassium ion, a sodium ion and a lithium ion are preferable, and a sodium ion and a lithium ion are most preferable. Particularly, from the viewpoint of improving solubility and suppressing bronze during ink jet recording, it is most preferable that the ionic hydrophilic group is a sulfo group and the counter ion thereof is a lithium ion.

The compound represented by Formula (M) preferably has 3 or more and 6 or less ionic hydrophilic groups in the molecule, more preferably 3 or more and 6 or less sulfo groups, and even more preferably 3 or more and 5 or less sulfo groups.

Specific examples of the compound represented by Formula (M) and a salt thereof are shown below. However, the present disclosure is not limited to the following specific examples.

(MAGENTA-1) 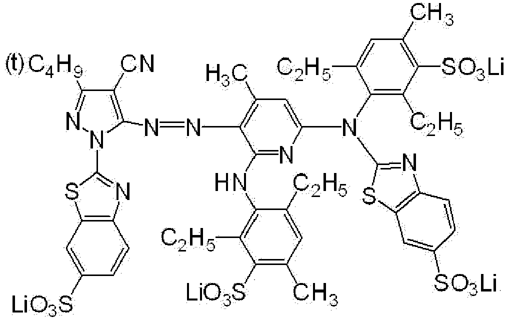
(MAGENTA-2) 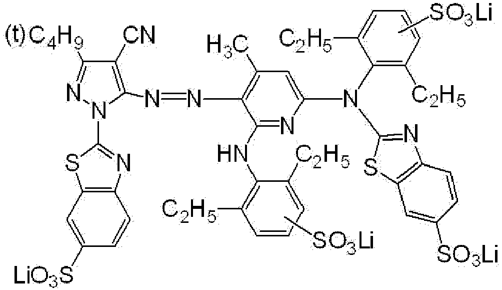
(MAGENTA-3) 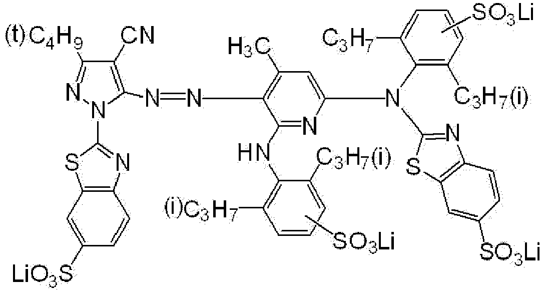
(MAGENTA-4)

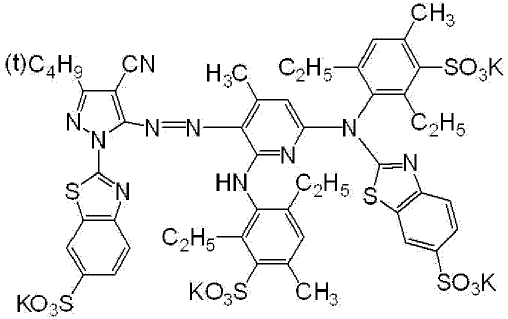

Next, the compound represented by Formula (MM) and a salt thereof will be described.

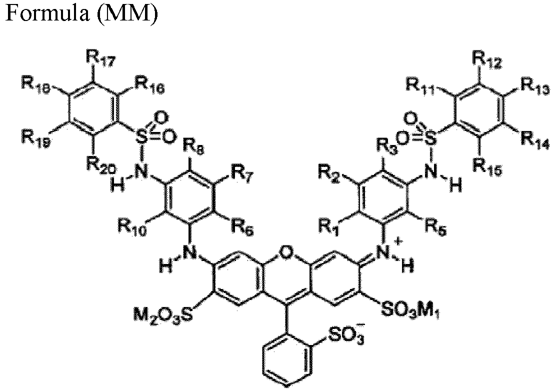

In Formula (MM), R₁, R₅, R₆, and R₁₀ each independently represent an alkyl group. R₂, R₃, R₇, R₈, R₁₁, R₁₂, R₁₃, R₁₄, R₁₅, R₁₆, R₁₇, R₁₈, R₁₉, and R₂₀ each independently represent a hydrogen atom or a substituent. M₁ and M₂ each independently represent a hydrogen atom, an alkali metal ion, or an ammonium ion.

R₁, R₅, R₆, and R₁₀ in Formula (MM) each independently represent an alkyl group, and from the viewpoint of availability of raw materials and ease of synthesis, preferably represent an alkyl group having 1 to 6 carbon atoms, more preferably represent an alkyl group having 1 to 3 carbon atoms, even more preferably represent a methyl group, an ethyl group or an isopropyl group, and particularly preferably represent a methyl group.

The alkyl group represented by R₁, R₅, R₆, and R₁₀ may have a substituent, and examples of the substituent include a substituent selected from the following substituent group A.

In case where R₃ and R₈ in Formula (MM) represent a substituent, examples of the substituent include a substituent selected from the following substituent group A, and among these, R₃ and R₈ preferably represent an alkyl group. In case where R₃ and R₈ represent an alkyl group, from the viewpoint of availability of raw materials and ease of synthesis, R₃ and R₈ preferably represent an alkyl group having 1 to 3 carbon atoms, more preferably represent a methyl group, an ethyl group, or an isopropyl group, and most preferably represent a methyl group.

In a case where R₂ and R₇ in Formula (MM) represent a substituent, examples of the substituent include a substituent selected from the following substituent group A, and among these, R₂ and R₇ preferably represent an alkyl group. R₂ and R₇ in Formula (MM) each preferably independently represent a hydrogen atom or an alkyl group, and more preferably represent a hydrogen atom from the viewpoint of availability of raw materials and ease of synthesis.

In a case where R₂, R₃, R₇, and R₈ in Formula (MM) represent an alkyl group, the alkyl group may have a substituent, and the substituent is a substituent selected from the following substituent group A.

### (Substituent Group A)

Examples include a halogen atom, an alkyl group, a cycloalkyl group, an aralkyl group, an alkenyl group, an alkynyl group, an aryl group, a heterocyclic group, a cyano group, a hydroxy group, a nitro group, an alkoxy group, an aryloxy group, a silyloxy group, a heterocyclic oxy group, an acyloxy group, a carbamoyloxy group, an alkoxycarbonyloxy group, an aryloxycarbonyloxy group, an amino group, an acylamino group, an aminocarbonylamino group, an alkoxycarbonylamino group, an aryloxycarbonylamino group, a sulfamoylamino group, an alkylsulfonylamino or arylsulfonylamino group, a mercapto group, an alkylthio group, an arylthio group, a heterocyclic thio group, a sulfamoyl group, an alkylsulfinyl or arylsulfinyl group, an alkylsulfonyl or arylsulfonyl group, an acyl group, an aryloxycarbonyl group, an alkoxycarbonyl group, a carbamoyl group, an aryl or heterocyclic azo group, an imide group, a phosphino group, a phosphinyl group, a phosphinyloxy group, a phosphinylamino group, a silyl group, and an ionic hydrophilic group. These substituents may be further substituted, and examples of the further substituent include groups selected from the substituent group A described above.

In Formula (MM), R₁₁, R₁₂, R₁₃, R₁₄, R₁₅, R₁₆, R₁₇, R₁₈, R₁₉, and R₂₀ each independently represent a hydrogen atom or a substituent. Examples of the substituent include a substituent selected from the above-mentioned substituent group A.

R₁₁ and R₁₆ each independently preferably represent a hydrogen atom, a hydroxy group, a chlorine atom, or a methyl group, more preferably represent a hydrogen atom, a hydroxy group, or a methyl group, even more preferably represent a hydrogen atom or a hydroxy group, and particularly preferably represent a hydroxy group.

R₁₂, R₁₄, R₁₇ and R₁₉ each independently preferably represent a hydrogen atom or an ionic hydrophilic group, amore preferably represent a hydrogen atom, a carboxy group, a salt of a carboxy group, a sulfo group, or a salt of a sulfo group, even more preferably represent a hydrogen atom or a carboxy group or a salt thereof, and particularly preferably represents a carboxy group or a salt thereof.

As the salt, an alkali metal or alkaline earth metal salt is preferable, and an alkali metal salt is more preferable. Examples of the alkali metal include lithium, potassium, and sodium.

R₁₃ and R₁₈ each independently preferably represent a hydrogen atom or an ionic hydrophilic group, more preferably represent a hydrogen atom or a carboxy group, and even more preferably represent a hydrogen atom, and it is particularly preferable that both R₁₃ and R₁₈ represent a hydrogen atom.

The compound represented by Formula (MM) preferably satisfies at least one of the following condition (i-1) or (i-2), and more preferably satisfies the following both conditions (i-1) and (i-2).

Condition (i-1): At least one of R₁₁, R₁₂, R₁₃, R₁₄, or R₁₅ represents a carboxy group or a salt thereof.

Condition (i-2): At least one of R₁₆, R₁₇, R₁₈, R₁₉, or R₂₀ represents a carboxy group or a salt thereof.

The compound represented by Formula (MM) preferably satisfies at least one of the following condition (ii-1) or (ii-2), and more preferably satisfies the following both conditions (ii-1) and (ii-2).

Condition (ii-1): At least one of R₁₁, R₁₂, R₁₃, R₁₄, or R₁₅ represents a hydroxy group, and at least one represents a carboxy group or a salt thereof.

Condition (ii-2): At least one of R₁₆, R₁₇, R₁₈, R₁₉, or R₂₀ represents a hydroxy group, and at least one represents a carboxy group or a salt thereof.

In a case where the condition (i-1) or (ii-1) is satisfied, it is particularly preferable that two of R₁₁, R₁₂, R₁₃, R₁₄ and R₁₅ represent a carboxy group or a salt thereof.

In a case where the condition (i-2) or (ii-2) is satisfied, it is particularly preferable that two of R₁₆, R₁₇, R₁₈, R₁₉ and R₂₀ represent a carboxy group or a salt thereof.

Particularly, it is most preferable that R₁₁ represents a hydroxy group, R₁₂ and R₁₄ represent a carboxy group or a salt thereof, R₁₃ and R₁₅ represent a hydrogen atom, R₁₆ represents a hydroxy group, R₁₇ and R₁₉ represent a carboxy group or a salt thereof, and R₁₈ and R₂₀ represent a hydrogen atom.

M₁ and M₂ in Formula (MM) each independently represent a hydrogen atom, an alkali metal ion or an ammonium ion, and preferably represent a hydrogen atom, a lithium ion (Li⁺), a sodium ion (Na⁺), a potassium ion (K⁺), or an ammonium ion (NH₄⁺), more preferably represent a lithium ion or a sodium ion, particularly preferably represents a lithium ion or a mixed ion having a lithium ion as a main component, and most preferably represent a lithium ion.

Among the compounds represented by Formula (MM) and salts thereof, a compound represented by Formula (MM-1A) and a salt thereof are preferable.

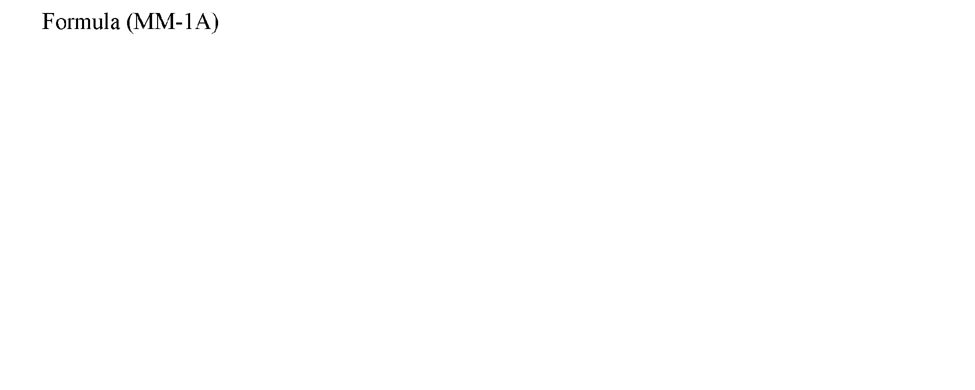

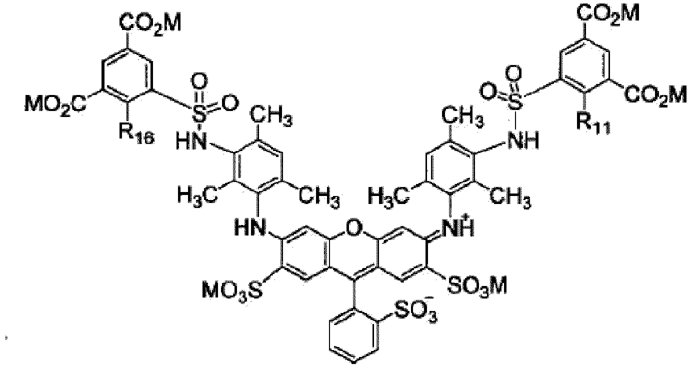

In Formula (MM-1A), R₁₁ and R₁₆ each independently represent a hydrogen atom, a halogen atom, a hydroxy group, or a methyl group. M represents a hydrogen atom, an alkali metal ion, or an ammonium ion.

In a case where R₁₁ and R₁₆ in Formula (MM-1A) represent a halogen atom, examples of the halogen atom include a fluorine atom, a chlorine atom, a bromine atom, and an iodine atom. Among these, a chlorine atom or a bromine atom is particularly preferable, and a chlorine atom is most preferable.

R₁₁ and R₁₆ in Formula (MM-1A) preferably represent a hydrogen atom, a chlorine atom, a hydroxy group, or a methyl group, more preferably represent a hydrogen atom or a hydroxy group, and most preferably represent a hydroxy group.

In Formula (MM-1A) above, a plurality of Ms' each independently represent a hydrogen atom, an alkali metal ion, or an ammonium ion. M's is preferably each independently an alkali metal ion (preferably a lithium ion, a sodium ion, or a potassium ion), among these, particularly, a lithium ion or a sodium ion is preferable, and a lithium ion is most preferable.

The compound represented by Formula (MM) can be synthesized by a known method (for example, the method described in WO2017/006939).

Specific examples of the compound represented by Formula (MM) are shown below. However, the present disclosure is not limited to these example. Here, Me represents a methyl group, and Et represents an ethyl group.

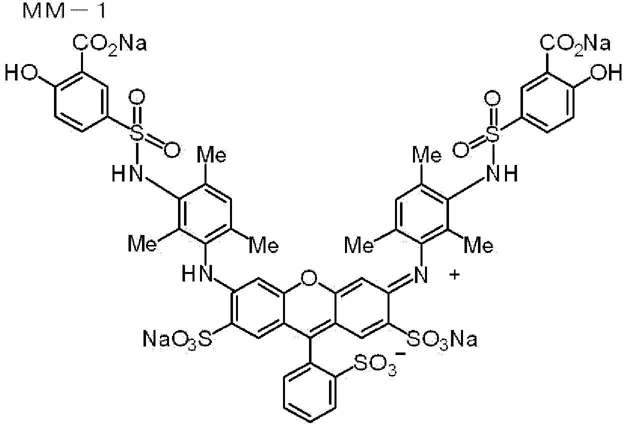

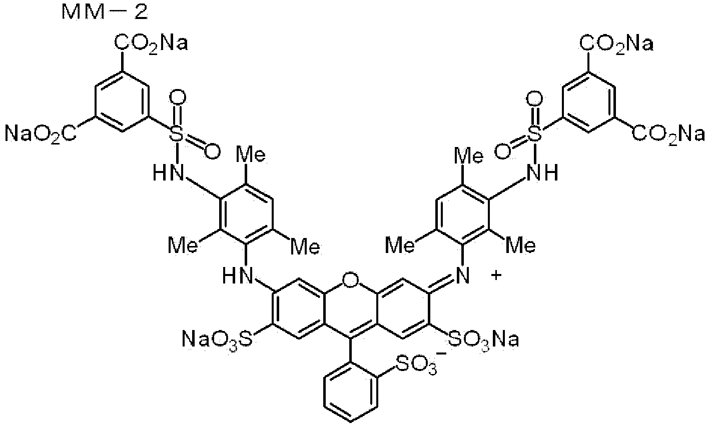

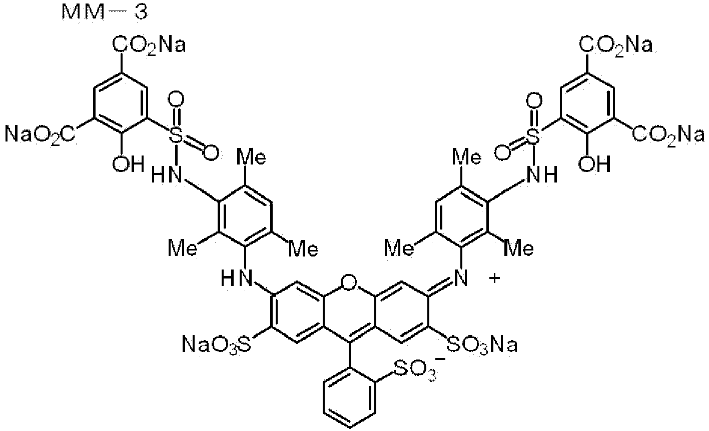

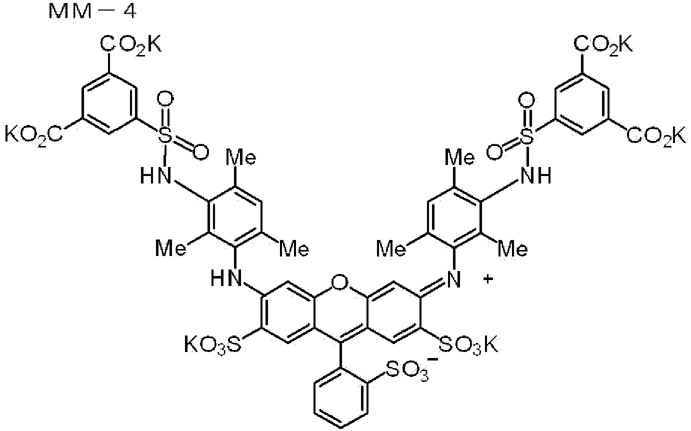

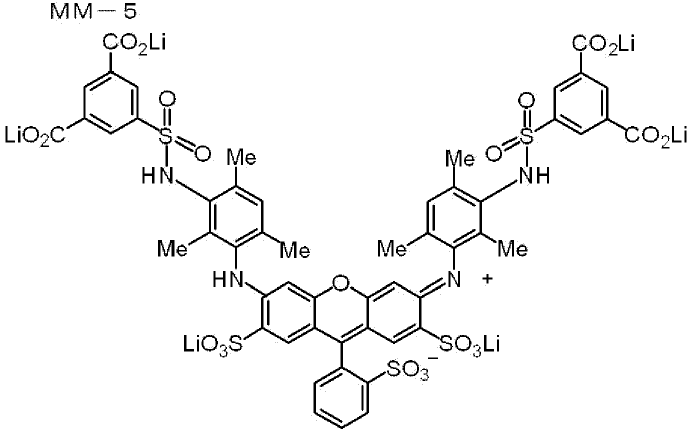

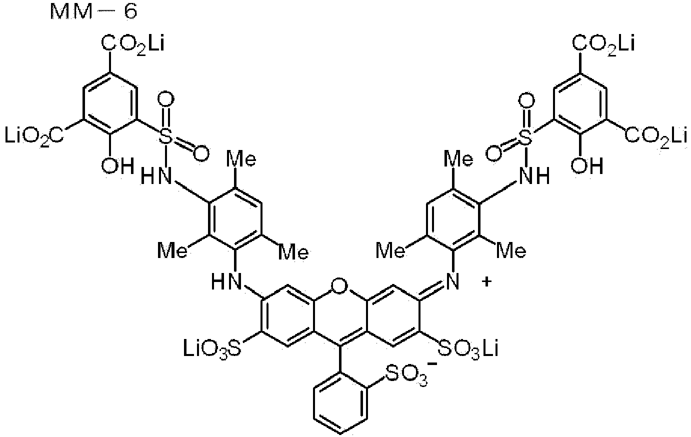

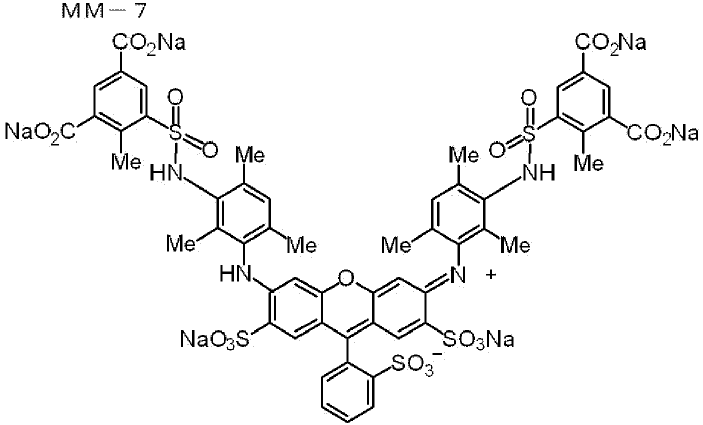

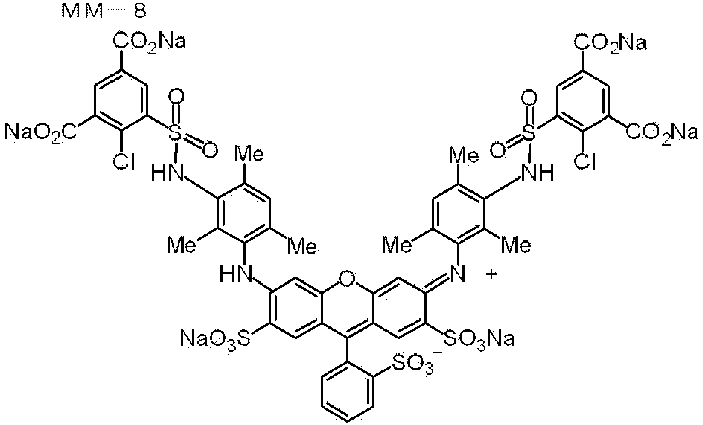

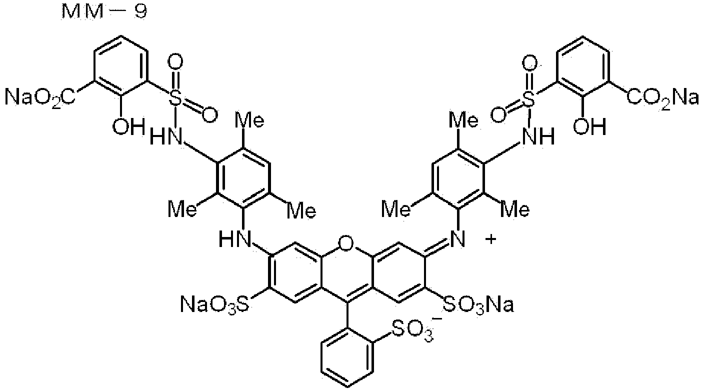

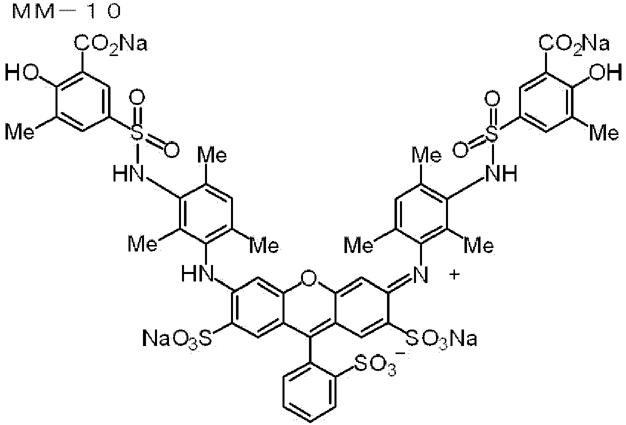

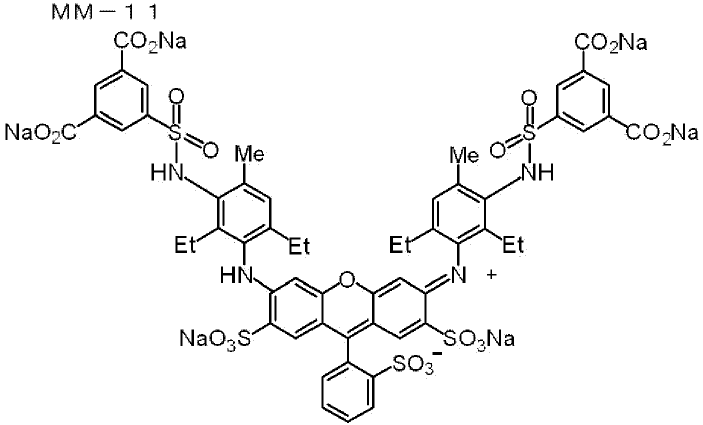

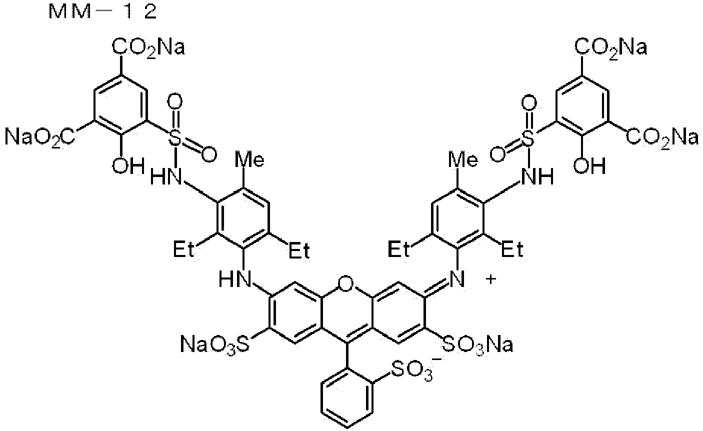

In the magenta ink composition, the ratio of the mass of lithium with respect to the total mass of alkali metals is preferably 70% by mass or more, more preferably 75% by mass or more, and even more preferably 80% by mass or more.

In a case where the mass ratio of lithium is 70% by mass or more, a viscosity increase phenomenon in a case of being mixed with an ink composition (particularly the yellow ink composition) other than the magenta ink composition is effectively suppressed. Accordingly, it is possible to suppress ink stains on the recording medium caused by the accumulation of the ink composition.

The magenta ink composition in the present disclosure contains a solvent A, a solvent B, a solvent C, and a solvent D.

The aspects of preferable solvents in the magenta ink composition are the same as the preferable aspects of the solvents in the yellow ink composition, among these, it is preferable that the solvent A is 2-ethyl-1,3-hexanediol, the solvent B is 2-pyrrolidone, the solvent C is 1,3-butanediol, and the solvent D is glycerin.

### (3) Cyan Ink Composition

The cyan ink composition in the present disclosure contains at least one compound (also referred to as a cyan dye) selected from the group consisting of a compound represented by Formula (C) and a salt thereof, a solvent A, a solvent B, a solvent C, a solvent D, and water, and may further contain other components such as a surfactant and an additive, as necessary.

The solvent A, the solvent B, the solvent C, the solvent D, and the water in the cyan ink composition are the same as those in the above-described yellow ink composition, and the preferable aspects are also the same. Therefore, the detailed description in this section is omitted.

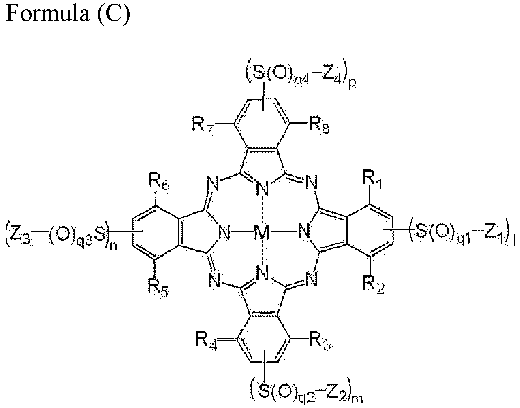

For the details of Formula (C), the descriptions of paragraphs 0608 to 0618, paragraph 0586, and paragraphs 0601 to 0603 of JP2007-138124A can be referred to.

In Formula (C), R₁, R₂, R₃, R₄, R₅, R₆, R₇, and R₈ each independently represent a hydrogen atom, a halogen atom, an alkyl group, a cycloalkyl group, an alkenyl group, an aralkyl group, an aryl group, a heterocyclic group, a cyano group, a hydroxy group, a nitro group, an amino group, an alkylamino group, an alkoxy group, an aryloxy group, an amide group, an arylamino group, a ureido group, a sulfamoylamino group, an alkylthio group, an arylthio group, an alkoxycarbonylamino group, a sulfonamide group, a carbamoyl group, a sulfamoyl group, a sulfinyl group, a sulfonyl group, an alkoxycarbonyl group, a heterocyclic oxy group, an azo group, an acyloxy group, a carbamoyloxy group, a silyloxy group, an aryloxycarbonyl group, an aryloxycarbonylamino group, an imide group, a heterocyclic thio group, a phosphoryl group, an acyl group, or an ionic hydrophilic group, and each group may further have a substituent.

R₁, R₂, R₃, R₄, R₅, R₆, R₇ and R₈ each independently preferably represent a hydrogen atom, a halogen atom, an alkyl group, an aryl group, a heterocyclic group, a cyano group, a hydroxy group, a nitro group, a carbamoyl group, a sulfamoyl group, a sulfinyl group, a sulfonyl group, an alkoxycarbonyl group, an aryloxycarbonyl group, a phosphoryl group, an acyl group or an ionic hydrophilic group, and a hydrogen atom, more preferably represent a halogen atom, a cyano group, a hydroxy group, a sulfamoyl group, a sulfinyl group, a sulfonyl group, or an ionic hydrophilic group, and even more preferably represent a hydrogen atom.

Z₁, Z₂, Z₃, and Z₄ each independently represent an alkyl group, a cycloalkyl group, an alkenyl group, an alkynyl group, an aralkyl group, an aryl group, or a heterocyclic group, and each group may further have a substituent. At least one of Z₁, Z₂, Z₃, or Z₄ has an ionic hydrophilic group as a substituent.

Z₁, Z₂, Z₃, and Z₄ each independently represent a substituted or unsubstituted alkyl group, a substituted or unsubstituted cycloalkyl group, a substituted or unsubstituted alkenyl group, a substituted or unsubstituted alkynyl group, a substituted or unsubstituted aralkyl group, a substituted or unsubstituted aryl group, or a substituted or unsubstituted heterocyclic group, preferably represent a substituted or unsubstituted alkyl group, a substituted or unsubstituted aryl group, or a substituted or unsubstituted heterocyclic group, more preferably represent a substituted alkyl group, a substituted aryl group, and a substituted heterocyclic group, and even more preferably represent a substituted alkyl group.

However, at least one of Z₁, Z₂, Z₃, or Z₄ has an ionic hydrophilic group as a substituent.

l, m, n, p, q1, q2, q3 and q4 each independently represent an integer of 1 or 2.

Among these, at least two of l, m, n, and p are preferably 1, and more preferably, 1 = m = n = p = 1.

Further, at least two of q1, q2, q3, and q4 are preferably 2, and more preferably q1 = q2 = q3 = q4 = 2.

M represents a metal atom or a metal oxide, hydroxide or halide.

Examples of metal atoms in M include Li, Na, K, Mg, Ti, Zr, V, Nb, Ta, Cr, Mo, W, Mn, Fe, Co, Ni, Ru, Rh, Pd, Os, Ir, Pt, Cu, Ag, Au, Zn, Cd, Hg, Al, Ga, In, Si, Ge, Sn, Pb, Sb, and Bi. Examples of the oxide include VO and GeO. Examples of the hydroxide include Si(OH)₂, Cr(OH)₂, and Sn(OH)₂. Examples of the halide include AlCl, SiCl₂, VCl, VCl₂, VOCl, FeCl, GaCl, and ZrCl.

Among these, Cu, Ni, Zn, and Al are preferable, and Cu is more preferable.

In addition, Pc (phthalocyanine ring) may form a dimer (for example, Pc-M-L-M-Pc) or a trimer via L (a divalent linking group). In this case, M's may be the same as or different from each other.

It is preferable that the divalent linking group represented by L includes an oxy group (-O-), a thio group (-S-), a carbonyl group (-CO-), a sulfonyl group (-SO₂-), an imino group (-NH-), a methylene group (-CH₂-), and a group obtained by combining at least two of these.

Among the compounds represented by Formula (C), a compound selected from the group consisting of a compound represented by Formula (C-1) below and a salt thereof is preferable.

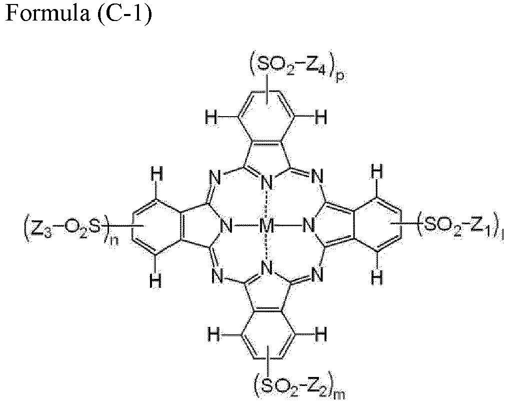

For the details of Formula (C-1), the description of paragraphs 0620 to 0626 of JP2007-138124A can be referred to.

In Formula (C-1), Z₁, Z₂, Z₃, Z₄, l, m, n, p, and M are the same as Z₁, Z₂, Z₃, Z₄, l, m, n, p, and M in Formula (C).

In Formula (C-1), n and p are each independently an integer of 1 or 2.

Among these, at least two or more of 1, m, n, and p are preferably 1, and more preferably l = m = n = p = 1.

Z₁, Z₂, Z₃, and Z₄ each independently represent a substituted or unsubstituted alkyl group, a substituted or unsubstituted cycloalkyl group, a substituted or unsubstituted alkenyl group, a substituted or unsubstituted alkynyl group, a substituted or unsubstituted group aralkyl group, a substituted or unsubstituted aryl group, or a substituted or unsubstituted heterocyclic group, preferably represent a substituted or unsubstituted alkyl group, a substituted or unsubstituted aryl group, a substituted or unsubstituted heterocyclic group. Among these, a substituted alkyl group, a substituted aryl group, and a substituted heterocyclic group are preferable, and a substituted alkyl group is more preferable.

Z₁, Z₂, Z₃, and Z₄ each independently represent Z₁₁ (Z₁₁ represents -(CH₂)₃SO₃M₂ and M₂ represents an alkali metal atom), and/or Z₁₂ (Z₁₂ represents -(CH₂)₃SO₂NHCH₂CH(OH)CH₃), and particularly, a mixture of dyes having a molar ratio of Z₁₁ and Z₁₂ included in the entire cyan dye represented by Formula (C-1) being Z₁₁/Z₁₂ = 4/0, 3/1, 2/2, 1/3 is preferable, and a dye mixture having Z₁₁/Z₁₂ = 3/1 as a main component and/or a dye mixture having Z₁₁/Z₁₂ = 2/2 as a main component are more preferable. However, at least one of Z₁, Z₂, Z₃, or Z₄ has an ionic hydrophilic group as a substituent.

In "-(CH₂)₃SO₃M₂" for Z₁₁, M₂ is preferably an alkali metal atom, more preferably a lithium ion, a sodium ion, or a potassium ion, and even more preferably a lithium ion.

M is the same as M in Formula (C), and the preferable aspect is also the same.

Specific examples of the compound represented by Formula (C) and a salt thereof are shown below. However, the present disclosure is not limited to the following specific examples.

(CYAN-1) 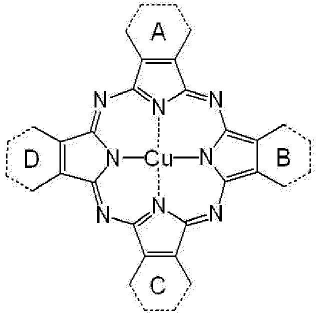 One of rings A to D is 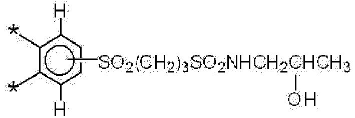 The remaining three rings are 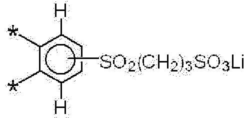 *means a bonding position of a phthalocyanine ring.
(CYAN-2) 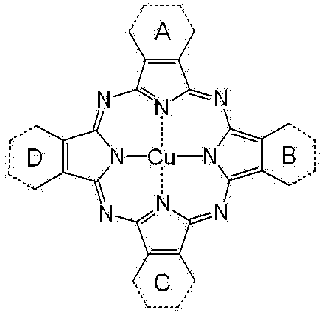 Two of rings A to D are 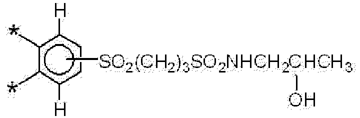 The remaining two rings are 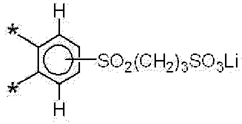 *means a bonding position of a phthalocyanine ring.
(CYAN-3) 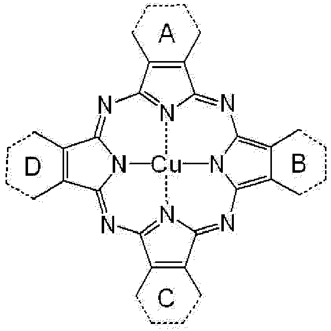 One of rings A to D is 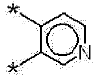 The remaining three rings are 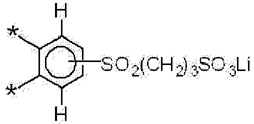 *means a bonding position of a phthalocyanine ring.
(CYAN-4) 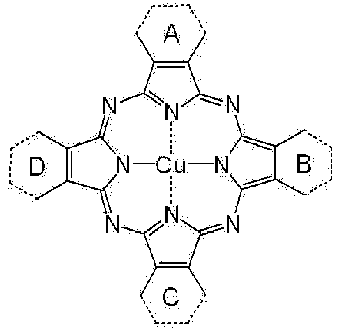 One of rings A to D is 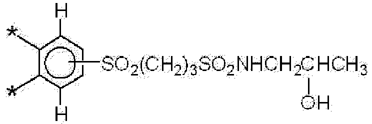 The remaining three rings are 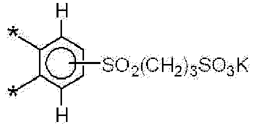 *means a bonding position of a phthalocyanine ring.

In the cyan ink composition, the ratio of the mass of lithium with respect to the total mass of alkali metals is preferably 70% by mass or more, more preferably 75% by mass or more, even more preferably 80% by mass or more, and particularly preferably 90% by mass or more.

In a case where the mass ratio of lithium is 70% by mass or more, a viscosity increase phenomenon in a case of being mixed with an ink composition (particularly, yellow ink composition) other than the cyan ink composition is effectively suppressed. Accordingly, it is possible to suppress ink stains on the recording medium caused by the accumulation of the ink composition.

The cyan ink composition in the present disclosure contains a solvent A, a solvent B, a solvent C, and a solvent D.

The combination of preferable solvents in the cyan ink composition is the same as the combination of the preferable solvents in the yellow ink composition, among these, it is preferable that the solvent A is 2-ethyl-1,3-hexanediol, the solvent B is 2-pyrrolidone, the solvent C is 1,3-butanediol, and the solvent D is glycerin.

### (4) Black Ink Composition

The black ink composition in the present disclosure contains at least one compound (also referred to as a black dye) selected from the group consisting of a compound represented by Formula (B) and a salt thereof, a solvent A, a solvent B, a solvent C, a solvent D, and water, and preferably contains at least one compound selected from the group consisting of a compound represented by Formula (B-1) and a salt thereof. The black ink composition may further contain other components such as a surfactant and an additive, as necessary.

The solvent A, the solvent B, the solvent C, the solvent D, and the water in the black ink composition are the same as those in the above-described yellow ink composition, and the preferred aspects are also the same. Therefore, the detailed description in this section is omitted.

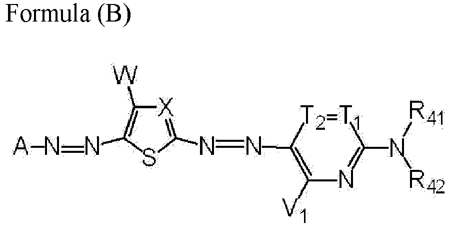

For the details of Formula (B), the description of paragraphs 0690 to 0764 and 0673 to 0687 of JP2007-138124A can be referred to.

In Formula (B), A represents an aromatic group that may be substituted or a heterocyclic group that may be substituted.

X represents a nitrogen atom or = C(W₁)-, and W₁ represents an electron-withdrawing group having a Hammett's substituent constant σₚ value of 0.20 or more.

T₁ and T₂ each represent = C(R₄₃)-and -C(R₄₄) =, or one of T₁ or T₂ represents a nitrogen atom, and the other represents = C(R₄₃)-or -C(R₄₄)=.

V₁, W, R₄₃, and R₄₄ each independently represent a hydrogen atom, a halogen atom, an alkyl group, an alkenyl group, an alkynyl group, an aralkyl group, an aryl group, a heterocyclic group, a cyano group, a carboxy group, a carbamoyl group, or an alkoxycarbonyl group, an aryloxycarbonyl group, a heterocyclic oxycarbonyl group, an acyl group, a hydroxy group, an alkoxy group, an aryloxy group, a heterocyclic oxy group, a silyloxy group, an acyloxy group, a carbamoyloxy group, an alkoxycarbonyloxy group, an aryloxycarbonyloxy group, an amino group (including an alkylamino group, an arylamino group, and a heterocyclic amino group), an acylamino group, a ureido group, a sulfamoylamino group, an alkoxycarbonylamino group, an aryloxycarbonylamino group, an alkylsulfonylamino group, an arylsulfonylamino group, a heterocyclic sulfonylamino group, a nitro group, an alkylarylthio group, an arylthio group, a heterocyclic thio group, an alkylsulfonyl group, an arylsulfonyl group, a heterocyclic sulfonyl group, an alkylsulfinyl group, an arylsulfinyl group, a heterocyclic sulfinyl group, a sulfamoyl group, or a sulfo group, and each group may be further substituted.

R₄₁ and R₄₂ each independently represent a hydrogen atom, an alkyl group, an alkenyl group, an alkynyl group, an aralkyl group, an aryl group, a heterocyclic group, an acyl group, an alkoxycarbonyl group, an aryloxycarbonyl group, a carbamoyl group, an alkylsulfonyl group, an arylsulfonyl group, or a sulfamoyl group, and each group may be further substituted.

However, R₄₁ and R₄₂ do not represent a hydrogen atom at the same time.

In addition, R₄₃ and R₄₁, or R₄₁ and R₄₂ may be bonded to each other to form a 5-membered or 6-membered ring.

Among the compounds represented by Formula (B), a compound selected from the group consisting of a compound represented by Formula (B-1) or (B-2) below and a salt thereof is preferable.

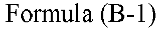

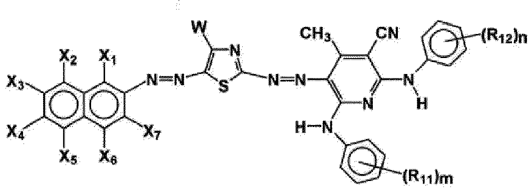

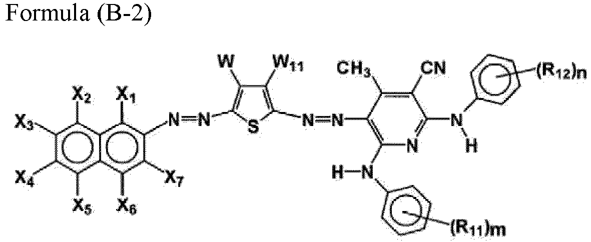

First, Formula (B-1) will be described in detail.

In Formula (B-1), R₁₁ and R₁₂ each independently represent an ionic hydrophilic group, preferably represent a sulfo group and a salt of a sulfo group, a carboxy group and a salt of a carboxy group, and more preferably represent a sulfo group. m and n each independently represent an integer of 1 to 3, preferably represent an integer of 1 to 2, and more preferably satisfy at least one of m = 1 or n = 1.

(A) X₁, X₂, X₃, X₄, X₅, X₆, and X₇ each independently represent a hydrogen atom, a halogen atom, an alkyl group, an aryl group, a heterocyclic group, a cyano group, an alkoxy group, an amide group, or a ureido group, an alkylsulfonylamino group, an arylsulfonylamino group, a sulfamoyl group, an alkylsulfonyl group, an arylsulfonyl group, a carbamoyl group, an alkoxycarbonyl group, a sulfo group (including a salt thereof), a carboxy group (including a salt thereof), and a hydroxyl group (which may be a salt), a phosphono group (which may be a salt) or a quaternary ammonium, among these, a hydrogen atom, a halogen atom, an alkyl group, a sulfo group (including a salt thereof), a carboxy group (including a salt thereof), and a hydroxyl group (which may be a salt) are preferable, a hydrogen atom, a sulfo group (including a salt thereof) and a carboxy group (including a salt thereof) are more preferable, and it is particularly preferable that at least one of X₁, X₂, X₃, X₄, X₅, X₆, or X₇ is a sulfo group (including a salt thereof) or a carboxy group (including a salt thereof).

(B) W represents a substituted phenyl group, a substituted or unsubstituted naphthyl group, a substituted or unsubstituted heterocyclic group (for example, a pyrrole ring, a thiophene ring, an imidazole ring, a thiazole ring, a benzothiazole ring, a pyridine ring, or a pyridazine ring), and a substituted phenyl group (particularly a phenyl group substituted at the para-position), a substituted or unsubstituted β-naphthyl group, a pyridine ring or a thiazole ring is preferable.

(C) R₄₁ and R₄₂ are each independently preferably represent a hydrogen atom, an alkyl group, an aryl group, a heterocyclic group, an acyl group, or an alkylsulfonyl or arylsulfonyl group, more preferably represent a hydrogen atom, an alkyl group, an aryl group, or a heterocyclic group, even more preferably represent a hydrogen atom, an aryl group having a substituent, or a heterocyclic group having a substituent, and particularly preferably represent a hydrogen atom or an aryl group having a substituent. However, R₄₁ and R₄₂ do not represent a hydrogen atom at the same time. R₄₃ and R₄₁, or R₄₁ and R₄₂ may be bonded to each other to form a 5-membered or 6-membered ring.

(D) R₄₃ and R₄₄ each independently represent a hydrogen atom, a halogen atom, an alkyl group, an alkenyl group, an alkynyl group, an aralkyl group, an aryl group, a heterocyclic group, a cyano group, a carboxy group, a carbamoyl group, an alkoxycarbonyl group, an aryloxycarbonyl group, a heterocyclic oxycarbonyl group, a hydroxy group, an amino group (including an alkylamino group, an arylamino group, and a heterocyclic amino group), an acylamino group, a ureido group, a sulfamoylamino group, an alkoxycarbonylamino group, an aryloxycarbonylamino group, an alkylsulfonylamino or arylsulfonylamino group, or a heterocyclic sulfonylamino group. Each group may be further substituted.

As R₄₃, a hydrogen atom, a halogen atom, an aryl group, a heterocyclic group, a cyano group, a carboxy group, a carbamoyl group, an alkoxycarbonyl group, an aryloxycarbonyl group, and a heterocyclic oxycarbonyl group are preferable, a cyano group, a carboxy group, or a carbamoyl group, and an alkoxycarbonyl group are more preferable, and a cyano group is even more preferable.

As R₄₄, a hydrogen atom, a halogen atom, an alkyl group, an alkenyl group, an alkynyl group, an aralkyl group, an aryl group, a heterocyclic group, a hydroxy group, an amino group (including an alkylamino group, an arylamino group, and a heterocyclic amino group), an acylamino group, a ureido group, a sulfamoylamino group, an alkoxycarbonylamino group, an aryloxycarbonylamino group, an alkylsulfonylamino or arylsulfonylamino group, and a heterocyclic sulfonylamino group are preferable, a hydrogen atom, a halogen atom, an alkyl group, and an alkenyl group, an alkynyl group, an aralkyl group, and an aryl group are more preferable, and a methyl group is even more preferable.

(E) R₄₅ and R₄₆ are each independently preferably represent a hydrogen atom, an alkyl group, an aryl group, a heterocyclic group, an acyl group, or an alkylsulfonyl or an arylsulfonyl group, more preferably represent a hydrogen atom, an alkyl group, an aryl group, or a heterocyclic group, particularly preferably represent a hydrogen atom, an aryl group having a substituent, or a heterocyclic group having a substituent, and most preferably represent a hydrogen atom or an aryl group having a substituent. However, R₄₅ and R₄₆ do not represent a hydrogen atom at the same time. R₄₅ and R₄₆ may be bonded to form a 5-membered or 6-membered ring.

As a preferred aspect of Formula (B-1), a case where all of the above (A) to (E) are satisfied is particularly preferable.

W preferably represents a substituted phenyl group, a substituted or unsubstituted naphthyl group, a substituted or unsubstituted heterocyclic group (for example, a pyrrole ring group, a thiophene ring group, an imidazole ring group, a thiazole ring group, a benzothiazole ring group, a pyridine ring group, or a pyridazine ring group), and more preferably represents a substituted phenyl group (particularly a phenyl group substituted at the para-position), a substituted or unsubstituted β-naphthyl group, a pyridine ring or a thiazole ring.

Next, Formula (B-2) will be described in detail.

In Formula (B-2), R₁₁ and R₁₂ each independently represent an ionic hydrophilic group, preferably represent a sulfo group (including a salt thereof), a carboxy group (including a salt thereof), and more preferably represent a sulfo group. m and n are each independently preferably an integer of 1 to 3 and more preferably an integer of 1 to 2, and a case where at least one of m = 1 or n = 1 is satisfied is even more preferable.
(A) X₁, X₂, X₃, X₄, X₅, X₆, and X₇ each independently represent a hydrogen atom, a halogen atom, an alkyl group, an aryl group, a heterocyclic group, a cyano group, an alkoxy group, an amide group, or a ureido group, an alkylsulfonylamino group, an arylsulfonylamino group, a sulfamoyl group, an alkylsulfonyl group, an arylsulfonyl group, a carbamoyl group, an alkoxycarbonyl group, a sulfo group (including a salt thereof), a carboxy group (including a salt thereof), and a hydroxyl group (which may be a salt), a phosphono group (which may be a salt) or a quaternary ammonium, among these, a hydrogen atom, a halogen atom, an alkyl group, a sulfo group (including a salt thereof), a carboxy group (including a salt thereof), and a hydroxyl group (which may be a salt) are preferable, a hydrogen atom, a sulfo group (including a salt thereof) and a carboxy group (including a salt thereof) are more preferable, and it is even more preferable that at least one of X₁, X₂, X₃, X₄, X₅, X₆, or X₇ is a sulfo group (including a salt thereof) or a carboxy group (including a salt thereof).
(B) W represents a substituted phenyl group, a substituted or unsubstituted naphthyl group, a substituted or unsubstituted heterocyclic group (for example, a pyrrole ring group, a thiophene ring group, an imidazole ring group, a thiazole ring group, a benzothiazole ring group, a pyridine ring group, or a pyridazine ring group), and preferably represents a substituted phenyl group (particularly a phenyl group substituted at the para-position), a substituted or unsubstituted β-naphthyl group, a pyridine ring or a thiazole ring.
(C) W₁₁ represents an electron-withdrawing group having a Hammett's substituent constant σₚ value of 0.20 or more, an electron-withdrawing group having a σₚ value of 0.30 or more is preferable, an electron-withdrawing group having a σₚ value of 0.45 or more is more preferable, and an electron-withdrawing group having a σₚ value of 0.60 or more is even more preferable. It is desirable that the σₚ value does not exceed 1.0. In the above description, as W₁₁, an acyl group having 2 to 20 carbon atoms, an alkyloxycarbonyl group having 2 to 20 carbon atoms, a nitro group, a cyano group, an alkylsulfonyl group having 1 to 20 carbon atoms, an arylsulfonyl group having 6 to 20 carbon atoms, a carbamoyl group having 1 to 20 carbon atoms, and a halogenated alkyl group having 1 to 20 carbon atoms are preferable, a cyano group, an alkylsulfonyl group having 1 to 20 carbon atoms, and an arylsulfonyl group having 6 to 20 carbon atoms are more preferable, and a cyano group is particularly preferable.
(D) R₄₁ and R₄₂ are each independently preferably represent a hydrogen atom, an alkyl group, an aryl group, a heterocyclic group, an acyl group, or an alkylsulfonyl or arylsulfonyl group, more preferably represent a hydrogen atom, an alkyl group, an aryl group, or a heterocyclic group, even more preferably represent a hydrogen atom, an aryl group having a substituent, or a heterocyclic group having a substituent, and particularly preferably represent a hydrogen atom or an aryl group having a substituent. However, R₄₁ and R₄₂ do not represent a hydrogen atom at the same time. R₄₃ and R₄₁, or R₄₁ and R₄₂ may be bonded to each other to form a 5-membered or 6-membered ring.
(E) R₄₃ and R₄₄ each independently represent a hydrogen atom, a halogen atom, an alkyl group, an alkenyl group, an alkynyl group, an aralkyl group, an aryl group, a heterocyclic group, a cyano group, a carboxy group, a carbamoyl group, an alkoxycarbonyl group, an aryloxycarbonyl group, a heterocyclic oxycarbonyl group, a hydroxy group, an amino group (including an alkylamino group, an arylamino group, and a heterocyclic amino group), an acylamino group, a ureido group, a sulfamoylamino group, an alkoxycarbonylamino group, an aryloxycarbonylamino group, an alkylsulfonylamino group, an arylsulfonylamino group, or a heterocyclic sulfonylamino group. Each group may be further substituted.

As R₄₃, a hydrogen atom, a halogen atom, an aryl group, a heterocyclic group, a cyano group, a carboxy group, a carbamoyl group, an alkoxycarbonyl group, an aryloxycarbonyl group, and a heterocyclic oxycarbonyl group are preferable, a cyano group, a carboxy group, or a carbamoyl group, and an alkoxycarbonyl group are more preferable, and a cyano group is even more preferable.

As R₄₄, a hydrogen atom, a halogen atom, an alkyl group, an alkenyl group, an alkynyl group, an aralkyl group, an aryl group, a heterocyclic group, a hydroxy group, an amino group (including an alkylamino group, an arylamino group, and a heterocyclic amino group), an acylamino group, a ureido group, a sulfamoylamino group, an alkoxycarbonylamino group, an aryloxycarbonylamino group, an alkylsulfonylamino or arylsulfonylamino group, and a heterocyclic sulfonylamino group are preferable, a hydrogen atom, a halogen atom, an alkyl group, and an alkenyl group, an alkynyl group, an aralkyl group, and an aryl group are more preferable, and a methyl group is even more preferable.

(F) R₄₅ and R₄₆ are each independently preferably represent a hydrogen atom, an alkyl group, an aryl group, a heterocyclic group, an acyl group, or an alkylsulfonyl or arylsulfonyl group, more preferably represent a hydrogen atom, an alkyl group, an aryl group, or a heterocyclic group, even more preferably represent a hydrogen atom, an aryl group having a substituent, or a heterocyclic group having a substituent, and particularly preferably represent a hydrogen atom or an aryl group having a substituent. However, R₄₅ and R₄₆ do not represent a hydrogen atom at the same time. R₄₅ and R₄₆ may be bonded to form a 5-membered or 6-membered ring.

As a preferred aspect of Formula (B-2), a case where all of the above (A) to (F) are satisfied is particularly preferable.

Specific examples of the compound represented by Formula (B) and a salt thereof are shown below. However, the present disclosure is not limited to the following specific examples.

(BLACK- 1) 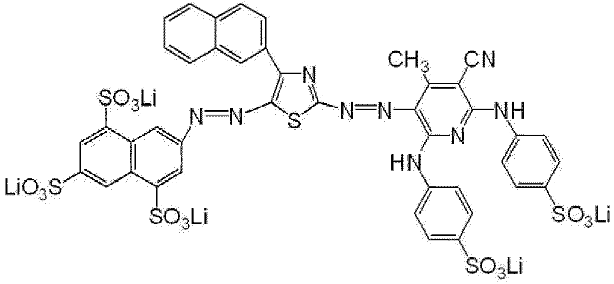
(BLACK-2) 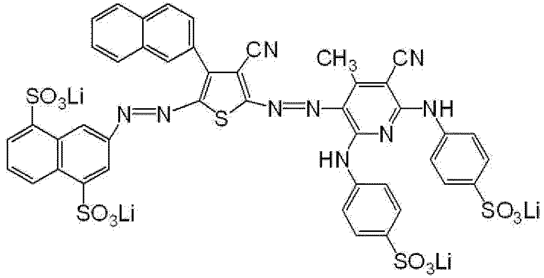
(BLACK-3)

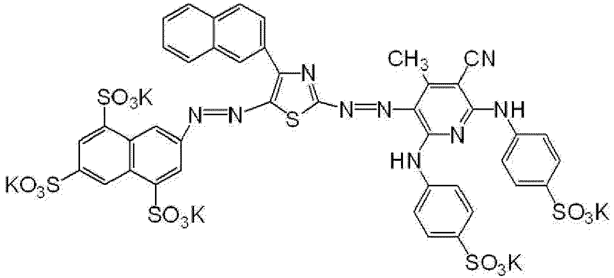

It is preferable that the black ink composition further contains a compound represented by Formula (BA) below. By containing the compound represented by Formula (BA), the tint of black can be adjusted.

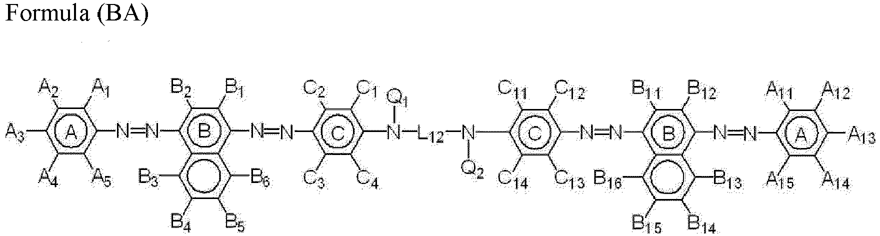

For the details of Formula (BA), the descriptions of paragraphs 0789 to 0792 of JP2007-138124A can be referred to.

In Formula (BA), ring A, ring B, and ring C each independently represent a substituted or unsubstituted aryl group or a substituted or unsubstituted heterocyclic group.

Q₁ and Q₂ each independently represent a hydrogen atom, an ionic hydrophilic group, a halogen atom, a cyano group, a nitro group, a substituted or unsubstituted acylamino group, a substituted or unsubstituted sulfonylamino group, a substituted or unsubstituted alkyl group, a substituted or unsubstituted alkoxy group, a substituted or unsubstituted aryl group, a substituted or unsubstituted heterocyclic group, a substituted or unsubstituted alkylsulfonyl group, a substituted or unsubstituted arylsulfonyl group, a substituted or unsubstituted sulfamoyl group, or a substituted or unsubstituted carbamoyl group.

L₁₂ represents a divalent linking group, and is preferably a carbonyl group or a substituted or unsubstituted heterocyclic group (for example, a substituted or unsubstituted triazine ring group).

A₁, A₂, A₃, A₄, A₅, A₁₁, A₁₂, A₁₃, A₁₄, A₁₅, B₁, B₂, B₃, B₄, B₅, B₆, B₁₁, B₁₂, B₁₃, B₁₄, B₁₅, B₁₆, C₁, C₂, C₃, C₄, C₁₁, C₁₂, C₁₃, and C₁₄ each independently represent a hydrogen atom, an ionic hydrophilic group, a halogen atom, a cyano group, a nitro group, a substituted or unsubstituted acylamino group, a substituted or unsubstituted sulfonylamino group, a substituted or unsubstituted alkyl group, a substituted or unsubstituted alkoxy group, a substituted or unsubstituted aryl group, a substituted or unsubstituted heterocyclic group, a substituted or unsubstituted alkylsulfonyl group, a substituted or unsubstituted arylsulfonyl group, a substituted or unsubstituted sulfamoyl group, or a substituted or unsubstituted carbamoyl group.

Among these, a ionic hydrophilic group, a halogen atom, a cyano group, a nitro group, a substituted or unsubstituted acylamino group, a substituted or unsubstituted alkyl group, a substituted or unsubstituted alkoxy group, a substituted or unsubstituted alkylsulfonyl group, a substituted or unsubstituted arylsulfonyl group, and a substituted or unsubstituted sulfamoyl group are preferable, an ionic hydrophilic group, a halogen atom, a cyano group, a nitro group, a substituted or unsubstituted acylamino group, a substituted or unsubstituted alkyl group are more preferable, and an ionic hydrophilic group is even more preferable.

As the ionic hydrophilic group, a sulfo group (including a salt thereof) and a carboxy group (including a salt thereof) are preferable, and a sulfo group is more preferable.

However, at least one of A₁ to A₅, A₁₁ to A₁₅, B₁ to B₆, B₁₁ to B₁₆, C₁ to C₄, C₁₁ to C₁₄, Q₁, Q₂, or L₁₂ has at least one ionic hydrophilic group.

Specific examples (BA-1 and BA-2) of the compound represented by formula (BA) and a salt thereof are shown below. However, the present disclosure is not limited to the following specific examples.

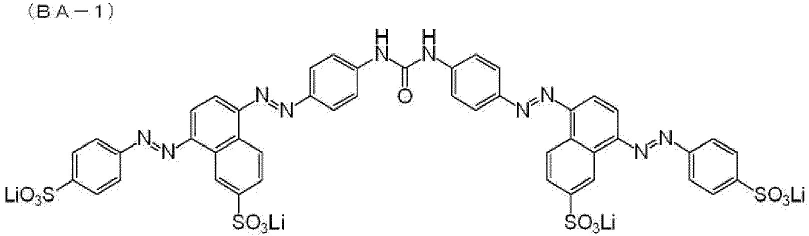

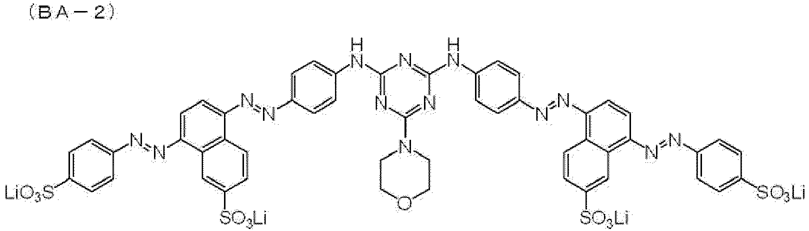

The ratio of the mass of lithium with respect to the total mass of alkali metals in the black ink composition is preferably 70% by mass or more, and more preferably 75% by mass or more.

In a case where the mass ratio of lithium is 70% by mass or more, a viscosity increase phenomenon in a case of being mixed with an ink composition (particularly, yellow ink composition) other than the black ink composition is effectively suppressed. Accordingly, it is possible to suppress ink stains on the recording medium caused by the accumulation of the ink composition.

The black ink composition in the present disclosure contains a solvent A, a solvent B, a solvent C, and a solvent D.

The combination of preferable solvents in the black ink composition is the same as the combination of the preferable solvents in the yellow ink composition, among these, it is preferable that the solvent A is 2-ethyl-1,3-hexanediol, the solvent B is 2-pyrrolidone, the solvent C is 1,3-butanediol, and the solvent D is glycerin.

### -Physical Properties of Ink Composition-

The physical properties of the yellow ink composition, the cyan ink composition, the magenta ink composition and the black ink composition in the present disclosure are not particularly limited, but preferably have the following physical properties.

### -pH-

The yellow ink composition, the cyan ink composition, the magenta ink composition, and the black ink composition in the present disclosure preferably have a pH of 6.5 to 11 at 25°C (± 1°C) and more preferably have a pH of 7.0 to 10.5.

### -Viscosity-

The viscosity at 25°C (± 1°C) of the yellow ink composition, the cyan ink composition, the magenta ink composition, and the black ink composition in the present disclosure is preferably in a range of 6 mPa·s to 10 mPa·s, and more preferably in a range of 7 mPa's to 9 mPa s.

The viscosity is measured under the condition at a temperature of 25°C using VISCOMETER TV-22 (manufactured by TOKI SANGYO CO. LTD).

### -Surface Tension-

The surface tension at 25°C (± 1°C) of the yellow ink composition, the cyan ink composition, the magenta ink composition, and the black ink composition in the present disclosure is preferably 60 mN/m or less, more preferably 30 mN/m to 50 mN/m, and even more preferably 35 mN/m to 45 mN/m.

The surface tension is measured using Automatic Surface Tensiometer CBVP-Z (manufactured by Kyowa Interface Science Co., Ltd.) at 25°C by a plate method.

In the present disclosure, for example, the yellow ink composition, the cyan ink composition, the magenta ink composition, and the black ink composition may be prepared by mixing and dissolving various components contained in the ink composition to maintain good uniformity, then subjecting the resultant to pressure filtration with a membrane filter having a pore diameter of 0.8 µm, and degassing the obtained solution using a vacuum pump. However, the ink composition in the present disclosure is not limited to the above method.

### <Ink Jet Recording Method>

An ink jet recording method according to the present disclosure records an image using the above-described ink set according to the present disclosure.

The inkjet recording method is a method in which an ink composition is jetted as liquid droplets from a thin nozzle, and the jetted liquid droplets are attached to a recording medium. Examples of the method include a method using an electrostatic attraction method, a method of applying pressure and mechanical vibration to an ink composition to jet the ink composition, a method using a piezoelectric element, and a method in which an ink composition is heated by an electrode according to print signal information to cause foaming and foam expansion. For the details of the ink jet recording method, the description in paragraphs 0560 to 0564 of JP2012-193330A can be referred to.

### Examples

Hereinafter, the present invention will be described more specifically with reference to examples. However, the present invention is not limited to the following examples as long as the present invention does not depart from the scope of the present claims.

### (Example 1)

### [Preparation of Yellow Ink-1]

The following components in the following composition were mixed to prepare yellow ink-1.

### -Composition of Yellow Ink-1-

| | |
|---|---|
| ·YELLOW-1 | 4.5% by mass |
| ·2-ethyl-1,3-hexanediol (solvent A) | 2.0% by mass |
| ·2-pyrrolidone (solvent B) | 1.0% by mass |
| ·1,3-butanediol (solvent C) | 25.0% by mass |
| ·Glycerin (solvent D) | 20.0% by mass |
| ·Sodium hydrogen carbonate | 0.01% by mass |
| ·Proxel GXL | 0.03% by mass (manufactured by Arch Chemicals Japan K.K., preservative) |
| ·Ion exchange water . . . | remaining amount in a case where the total amount is 100% by mass |

### (YELLOW-1)

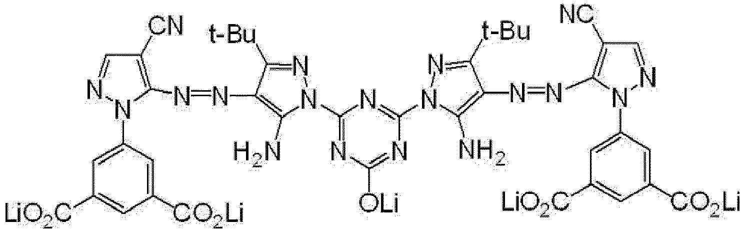

Here, a method of synthesizing the YELLOW-1 dye will be described.

The YELLOW-1 dye as a Li salt was synthesized with reference to the synthesis method of Synthesis Example 1 described in paragraphs 0864 to 0870 of JP4977371B. Here, KOH used in forming a 10% by mass aqueous solution of potassium hydroxide (KOH) as an aqueous solution of the obtained crystals was changed to lithium hydroxide (LiOH) to obtain a Li salt. A mixture of a K salt and a Li salt may be used.

### [Preparation of Magenta Ink-1]

The components in the following composition were mixed to prepare magenta ink-1.

### -Composition of Magenta Ink-1-

| | |
|---|---|
| ·MAGENTA-1 | 4.0% by mass |
| ·2-ethyl-1,3-hexanediol (solvent A) | 2.0% by mass |
| ·2-amino-2-ethyl-1,3-propanediol (solvent B) | 0.03% by mass |
| ·2-pyrrolidone (solvent B) | 1.0% by mass |
| ·1,3-butanediol (solvent C) | 35.0% by mass |
| ·Glycerin (solvent D) | 13.0% by mass |
| ·Sodium hydrogen carbonate | 0.04% by mass |
| ·Proxel GXL | 0.05% by mass (manufactured by Arch Chemicals Japan K.K., preservative) |
| Ion exchange water . . | remaining amount in a case where the total amount is 100% by mass |

·

### (MAGENTA-1)

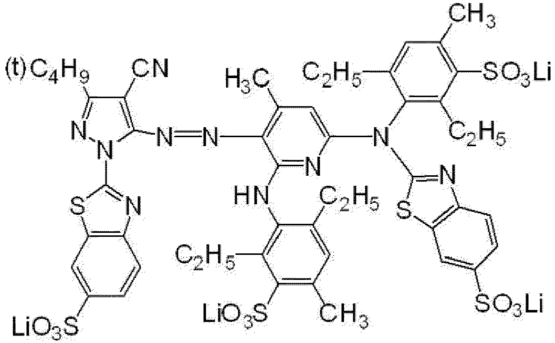

### [Preparation of Cyan Ink-1]

The components in the following composition were mixed to prepare cyan ink-1.

### -Composition of Cyan Ink-1-

| | |
|---|---|
| ·CYAN-1 | 5.0% by mass |
| ·2-ethyl-1,3-hexanediol (solvent A) | 2.0% by mass |
| ·2-amino-2-ethyl-1,3-propanediol (solvent B) | 0.05% by mass |
| ·2-pyrrolidone (solvent B) | 1.0% by mass |
| ·1,3-butanediol (solvent C) | 32.0% by mass |
| ·Glycerin (solvent D) | 13.0% by mass |
| ·Sodium hydrogen carbonate | 0.01% by mass |
| ·Proxel GXL | 0.03% by mass (manufactured by Arch Chemicals Japan K.K., preservative) |
| Ion exchange water . . . | remaining amount in a case where the total amount is 100% by mass |

·

### (CYAN-1)

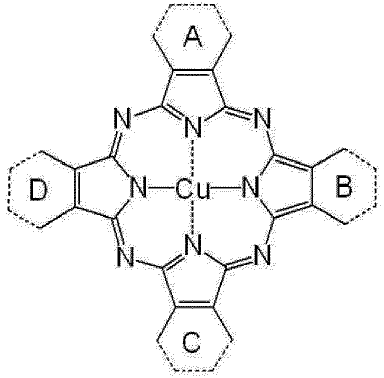

One of rings A to D is 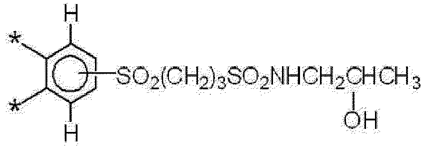 The remaining three rings are 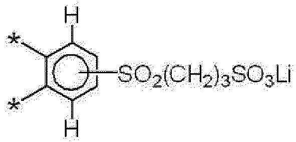 *means a bonding position of a phthalocyanine ring.

### [Preparation of Black Ink-1]

The following components in the following composition were mixed to prepare black ink-1.

### -Composition of Black Ink-1-

| | |
|---|---|
| ·BLACK-1 | 4.5% by mass |
| ·BA-1 (compound represented by Formula (BA)) | 0.5% by mass |
| ·2-ethyl-1,3-hexanediol (solvent A) | 2.0% by mass |
| ·2-pyrrolidone (solvent B) | 2.0% by mass |
| ·1,3-butanediol (solvent C) | 32.0% by mass |
| ·Glycerin (solvent D) | 12.0% by mass |
| ·Potassium hydrogen carbonate | 0.04% by mass |
| ·Sodium hydrogen carbonate | 0.01% by mass |
| ·Proxel GXL | 0.08% by mass (manufactured by Arch Chemicals Japan K.K., preservative) |
| ·Ion exchange water . . . | remaining amount in a case where the total amount is 100% by mass |

### (BLACK-1)

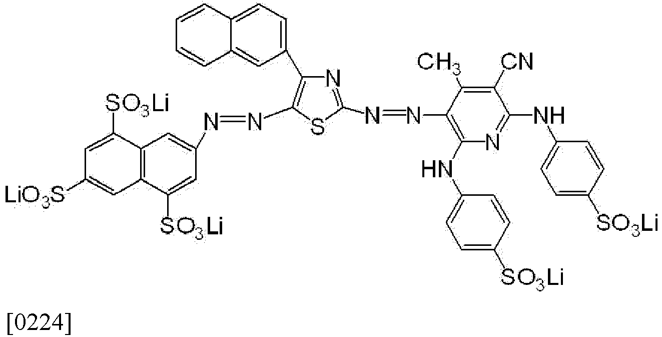

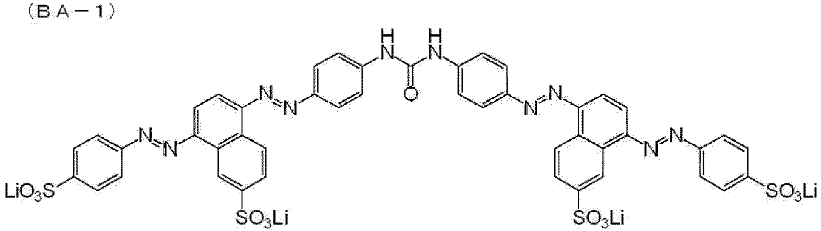

### -Measurement of Alkali Metal-

The cations of the yellow ink-1, magenta ink-1, cyan ink-1 and black ink-1 prepared as described above were measured by ion chromatography under the following conditions to determine the content of the alkali metals. Table 1 shows the measurement results.

### <Conditions>

Measuring device: Thermo Fisher ICS-1500 (manufactured by Thermo Fisher Scientific Inc.)
Column: Ionpac CS12A (manufactured by Thermo Fisher Scientific Inc.)

**[Table 1]**

| | IC (ppm) | | | | | | Li mass ratio (%) |
|---|---|---|---|---|---|---|---|
| | Li⁺ | Na⁺ | K⁺ | NH₄⁺ | Mg²⁺ | Ca²⁺ | |
| Yellow ink-1 | 1510 | 90 | 0 | 0 | 0 | 0 | 94% |
| Magenta ink-1 | 790 | 45 | 140 | 10 | 0 | 0 | 80% |
| Cyan ink-1 | 760 | 35 | 10 | 10 | 0 | 0 | 93% |
| Black ink-1 | 1200 | 200 | 140 | 0 | 0 | 0 | 78% |
| Total of entire ink | 4260 | 370 | 290 | 20 | 0 | 0 | 86% |

### -Test 1-

Each ink of the yellow ink-1, the magenta ink-1, the cyan ink-1, and the black ink-1 prepared as described above were prepared, and the yellow ink-1, the magenta ink-1, the cyan ink-1, and the black ink-1 were mixed at a mass ratio of 1:1:1:1 to obtain a mixed ink. Using each of four kinds of inks and the mixed ink, evaluation was performed by the following method. The evaluation results are shown in Tables 4 and 5 below.

### (1) Viscosity Increase Rate When Mixing Inks

Five 50 ml gas bottles were prepared, 30 g of each of the four kinds of inks and the mixed ink were put in separate gas bottles, and the inks were left to stand for 4 days under the condition of a temperature of 45°C and a humidity of 20% RH in a state where the gas bottles were opened without caps. After the inks were left to stand, the viscosity (unit: mPa s) of each ink and the mixed ink was measured under the condition of 25°C.

The viscosity was measured using VISCOMETER TV-22 (manufactured by TOKI SANGYO CO. LTD).

The measured values of the viscosities of the four color inks after being left to stand alone were averaged, and the ratio of the viscosity of the mixed ink after being left to stand to the average value was calculated to obtain a percentage (%). The value of the percentage obtained here was defined as a viscosity increase rate (%) of the viscosity of the ink which increased in a case where a plurality of kinds of inks were mixed. It is considered that as the viscosity increase rate increases, fine precipitates are more easily formed from the mixed ink during drying and concentration, and the viscosity increases.

### (2) Ink Stain

The prepared ink was set on Frontier (registered trademark) DE100 manufactured by Fujifilm Corporation, and the equivalent of 5000 sheets of KG size was output under a low temperature and low humidity condition of a temperature of 10°C and a humidity of 20% RH. Then, ink stains attached to the back surface and the edge portion (side surface) with a thickness width of the output paper were visually evaluated according to the following evaluation standards.

### <Evaluation Standards>

A: There is no stain at all.
B: In a case of close view, there is a slight stain on a very small portion, but it does not cause any trouble.
C: Slightly dirty, but within a practically acceptable range.
D: Stains are observed and not within a practically acceptable range.
E: Extremely dirty.

### -Tests 2 to 24-

Yellow inks-2 to 24 were prepared in the same manner as in the preparation of the yellow ink-1 in Test 1 except that the kind of the dye or the kind or content of the solvent in the yellow ink-1 was changed as shown in Tables 2 and 3, and the viscosity increase rate (%) when mixing the inks and ink stains were evaluated in the same manner as in Test 1 except that the yellow ink-1 was replaced with any of the yellow inks-2 to 24. The evaluation results are shown in Tables 4 and 5.

The notation "-" in each table indicates that the component is not contained.

The cations of the prepared yellow inks-2 to 24 were measured in the same manner as above, and the ratio of the mass of lithium with respect to the total mass of the cations was determined.

**[Table 2]**

| | | Yellow ink-1 | Yellow ink-2 | Yellow ink-3 | Yellow ink-4 | Yellow ink-5 | Yellow ink-6 | Yellow ink-7 | Yellow ink-8 | Yellow ink-9 | Yellow ink-10 | Yellow ink-11 | Yellow ink-12 |
|---|---|---|---|---|---|---|---|---|---|---|---|---|---|
| Dye | YELLOW-1 | 4.5 | - | 4.1 | 4.3 | - | - | 4.5 | 4.5 | 4.5 | 4.5 | 4.5 | 4.5 |
| | YELLOW-2 | - | 5.2 | 0.5 | 0.2 | - | - | - | - | - | - | - | - |
| | YELLOW-4 | - | - | - | - | 4.5 | - | - | - | - | - | - | - |
| | YELLOW-5 | - | - | - | - | - | 5.2 | - | - | - | - | - | - |
| Comparative solvent | TEGmBE (21) | - | - | - | - | - | - | - | 2 | - | - | - | - |
| Solvent A | DEGmBE (22) | - | - | - | - | - | - | - | - | 2 | 7 | - | - |
| | 1,2-hexanediol (24) | - | - | - | - | - | - | - | - | - | - | 3 | - |
| | 2-ethyl-1,3-hexanediol (25) | 2 | 2 | 2 | 2 | 2 | 2 | 5 | - | - | - | - | 5 |
| Comparative solvent | N-ethy-pyrrolidone (23) | - | - | - | - | - | - | - | - | - | - | - | - |
| Solvent B | Structural formula (1)-2 (24) | - | - | - | - | - | - | - | - | - | - | - | - |
| | 2-pyrrolidone (26) | 1 | 1 | 1 | 1 | 1 | 1 | 1 | 1 | 1 | 1 | 1 | 1 |
| Comparative solvent | Triethylene glycol (28) | - | - | - | - | - | - | - | - | - | - | - | - |
| Solvent C | 1,3-butanediol (30) | 25 | 25 | 25 | 25 | 25 | 25 | 22 | 25 | 25 | 17 | 24 | 22 |
| Solvent D | Glycerin (34) | 20 | 20 | 20 | 20 | 20 | 20 | 20 | 20 | 20 | 20 | 20 | 20 |

| | | Unit of above numerical value: % by mass | | | | | | | | | | | |
|---|---|---|---|---|---|---|---|---|---|---|---|---|---|
| Ratio of mass of lithium with respect to total mass of alkali metals in each ink (% by mass) | | 94% | 0% | 58% | 76% | 90% | 0% | 94% | 94% | 94% | 94% | 94% | 94% |

**[Table 3]**

| | | Yellow ink-13 | Yellow ink-14 | Yellow ink-15 | Yellow ink-16 | Yellow ink-17 | Yellow ink-18 | Yellow ink-19 | Yellow ink-20 | Yellow ink-21 | Yellow ink-22 | Yellow ink-23 | Yellow ink-24 |
|---|---|---|---|---|---|---|---|---|---|---|---|---|---|
| Dye | YELLOW-1 | 4.5 | 4.5 | 4.5 | 4.5 | 4.5 | 4.5 | 4.5 | 4.5 | 4.5 | 4.5 | 4.5 | 3.6 |
| | YELLOW-2 | - | - | - | - | - | - | - | - | - | - | - | 1.0 |
| | YELLOW-4 | - | - | - | - | - | - | - | - | - | - | - | - |
| | YELLOW-5 | - | - | - | - | - | - | - | - | - | - | - | - |
| Comparative solvent | TEGmBE (21) | - | - | - | - | - | - | - | - | - | - | - | - |
| Solvent A | DEGmBE (22) | - | - | - | - | - | - | - | - | - | - | - | - |
| | 1,2-hexanediol (24) | - | - | - | - | - | - | - | - | - | - | - | - |
| | 2-ethyl-1,3-hexanediol (25) | 2 | 2 | 2 | 2 | 5 | 2 | 2 | 2 | 2 | 2 | 2 | 2 |
| Comparative solvent | N-ethy-pyrrolidone (23) | 2 | - | - | - | - | - | - | - | - | - | - | - |
| Solvent B | Structural formula (1)-2 (24) | - | 5 | 7 | - | - | - | - | - | - | - | - | - |
| | 2-pyrrolidone (26) | - | - | - | 1 | 3 | 1 | 1 | 1 | 1 | 1 | 1 | 1 |
| Comparative solvent | Triethylene glycol | - | - | - | 25 | - | - | - | - | - | - | - | - |
| Solvent C | 1,3-butanediol (30) | 24 | 21 | 16 | - | 10 | 8 | 15 | 20 | 42 | 10 | 52 | 25 |
| Solvent D | Glycerin (34) | 20 | 20 | 20 | 20 | 30 | 32 | 30 | 25 | 3 | 35 | - | 20 |

| | | Unit of above numerical value: % by mass | | | | | | | | | | | |
|---|---|---|---|---|---|---|---|---|---|---|---|---|---|
| Ratio of mass of lithium with respect to total mass of alkali metals in each ink (% bv mass) | | 94% | 94% | 94% | 94% | 94% | 94% | 94% | 94% | 94% | 94% | 94% | 39% |

**[Table 4]**

| | | Test 1 | Test 2 | Test 3 | Test 4 | Test 5 | Test 6 | Test 7 | Test 8 | Test 9 | Test 10 | Test 11 | Test 12 |
|---|---|---|---|---|---|---|---|---|---|---|---|---|---|
| Ink set | Yellow ink-No. | 1 | 2 | 3 | 4 | 5 | 6 | 7 | 8 | 9 | 10 | 11 | 12 |
| | Magenta ink-No. | 1 | 1 | 1 | 1 | 1 | 1 | 1 | 1 | 1 | 1 | 1 | 1 |
| | Cyan ink-No. | 1 | 1 | 1 | 1 | 1 | 1 | 1 | 1 | 1 | 1 | 1 | 1 |
| | Black ink-No. | 1 | 1 | 1 | 1 | 1 | 1 | 1 | 1 | 1 | 1 | 1 | 1 |
| Ratio of mass of lithium with respect to total mass of alkali metals in entire ink (% by mass) *1 | | 86% | 22% | 72% | 80% | 85% | 20% | 86% | 86% | 86% | 86% | 86% | 86% |
| Viscosity increase rate when mixing inks (%) | | 103% | 300% | 140% | 110% | 105% | 310% | 145% | 220% | 120% | 210% | 120% | 145% |
| Ink stain | | A | E | C | B | A | E | C | D | B | D | B | C |
| Remarks | | Example | Comparative Example | Example | Example | Example | Comparative Example | Example | Comparative Example | Example | Comparative Example | Example | Example |

| | | | | | | | | | | | | | |
|---|---|---|---|---|---|---|---|---|---|---|---|---|---|
| * 1: Ratio of total mass of lithium with respect to total mass of alkali metals in case where each of yellow ink, magenta ink, cyan ink, and black ink is mixed at equal mass ratio | | | | | | | | | | | | | |

**[Table 5]**

| | | Test 13 | Test 14 | Test 15 | Test 16 | Test 17 | Test 18 | Test 19 | Test 20 | Test 21 | Test 22 | Test 23 | Test 24 |
|---|---|---|---|---|---|---|---|---|---|---|---|---|---|
| Ink set | Yellow ink-No. | 13 | 14 | 15 | 16 | 17 | 18 | 19 | 20 | 21 | 22 | 23 | 24 |
| | Magenta ink-No. | 1 | 1 | 1 | 1 | 1 | 1 | 1 | 1 | 1 | 1 | 1 | 1 |
| | Cyan ink-No. | 1 | 1 | 1 | 1 | 1 | 1 | 1 | 1 | 1 | 1 | 1 | 1 |
| | Black ink-No. | 1 | 1 | 1 | 1 | 1 | 1 | 1 | 1 | 1 | 1 | 1 | 1 |
| Ratio of mass of lithium with respect to total mass of alkali metals in entire ink (% by mass) * 1 | | 86% | 86% | 86% | 86% | 86% | 86% | 86% | 86% | 86% | 86% | 86% | 61% |
| Viscosity increase rate when mixing inks (%) | | 250% | 125% | 230% | 250% | 150% | 210% | 125% | 108% | 240% | 220% | 300% | 230% |
| Ink stain | | D | B | D | D | C | D | B | A | D | D | E | D |
| Remarks | | Comparative Example | Example | Comparative Example | Comparative Example | Example | Comparative Example | Example | Example | Comparative Example | Comparative Example | Comparative Example | Comparative Example |

| | | | | | | | | | | | | | |
|---|---|---|---|---|---|---|---|---|---|---|---|---|---|
| * 1: Ratio of total mass of lithium with respect to total mass of alkali metals in case where each of yellow ink, magenta ink, cyan ink, and black ink is mixed at equal mass ratio | | | | | | | | | | | | | |

YELLOW-1, 2, 4, and 5 in the above table show specific examples of the above-mentioned yellow dye. In addition, the numerical value in parentheses in the column of the solvent in the table represents the SP value (unit: MPa^{1/2}) measured by the method described above.

As shown in Tables 4 and 5, in the ink sets of Examples, the ratio of the total mass of lithium to the total mass of alkali metals in a case where all the inks were mixed at an equal mass ratio is 70% by mass or more. As a result, the increase rate of the viscosity (viscosity increase rate) of the ink in a case where a plurality of inks were mixed was kept low, and the ink stains particularly on the back surface and the side surface corresponding to the thickness of the recording medium were reduced as compared with Comparative Examples.

In Examples, the ratio of the total mass of lithium with respect to the total mass of alkali metals is more preferably 80% by mass or more, and even more preferably 85% by mass or more from the viewpoint of the effect of suppressing the viscosity increase rate and improving the ink stains.

On the other hand, in the ink sets of Comparative Examples, the increase rate of the viscosity of the ink in a case where the inks was mixed was remarkable, and as a result, the ink stains were also remarkable.

### (Example 2)

Magenta ink-2, magenta ink-3, cyan ink-2, cyan ink-3, black ink-2, and black ink-3 were prepared in the same manner as in the preparation of the magenta ink-1, the cyan ink-1, and the black ink-1 except that in Example 1, the kind of the dye and the kind and content of the solvent in the magenta ink-1, the cyan ink-1, and the black ink-1 were changed as shown in Table 6.

The content of the alkali metals included in each ink was determined in the same manner as in Example 1. Table 6 shows the measurement results.

**[Table 6]**

| | | Magenta ink-2 | Magenta ink-3 | Cyan ink-2 | Cyan ink-3 | Black ink-2 | Black ink-3 |
|---|---|---|---|---|---|---|---|
| Dye (% by mass) | MAGENTA-2 | 4.0 | - | - | - | - | - |
| | MAGENTA-4 | - | 4.5 | - | - | - | - |
| | CYAN-2 | - | - | 5.0 | - | - | - |
| | CYAN-4 | - | - | - | 5.5 | - | - |
| | BLACK-2 | - | - | - | - | 4.5 | - |
| | BLACK-3 | - | - | - | - | - | 5.0 |
| Ratio of mass of lithium with respect to total mass of alkali metals in each ink (% by mass) | | 75% | 0% | 90% | 0% | 75% | 0% |

In the above table, MAGENTA-2 and 4, CYAN-2 and 4, and BLACK-2 and 3 are specific examples of the above-described magenta dye, specific examples of the cyan dye, and specific examples of the black dye, respectively.

### -Tests 31 to 36-

Next, the viscosity increase rate (%) when mixing the inks and ink stains were evaluated in the same manner as in Example 1 except that the magenta ink-1 is replaced with the magenta ink-2 or the magenta ink-3, the cyan ink-1 is replaced with the cyan ink-2 or the cyan ink-3, and the black ink-1 is replaced with the black ink-2 or the black ink-3. Table 7 shows the evaluation results.

The cations were measured for the prepared magenta ink-2, magenta ink-3, cyan ink-2, cyan ink-3, black ink-2, and black ink-3 in the same manner as in Example 1, and the ratio of the mass of lithium with respect to the total mass of alkali metals was determined.

**[Table 7]**

| | | Test 31 | Test 32 | Test 33 | Test 34 | Test 35 | Test 36 |
|---|---|---|---|---|---|---|---|
| Ink set | Yellow ink-No. | 1 | 1 | 1 | 1 | 1 | 1 |
| | Magenta ink-No. | 2 | 3 | 1 | 1 | 1 | 1 |
| | Cyan ink-No. | 1 | 1 | 2 | 3 | 1 | 1 |
| | Black ink-No. | 1 | 1 | 1 | 1 | 2 | 3 |
| Ratio of mass of lithium with respect to total mass of alkali metals in entire ink (% by mass) *1 | | 83% | 35% | 83% | 33% | 84% | 20% |
| Viscosity increase rate when mixing inks (%) | | 108% | 300% | 107% | 350% | 106% | 380% |
| Ink stain | | B | E | B | E | B | E |
| Remarks | | Example | Comparative Example | Example | Comparative Example | Example | Comparative Example |

| | | | | | | | |
|---|---|---|---|---|---|---|---|
| * 1: Ratio of total mass of lithium with respect to total mass of alkali metals in case where each of yellow ink, magenta ink, cyan ink, and black ink is mixed at equal mass ratio | | | | | | | |

### As shown in Table 7, in the ink sets of Examples, the ratio of the total mass of lithium with respect to the total mass of alkali metals in a case where all the inks were mixed at the equal mass ratio was adjusted to 80% by mass or more. As a result, the increase rate of the viscosity (viscosity increase rate) of the ink in a case where a plurality of inks were mixed was suppressed, and the ink stains particularly on the back surface and the side surface corresponding to the thickness of the recording medium were reduced as compared with Comparative Examples.

In Examples, the ratio of the total mass of lithium with respect to the total mass of alkali metals is more preferably 80% by mass or more, and even more preferably 85% by mass or more from the viewpoint of the effect of suppressing the viscosity increase rate and improving the ink stains.

On the other hand, in the ink sets of Comparative Examples, the increase rate of the viscosity of the ink in a case where the inks was mixed was remarkable, and as a result, the ink stains were also remarkable.

In Test 37, the inks of the respective colors were prepared in the same manner as in Experiment 1 except that in the preparation of the yellow ink-1, the magenta ink-1, the cyan ink-1, and the black ink-1, all of sodium hydrogen carbonate and potassium hydrogen carbonate were changed to an equimolar amount to lithium hydrogen carbonate.

As a result, the mass ratio of lithium with respect to all of the alkali metals in each color ink was 80% by mass or more. Further, the viscosity increase rate in a case where the inks of the respective colors were mixed was 100%, and as the evaluation of the ink stain, the most preferable result of "A" was obtained.

### (Example 3)

Magenta ink-4 and magenta ink-5 were prepared in the same manner as in the preparation of the magenta ink-1 except that only the kind of the dye of the magenta ink-1 in Example 1 was changed as shown in Table 8. Further, the content of the alkali metals included in each ink was determined in the same manner as in Example 1. Table 8 shows the measurement results.

**[Table 8]**

| | | Magenta ink-4 | Magenta ink-5 |
|---|---|---|---|
| Dye (% by mass) | MM-2 | 4.0 | - |
| | MM-5 | - | 4.0 |
| Ratio of mass of lithium with respect to total mass of alkali metals in each ink (% by mass) | | 0% | 85% |

### -Tests 37 and 38-

Next, the viscosity increase rate (%) when mixing the inks and ink stains were evaluated in the same manner as in Example 1 except that the magenta ink-1 was changed to the magenta ink-4 or the magenta ink-5. Table 9 shows the evaluation results.

The cations were measured for the prepared magenta ink-4 and magenta ink-5 in the same manner as in Example 1, and the ratio of the mass of lithium with respect to the total mass of alkali metals was determined.

**[Table 9]**

| | | Test 37 | Test 38 |
|---|---|---|---|
| Ink set | Yellow ink-No. | 1 | 1 |
| | Magenta ink-No. | 4 | 5 |
| | Cyan ink-No. | 1 | 1 |
| | Black ink-No. | 1 | 1 |
| Ratio of mass of lithium with respect to total mass of alkali metals in entire ink (% by mass) *1 | | 33% | 90% |
| Viscosity increase rate when mixing inks (%) | | 340% | 102% |
| Ink stain | | E | A |
| Remarks | | Comparative Example | Example |

| | | | |
|---|---|---|---|
| *1: Ratio of total mass of lithium with respect to total mass of alkali metals in case where each of yellow ink, magenta ink, cyan ink, and black ink is mixed at equal mass ratio | | | |

From the above results, in a case where the ratio of the total mass of lithium with respect to the total mass of alkali metals in a case where all the inks were mixed at an equal mass ratio was adjusted to 80% by mass or more was adjusted to 80% by mass or more using the compound represented by Formula (MM), the increase rate of the viscosity (viscosity increase rate) of the ink was suppressed, and the ink stains were also reduced.

## Claims

1. An ink set comprising, at least:
a yellow ink composition containing at least one compound selected from the group consisting of a compound represented by Formula (Y-1) or (Y-2) and a salt thereof;
a magenta ink composition containing at least one compound selected from the group consisting of a compound represented by Formula (M) or (MM) and a salt thereof;
a cyan ink composition containing at least one compound selected from the group consisting of a compound represented by Formula (C) and a salt thereof; and
a black ink composition containing at least one compound selected from the group consisting of a compound represented by Formula (B) and a salt thereof,
wherein the yellow ink composition, the magenta ink composition, the cyan ink composition, and the black ink composition each contains, with respect to a total mass of each ink composition, 0.5% by mass to 5.0% by mass of a solvent A containing no nitrogen atom and having a solubility parameter value of 22 MPa^{1/2} to 26 MPa^{1/2}, 0.1% by mass to 5.0% by mass of a solvent B containing a nitrogen atom and having a solubility parameter value of 24 MPa^{1/2} to 29 MPa^{1/2}, 10% by mass to 50% by mass of a solvent C containing no nitrogen atom and having a solubility parameter value of 29 MPa^{1/2} to 31 MPa^{1/2}, 5% by mass to 30% by mass of a solvent D containing no nitrogen atom and having a solubility parameter value of 32 MPa^{1/2} to 34 MPa^{1/2}, and 30% by mass to 60% by mass of water, and
a ratio of a total mass of lithium with respect to a total mass of alkali metals included in an entire ink provided by mixing each of the yellow ink composition, the magenta ink composition, the cyan ink composition, and the black ink composition at an equal mass ratio is 70% by mass or more, 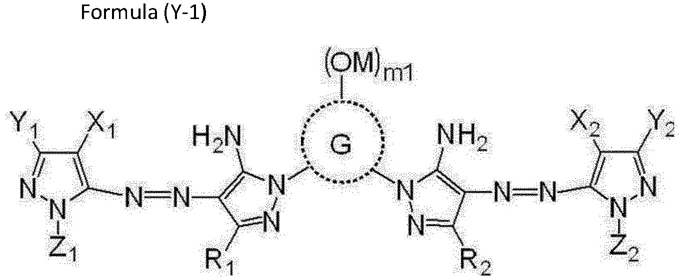
in Formula (Y-1), R₁, R₂, X₁, X₂, Y₁, Y₂, Z₁, and Z₂ each independently represent a hydrogen atom, a halogen atom, an alkyl group, an aryl group, a heterocyclic group, a cyano group, an alkoxy group, an amide group, a ureido group, an alkylsulfonylamino group, an arylsulfonylamino group, a sulfamoyl group, an alkylsulfonyl group, an arylsulfonyl group, a carbamoyl group, or an alkoxycarbonyl group, G represents an atom group forming a 5-membered to 8-membered nitrogen-containing heterocyclic ring, M represents a hydrogen atom or a cation, and m1 represents an integer of 0 to 3, 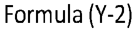 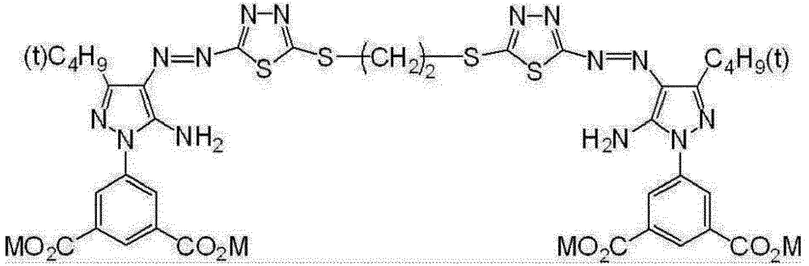
in Formula (Y-2), M's each independently represent a hydrogen atom or a cation, 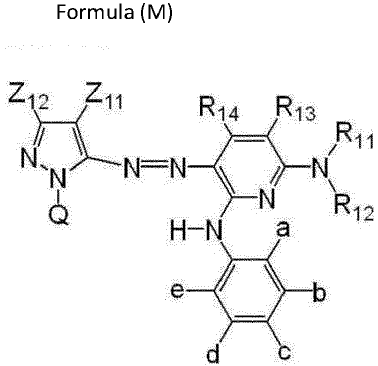
in Formula (M), Z₁₁ represents an electron-withdrawing group having a Hammett's substituent constant σₚ value of 0.2 or more, Z₁₂ represents a hydrogen atom, an alkyl group, an alkenyl group, an alkynyl group, an aralkyl group, an aromatic group, a heterocyclic group, or an acyl group, R₁₁ and R₁₂ each independently represent a hydrogen atom, an alkyl group, a cycloalkyl group, an alkenyl group, an alkynyl group, an aralkyl group, an aryl group, a heterocyclic group, an acyl group, an alkoxycarbonyl group, an aryloxycarbonyl group, a carbamoyl group, an alkylsulfonyl group, an arylsulfonyl group, or a sulfamoyl group, R₁₁ and R₁₂ do not represent a hydrogen atom at the same time, R₁₃, R₁₄, b, c, and d each independently represent a hydrogen atom, a halogen atom, an alkyl group, an alkenyl group, an alkynyl group, an aralkyl group, an aryl group, a heterocyclic group, a cyano group, a carboxy group, a carbamoyl group, an alkoxycarbonyl group, an aryloxycarbonyl group, an acyl group, a hydroxy group, an alkoxy group, an aryloxy group, a silyloxy group, an acyloxy group, a carbamoyloxy group, a heterocyclic oxy group, an alkoxycarbonyloxy group, an aryloxycarbonyloxy group, an amino group substituted with an alkyl group, an aryl group or a heterocyclic group, an acylamino group, a ureido group, a sulfamoylamino group, an alkoxycarbonylamino group, an aryloxycarbonylamino group, an alkylsulfonylamino group, an arylsulfonylamino group, a nitro group, an alkylthio group, an arylthio group, an alkylsulfonyl group, an arylsulfonyl group, an alkylsulfinyl group, an arylsulfinyl group, a sulfamoyl group, a heterocyclic thio group, or an ionic hydrophilic group, R₁₃ and R₁₁, or R₁₁ and R₁₂ may be bonded to each other to form a 5-membered or 6-membered ring, a and e each independently represent an alkyl group, an alkoxy group, or a halogen atom, and in a case where a and e both represent an alkyl group, a total carbon number of the alkyl group is 3 or more, a and b, or e and d may be bonded to each other to form a ring, Q represents a hydrogen atom, an alkyl group, an alkenyl group, an alkynyl group, an aralkyl group, an aromatic group, or a heterocyclic group, where Formula (M) has at least one ionic hydrophilic group, 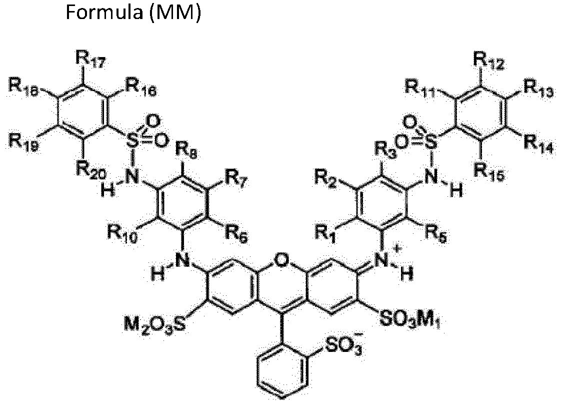
in Formula (MM), R₁, R₅, R₆, and R₁₀ each independently represent an alkyl group, R₂, R₃, R₇, R₈, R₁₁, R₁₂, R₁₃, R₁₄, R₁₅, R₁₆, R₁₇, R₁₈, R₁₉, and R₂₀ each independently represent a hydrogen atom or a substituent, M₁ and M₂ each independently represent a hydrogen atom, an alkali metal ion, or an ammonium ion, 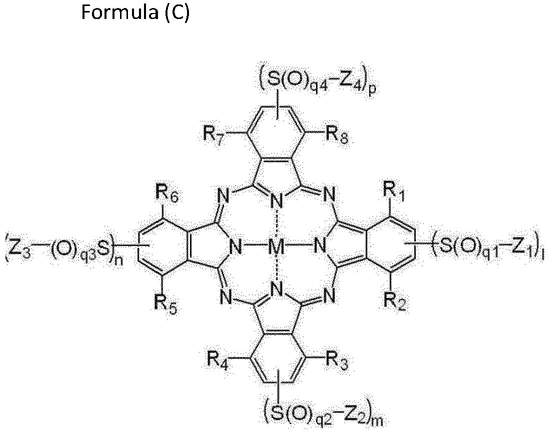
in Formula (C), R₁, R₂, R₃, R₄, R₅, R₆, R₇, and R₈ each independently represent a hydrogen atom, a halogen atom, an alkyl group, a cycloalkyl group, an alkenyl group, an aralkyl group, an aryl group, a heterocyclic group, a cyano group, a hydroxy group, a nitro group, an amino group, an alkylamino group, an alkoxy group, an aryloxy group, an amide group, an arylamino group, a ureido group, a sulfamoylamino group, an alkylthio group, an arylthio group, an alkoxycarbonylamino group, a sulfonamide group, a carbamoyl group, a sulfamoyl group, a sulfinyl group, a sulfonyl group, an alkoxycarbonyl group, a heterocyclic oxy group, an azo group, an acyloxy group, a carbamoyloxy group, a silyloxy group, an aryloxycarbonyl group, an aryloxycarbonylamino group, an imide group, a heterocyclic thio group, a phosphoryl group, an acyl group or an ionic hydrophilic group, Z₁, Z₂, Z₃, and Z₄ each independently represent an alkyl group, a cycloalkyl group, an alkenyl group, an alkynyl group, an aralkyl group, an aryl group, or a heterocyclic group, and at least one of Z₁, Z₂, Z₃, or Z₄ has an ionic hydrophilic group as a substituent, l, m, n, p, q1, q2, q3, and q4 each independently represent an integer of 1 or 2, M represents a metal atom or an oxide, hydroxide, or halide of a metal, 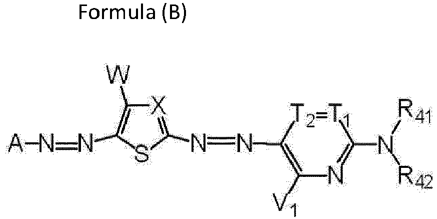
in Formula (B), A represents an aromatic group or a heterocyclic group, X represents a nitrogen atom or =C(W₁)-, and W₁ represents an electron-withdrawing group having a Hammett's substituent constant σₚ value of 0.20 or more, T₁ and T₂ each represent =C(R₄₃)- and -C(R₄₄)=, or one of T₁ and T₂ represents a nitrogen atom, and the other represents =C(R₄₃)- or -C(R₄₄)=, V₁, W, R₄₃, and R₄₄ each independently represent a hydrogen atom, a halogen atom, an alkyl group, an alkenyl group, an alkynyl group, an aralkyl group, an aryl group, a heterocyclic group, a cyano group, a carboxy group, a carbamoyl group, or an alkoxycarbonyl group, an aryloxycarbonyl group, a heterocyclic oxycarbonyl group, an acyl group, a hydroxy group, an alkoxy group, an aryloxy group, a heterocyclic oxy group, a silyloxy group, an acyloxy group, a carbamoyloxy group, an alkoxycarbonyloxy group, an aryloxycarbonyloxy group, an amino group (including an alkylamino group, an arylamino group, and a heterocyclic amino group), an acylamino group, a ureido group, a sulfamoylamino group, an alkoxycarbonylamino group, an aryloxycarbonylamino group, an alkylsulfonylamino group, an arylsulfonylamino group, a heterocyclic sulfonylamino group, a nitro group, an alkylarylthio group, an arylthio group, a heterocyclic thio group, an alkylsulfonyl group, an arylsulfonyl group, a heterocyclic sulfonyl group, an alkylsulfinyl group, an arylsulfinyl group, a heterocyclic sulfinyl group, a sulfamoyl group, or a sulfo group, R₄₁ and R₄₂ each independently represent a hydrogen atom, an alkyl group, an alkenyl group, an alkynyl group, an aralkyl group, an aryl group, a heterocyclic group, an acyl group, an alkoxycarbonyl group, an aryloxycarbonyl group, a carbamoyl group, an alkylsulfonyl group, an arylsulfonyl group, or a sulfamoyl group, R₄₁ and R₄₂ do not represent a hydrogen atom at the same time, and R₄₃ and R₄₁, or R₄₁ and R₄₂ may be bonded to each other to form a 5-membered or 6-membered ring.

2. The ink set according to claim 1,
wherein the ratio of the total mass of lithium with respect to the total mass of alkali metals included in the entire ink provided by mixing each of the yellow ink composition, the magenta ink composition, the cyan ink composition, and the black ink composition at an equal mass ratio is 80% by mass or more.

3. The ink set according to claim 1 or 2,
wherein the ratio of the total mass of lithium with respect to the total mass of alkali metals included in the entire ink provided by mixing each of the yellow ink composition, the magenta ink composition, the cyan ink composition, and the black ink composition at an equal mass ratio is 85% by mass or more.

4. The ink set according to any one of claims 1 to 3,
wherein the ratio of the mass of lithium with respect to the total mass of alkali metals in each of the yellow ink composition, the magenta ink composition, the cyan ink composition, and the black ink composition is 70% by mass or more.

5. The ink set according to any one of claims 1 to 4,
wherein the black ink composition further contains a compound represented by Formula (BA), 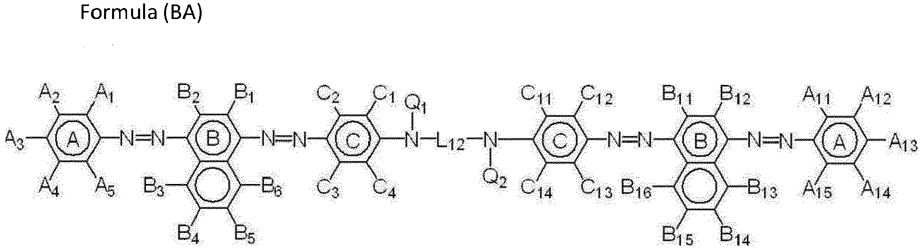
in Formula (BA), ring A, ring B, and ring C each independently represent an aryl group or a heterocyclic group, A₁, A₂, A₃, A₄, A₅, A₁₁, A₁₂, A₁₃, A₁₄, A₁₅, B₁, B₂, B₃, B₄, B₅, B₆, B₁₁, B₁₂, B₁₃, B₁₄, B₁₅, B₁₆, C₁, C₂, C₃, C₄, C₁₁, C₁₂, C₁₃, and C₁₄ each independently represent a hydrogen atom or a substituent, Q₁ and Q₂ each independently represent a hydrogen atom or a substituent, and L₁₂ represents a divalent linking group, where at least one of A₁ to A₅, An to A₁₅, B₁ to B₆, B₁₁ to B₁₆, C₁ to C₄, Cn to C₁₄, Q₁, Q₂, or L₁₂ has at least one ionic hydrophilic group.

6. The ink set according to any one of claims 1 to 5,
wherein the solvent A is 2-ethyl-1,3-hexanediol, the solvent B is 2-pyrrolidone, the solvent C is 1,3-butanediol, and the solvent D is glycerin.

7. The ink set according to any one of claims 1 to 6,
wherein a content of a surfactant in each of the yellow ink composition, the magenta ink composition, the cyan ink composition, and the black ink composition is less than 0.1% by mass with respect to the total mass of each ink composition.

8. An ink jet recording method for recording an image using the ink set according to any one of claims 1 to 7.

## Patentansprüche

1. Tintenset, mindestens umfassend:
eine gelbe Tintenzusammensetzung enthaltend mindestens eine Verbindung, ausgewählt aus der Gruppe bestehend aus einer durch die Formel (Y-1) oder (Y-2) dargestellten Verbindung und einem Salz davon;
eine magentafarbene Tintenzusammensetzung enthaltend mindestens eine Verbindung, ausgewählt aus der Gruppe bestehend aus einer durch Formel (M) oder (MM) dargestellten Verbindung und einem Salz davon;
eine cyanfarbene Tintenzusammensetzung enthaltend mindestens eine Verbindung, ausgewählt aus der Gruppe bestehend aus einer durch Formel (C) dargestellten Verbindung und einem Salz davon; und
eine schwarze Tintenzusammensetzung enthaltend mindestens eine Verbindung, ausgewählt aus der Gruppe bestehend aus einer durch Formel (B) dargestellten Verbindung und einem Salz davon,
wobei die gelbe Tintenzusammensetzung, die magentafarbene Tintenzusammensetzung, die cyanfarbene Tintenzusammensetzung und die schwarze Tintenzusammensetzung jeweils bezogen auf die Gesamtmasse jeder Tintenzusammensetzung 0,5 Masse% bis 5,0 Masse% eines kein Stickstoffatom enthaltenden Lösungsmittels A mit einem Löslichkeitsparameterwert von 22 MPa^{1/2} bis 26 MPa^{1/2}, 0,1 Masse% bis 5,0 Masse% eines ein Stickstoffatom enthaltenden Lösungsmittels B mit einem Löslichkeitsparameterwert von 24 MPa^{1/2} bis 29 MPa^{1/2}, 10 Masse% bis 50 Masse% eines kein Stickstoffatom enthaltenden Lösungsmittels C mit einem Löslichkeitsparameterwert von 29 MPa^{1/2} bis 31 MPa^{1/2}, 5 Masse% bis 30 Masse% eines kein Stickstoffatom enthaltenden Lösungsmittels D mit einem Löslichkeitsparameterwert von 32 MPa^{1/2} bis 34 MPa^{1/2} und 30 Masse% bis 60 Masse% Wasser enthält, und
ein Verhältnis einer Gesamtmasse an Lithium, bezogen auf eine Gesamtmasse an Alkalimetallen, die in einer Gesamttinte enthalten sind, die durch Mischen jeder der gelben Tintenzusammensetzung, der magentafarbenen Tintenzusammensetzung, der cyanfarbenen Tintenzusammensetzung und der schwarzen Tintenzusammensetzung in einem gleichen Masseverhältnis bereitgestellt wird, 70 Masse% oder mehr beträgt, 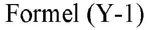 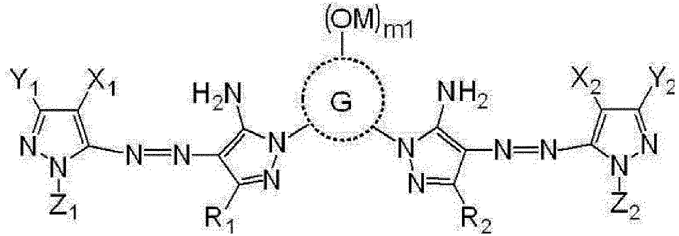
wobei in Formel (Y-1), R₁, R₂, X₁, X₂, Y₁, Y₂, Z₁ und Z₂ jeweils unabhängig ein Wasserstoffatom, ein Halogenatom, eine Alkylgruppe, eine Arylgruppe, eine heterozyklische Gruppe, eine Cyanogruppe, eine Alkoxygruppe , eine Amidogruppe, eine Ureidogruppe, eine Alkylsulfonylaminogruppe, eine Arylsulfonylaminogruppe, eine Sulfamoylgruppe, eine Alkylsulfonylgruppe, eine Arylsulfonylgruppe, eine Carbamoylgruppe oder eine Alkoxycarbonylgruppe darstellen, G eine Atomgruppe darstellt, die einen 5-gliedrigen bis 8-gliedrigen stickstoffenthaltenden heterozyklischen Ring bildet, M ein Wasserstoffatom oder ein Kation darstellt und m1 eine ganze Zahl von 0 bis 3 darstellt, 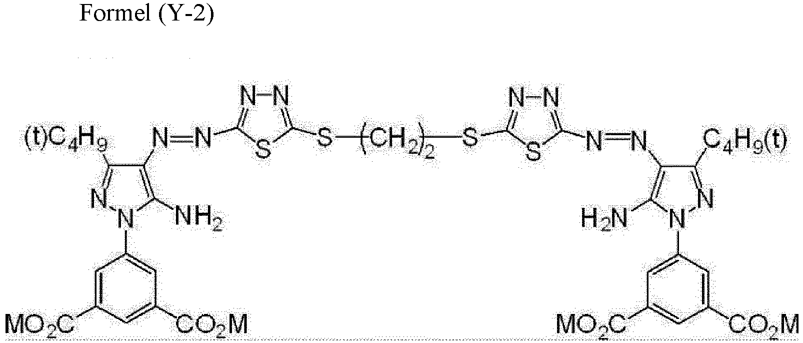
wobei in Formel (Y-2), M jeweils unabhängig ein Wasserstoffatom oder ein Kation darstellen, 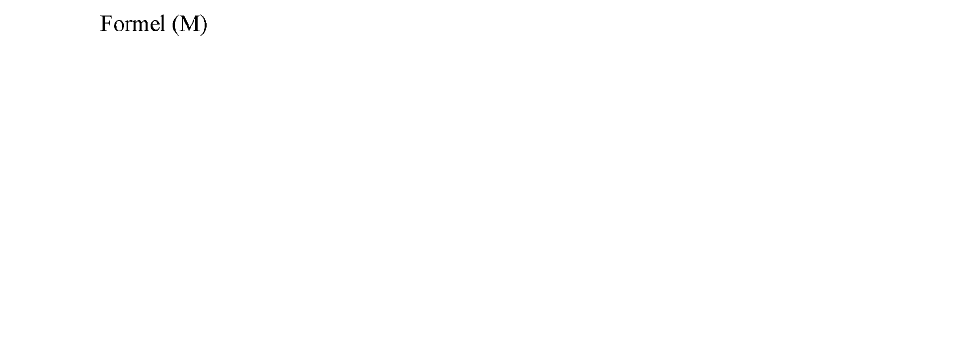 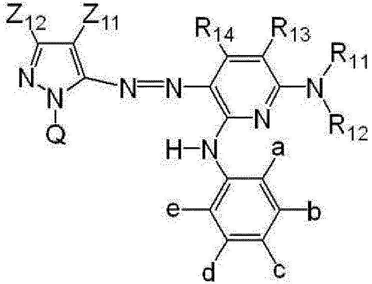
wobei in Formel (M), Z₁₁ eine Elektron-ziehende Gruppe mit einem Hammett Substituent Konstanten σₚ Wert von 0,2 oder mehr darstellt, Z₁₂ ein Wasserstoffatom, eine Alkylgruppe, eine Alkenylgruppe, eine Alkinylgruppe, eine Aralkylgruppe, eine aromatische Gruppe, eine heterozyklische Gruppe oder eine Acylgruppe darstellt, R₁₁ und R₁₂ jeweils unabhängig ein Wasserstoffatom, eine Alkylgruppe, eine Cycloalkylgruppe, eine Alkenylgruppe, eine Alkinylgruppe, eine Aralkylgruppe, eine Arylgruppe, eine heterozyklische Gruppe, eine Acylgruppe, eine Alkoxycarbonylgruppe, eine Aryloxycarbonylgruppe, eine Carbamoylgruppe, eine Alkylsulfonylgruppe, eine Arylsulfonylgruppe oder eine Sulfamoylgruppe darstellen, R₁₁ und R₁₂ gleichzeitig kein Wasserstoffatom darstellen, R₁₃, R₁₄, b, c und d jeweils unabhängig ein Wasserstoffatom, ein Halogenatom, eine Alkylgruppe, eine Alkenylgruppe, eine Alkinylgruppe, eine Aralkylgruppe, eine Arylgruppe, eine heterozyklische Gruppe, eine Cyanogruppe, eine Carboxygruppe, eine Carbamoylgruppe, eine Alkoxycarbonylgruppe, eine Aryloxycarbonylgruppe, eine Acylgruppe, eine Hydroxygruppe, eine Alkoxygruppe, eine Aryloxygruppe, eine Silyloxygruppe, eine Acyloxygruppe, eine Carbamoyloxygruppe, eine heterozyklische Oxygruppe, eine Alkoxycarbonyloxygruppe, eine Aryloxycarbonyloxygruppe, eine mit einer Alkylgruppe, einer Arylgruppe oder einer heterozyklischen Gruppe substituierten Aminogruppe, eine Acylaminogruppe, eine Ureidogruppe, eine Sulfamoylaminogruppe, eine Alkoxycarbonylaminogruppe, eine Aryloxycarbonylaminogruppe, eine Alkylsulfonylaminogruppe, eine Arylsulfonylaminogruppe, eine Nitrogruppe, eine Alkylthiogruppe, eine Arylthiogruppe, eine Alkylsulfonylgruppe, eine Arylsulfonylgruppe, eine Alkylsulfinylgruppe, eine Arylsulfinylgruppe, eine Sulfamoylgruppe, eine heterozyklische Thiogruppe oder eine ionische hydrophile Gruppe darstellen, R₁₃ und R₁₁ oder R₁₁ und R₁₂ aneinander gebunden sein können, um einen 5-gliedrigen oder 6-gliedrigen Ring zu bilden, a und e jeweils unabhängig eine Alkylgruppe, eine Alkoxygruppe oder ein Halogenatom darstellen, und in einem Fall, in dem a und e jeweils eine Alkylgruppe darstellen, eine Gesamtkohlenstoffanzahl der Alkylgruppe 3 oder mehr beträgt, a und b oder e und d jeweils aneinander gebunden sein können, um einen Ring zu bilden, Q ein Wasserstoffatom, eine Alkylgruppe, eine Alkenylgruppe, eine Alkinylgruppe, eine Aralkylgruppe, eine aromatische Gruppe oder eine heterozyklische Gruppe darstellt, wobei Formel (M) mindestens eine ionische hydrophile Gruppe aufweist, 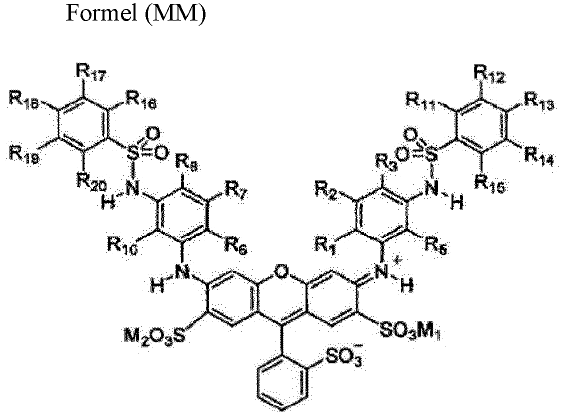
wobei in Formel (MM), R₁, R₅, R₆ und R₁₀ jeweils unabhängig eine Alkylgruppe darstellen, R₂, R₃, R₇, R₈, R₁₁, R₁₂, R₁₃, R₁₄, R₁₅, R₁₆, R₁₇, R₁₈, R₁₉ und R₂₀ jeweils unabhängig ein Wasserstoffatom oder einen Substituent darstellen, M₁ und M₂ jeweils unabhängig ein Wasserstoffatom, ein Alkalimetallion oder ein Ammoniumion darstellen, 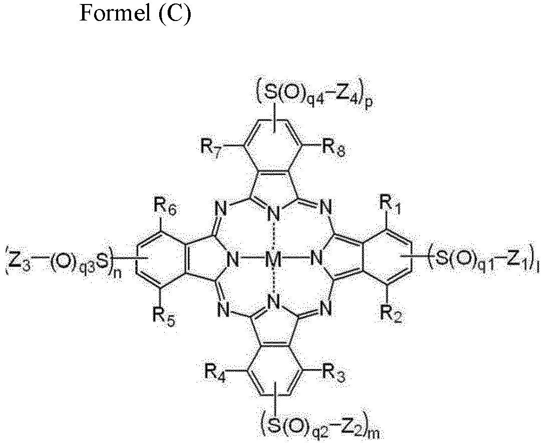
wobei in Formel (C), R₁, R₂, R₃, R₄, R₅, R₆, R₇ und R₈ jeweils unabhängig ein Wasserstoffatom, ein Halogenatom, eine Alkylgruppe, eine Cycloalkylgruppe, eine Alkenylgruppe, eine Aralkylgruppe, eine Arylgruppe, eine heterozyklische Gruppe, eine Cyanogruppe, eine Hydroxygruppe, eine Nitrogruppe, eine Aminogruppe, eine Alkylaminogruppe, eine Alkoxygruppe, eine Aryloxygruppe, eine Amidgruppe, eine Arylaminogruppe, eine Ureidogruppe, eine Sulfamoylaminogruppe, eine Alkylthiogruppe, eine Arylthiogruppe, eine Alkoxycarbonylaminogruppe, eine Sulfonamidgruppe, eine Carbamoylgruppe, eine Sulfamoylgruppe, eine Sulfinylgruppe, eine Sulfonylgruppe, eine Alkoxycarbonylgruppe, eine heterozyklische Oxygruppe, eine Azogruppe, eine Acyloxygruppe, eine Carbamoyloxygruppe, eine Silyloxygruppe, eine Aryloxycarbonylgruppe, eine Aryloxycarbonylaminogruppe, eine Imidgruppe, eine heterozyklische Thiogruppe, eine Phosphorylgruppe, eine Acylgruppe oder eine ionische hydrophile Gruppe darstellen, Z₁, Z₂, Z₃ und Z₄ jeweils unabhängig eine Alkylgruppe, eine Cycloalkylgruppe, eine Alkenylgruppe, eine Alkinylgruppe, eine Aralkylgruppe, eine Arylgruppe oder eine heterozyklische Gruppe darstellen, und mindestens eines aus Z₁, Z₂, Z₃ oder Z₄ eine ionische hydrophile Gruppe als Substituent aufweist, 1, m, n, p, q1, q2, q3 und q4 jeweils unabhängig eine ganze Zahl aus 1 oder 2 darstellen, M ein Metallatom oder ein Oxid, Hydroxid oder Halid eines Metalls darstellt, 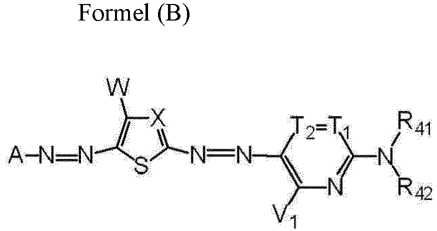
wobei in Formel (B), A eine aromatische Gruppe oder eine heterozyklische Gruppe darstellt, X ein Stickstoffatom oder =C(W₁)- darstellt und W₁ eine Elektron-ziehende Gruppe mit einem Hammett Substituent Konstante σₚ Wert von 0,20 oder mehr darstellt, T₁ und T₂ jeweils =C(R₄₃)- und -C(R₄₄)= darstellen oder eines aus T₁ und T₂ ein Stickstoffatom darstellt und der andere =C(R₄₃)- oder -C(R₄₄)= darstellt, V₁, W, R₄₃ und R₄₄ jeweils unabhängig ein Wasserstoffatom, ein Halogenatom, eine Alkylgruppe, eine Alkenylgruppe, eine Alkinylgruppe, eine Aralkylgruppe, eine Arylgruppe, eine heterozyklische Gruppe, eine Cyanogruppe, eine Carboxygruppe, eine Carbamoylgruppe oder eine Alkoxycarbonylgruppe, eine Aryloxycarbonylgruppe, eine heterozyklische Oxycarbonylgruppe, eine Acylgruppe, eine Hydroxygruppe, eine Alkoxygruppe, eine Aryloxygruppe, eine heterozyklische Oxygruppe, eine Silyloxygruppe, eine Acyloxygruppe, eine Carbamoyloxygruppe, eine Alkoxycarbonyloxygruppe, eine Aryloxycarbonyloxygruppe, eine Aminogruppe (einschließlich einer Alkylaminogruppe, eine Arylaminogruppe und einer heterozyklischen Aminogruppe), eine Acylaminogruppe, eine Ureidogruppe, eine Sulfamoylaminogruppe, eine Alkoxycarbonylaminogruppe, eine Aryloxycarbonylaminogruppe, eine Alkylsulfonylaminogruppe, eine Arylsulfonylaminogruppe, eine heterozyklische Sulfonylaminogruppe, eine Nitrogruppe, eine Alkylarylthiogruppe, eine Arylthiogruppe, eine heterozyklische Thiogruppe, eine Alkylsulfonylgruppe, eine Arylsulfonylgruppe, eine heterozyklische Sulfonylgruppe, eine Alkylsulfinylgruppe, eine Arylsulfinylgruppe, eine heterozyklische Sulfinylgruppe, eine Sulfamoylgruppe oder eine Sulfogruppe darstellen, R₄₁ und R₄₂ jeweils unabhängig voneinander ein Wasserstoffatom, eine Alkylgruppe, eine Alkenylgruppe, eine Alkinylgruppe, eine Aralkylgruppe, eine Arylgruppe, eine heterozyklische Gruppe, eine Acylgruppe, eine Alkoxycarbonylgruppe, eine Aryloxycarbonylgruppe, eine Carbamoylgruppe, eine Alkylsulfonylgruppe, eine Arylsulfonylgruppe oder eine Sulfamoylgruppe darstellen, wobei R₄₁ und R₄₂ nicht gleichzeitig ein Wasserstoffatom darstellen und R₄₃ und R₄₁ oder R₄₁ und R₄₂ miteinander verbunden sein können, um einen 5- oder 6-gliedrigen Ring zu bilden.

2. Tintenset nach Anspruch 1,
wobei das Verhältnis der Gesamtmasse an Lithium in Bezug auf die Gesamtmasse der Alkalimetalle, die in der Gesamttinte enthalten sind, die durch Mischen jeder der gelben Tintenzusammensetzung, der magentafarbenen Tintenzusammensetzung, der cyanfarbenen Tintenzusammensetzung und der schwarzen Tintenzusammensetzung in einem gleichen Massenverhältnis bereitgestellt wird, 80 Masse% oder mehr beträgt.

3. Tintenset nach Anspruch 1 oder 2,
wobei das Verhältnis der Gesamtmasse an Lithium in Bezug auf die Gesamtmasse der Alkalimetalle, die in der Gesamttinte enthalten sind, die durch Mischen jeder der gelben Tintenzusammensetzung, der magentafarbenen Tintenzusammensetzung, der cyanfarbenen Tintenzusammensetzung und der schwarzen Tintenzusammensetzung in einem gleichen Massenverhältnis bereitgestellt wird, 85 Masse% oder mehr beträgt.

4. Tintenset nach einem der Ansprüche 1 bis 3,
wobei das Verhältnis der Masse an Lithium in Bezug auf die Gesamtmasse der Alkalimetalle in jeder der gelben Tintenzusammensetzung, der magentafarbenen Tintenzusammensetzung, der cyanfarbenen Tintenzusammensetzung und der schwarzen Tintenzusammensetzung 70 Masse% oder mehr beträgt.

5. Tintenset nach einem der Ansprüche 1 bis 4,
wobei die schwarze Tintenzusammensetzung ferner eine Verbindung der Formel (BA) enthält, 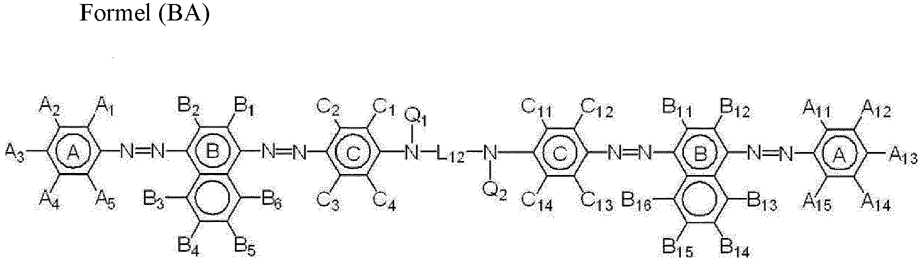
wobei in Formel (BA), Ring A, Ring B und Ring C jeweils unabhängig eine Arylgruppe oder eine heterozyklische Gruppe darstellen, A₁, A₂, A₃, A₄, A₅, A₁₁, A₁₂, A₁₃, A₁₄, A₁₅, B₁, B₂, B₃, B₄, B₅, B₆, B₁₁, B₁₂, B₁₃, B₁₄, B₁₅, B₁₆, C₁, C₂, C₃, C₄, C₁₁, C₁₂, C₁₃ und C₁₄ jeweils unabhängig ein Wasserstoffatom oder einen Substituent darstellen, Q₁ und Q₂ jeweils unabhängig ein Wasserstoffatom oder einen Substituent darstellen und L₁₂ eine divalente Verbindungsgruppe darstellt, wobei mindestens einer aus A₁ bis A₅, An bis A₁₅, B₁ bis B₆, B₁₁ bis B₁₆, C₁ bis C₄, C₁₁ bis C₁₄, Q₁, Q₂ oder L₁₂ mindestens eine ionische hydrophile Gruppe aufweist.

6. Tintenset nach einem der Ansprüche 1 bis 5,
worin das Lösungsmittel A 2-Ethyl-1,3-Hexandiol ist, das Lösungsmittel B 2-Pyrrolidon ist, das Lösungsmittel C 1,3-Butandiol ist und das Lösungsmittel D Glycerin ist.

7. Tintenset nach einem der Ansprüche 1 bis 6,
wobei ein Gehalt eines Tensids in jeder der gelben Tintenzusammensetzung, der magentafarbenen Tintenzusammensetzung, der cyanfarbenen Tintenzusammensetzung und der schwarzen Tintenzusammensetzung weniger als 0,1 Masse% in Bezug auf die Gesamtmasse jeder Tintenzusammensetzung beträgt.

8. Tintenstrahlaufzeichnungsverfahren zur Aufzeichnung eines Bildes unter Verwendung des Tintensets nach einem der Ansprüche 1 bis 7.

## Revendications

1. Jeu d'encres, comprenant au moins :
une composition d'encre jaune contenant au moins un composé sélectionné parmi le groupe consistant en un composé représenté par la formule (Y-1) ou (Y-2) et un sel de celui-ci ;
une composition d'encre magenta contenant au moins un composé sélectionné parmi le groupe consistant en un composé représenté par la formule (M) ou (MM) et un sel de celui-ci ;
une composition d'encre cyan contenant au moins un composé sélectionné parmi le groupe consistant en un composé représenté par la formule (C) et un sel de celui-ci, et
une composition d'encre noire contenant au moins un composé sélectionné parmi le groupe consistant en un composé représenté par la formule (B) et un sel de celui-ci,
dans lequel la composition d'encre jaune, la composition d'encre magenta, la composition d'encre cyan, et la composition d'encre noire contiennent chacune, par rapport à une masse totale de chaque composition d'encre, de 0,5 % en masse à 5,0 % en masse d'un solvant A ne contenant pas d'atome d'azote et présentant une valeur de paramètre de solubilité allant de 22 MPa^{1/2} à 26 MPa^{1/2}, de 0,1 % en masse à 5,0 % en masse d'un solvant B contenant un atome d'azote et présentant une valeur de paramètre de solubilité allant de 24 MPa^{1/2} à 29 MPa^{1/2}, de 10 % en masse à 50 % en masse d'un solvant C ne contenant pas d'atome d'azote et présentant une valeur de paramètre de solubilité allant de 29 MPa^{1/2} à 31 MPa^{1/2}, de 5 % en masse à 30 % en masse d'un solvant D ne contenant pas d'atome d'azote et présentant une valeur de paramètre de solubilité allant de 32 MPa^{1/2} à 34 MPa^{1/2}, et de 30 % en masse à 60 % en masse d'eau, et
un rapport entre une masse totale de lithium et une masse totale de métaux alcalins inclus dans une encre entière fournie en mélangeant chacune des composition d'encre jaune, composition d'encre magenta, composition d'encre cyan, et composition d'encre noire, pour un rapport de masse égal, est supérieur ou égal à 70 % en masse, 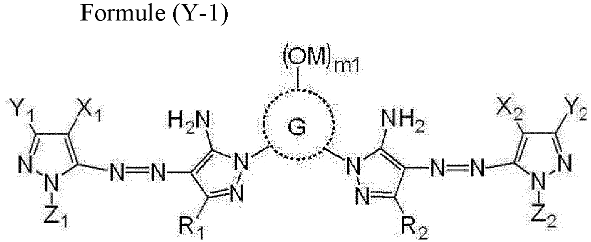
dans la Formule (Y-1), R₁, R₂, X₁, X₂, Y₁, Y₂, Z₁ et Z₂ représentent indépendamment un atome d'hydrogène, un atome d'halogène, un groupe alkyle, un groupe aryle, un groupe hétérocyclique, un groupe cyano, un groupe alkoxy, un groupe amide, un groupe uréido, un groupe alkylsulfonylamino, un groupe arylsulfonylamino, un groupe sulfamoyle, un groupe alkylsulfonyle, un groupe arylsulfonyle, un groupe carbamoyle, ou un groupe alkoxycarbonyle ; G représente un groupe d' atomes formant un noyau hétérocyclique contenant de l'azote avec de 5 chaînons à 8 chaînons ; M représente un atome d'hydrogène ou un cation, et m1 représente un entier de 0 à 3 ; 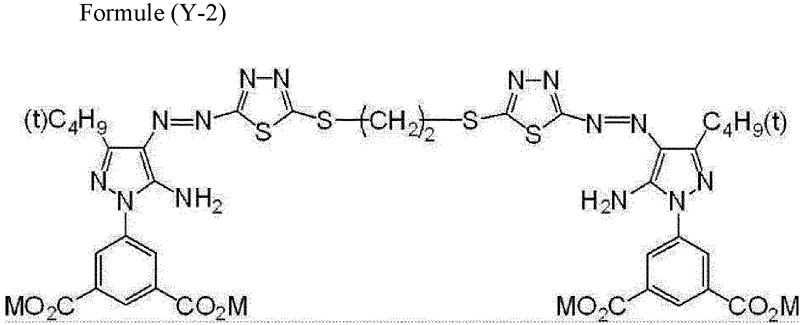
dans la Formule (Y-2), les M représentent chacun indépendamment un atome d'hydrogène ou un cation ; 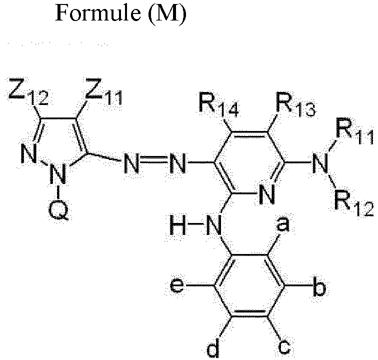
dans la Formule (M), Z₁₁ représente un groupe électroattracteur présentant une valeur de constante σp de substituant de Hammett supérieure ou égale à 0,2 ; Z₁₂ représente un atome d'hydrogène, un groupe alkyle, un groupe alcényle, un groupe alcynyle, un groupe aralkyle, un groupe aromatique, un groupe hétérocyclique, ou un groupe acyle ; R₁₁ et R₁₂ représentent chacun indépendamment un atome d'hydrogène, un groupe alkyle, un groupe cycloalkyle, un groupe alcényle, un groupe alcynyle, un groupe aralkyle, un groupe aryle, un groupe hétérocyclique, un groupe acyle, un groupe alkoxycarbonyle, un groupe aryloxycarbonyle, un groupe carbomoyle, un groupe alkylsulfonyle, un groupe arylsulfonyle ou un groupe sulfamoyle ; R₁₁ et R₁₂ ne représentent pas d'atome d'hydrogène en même temps ; R₁₃, R₁₄, b, c et d représentent chacun indépendamment un atome d'hydrogène, un atome d'halogène, un groupe alkyle, un groupe alcényle, un groupe alcynyle, un groupe aralkyle, un groupe aryle, un groupe hétérocyclique, un groupe cyano, un groupe carboxy, un groupe carbomoyle, un groupe alkoxycarbonyle, un groupe aryloxycarbonyle, un groupe acyle, un groupe hydroxy, un groupe alkoxy, un groupe aryloxy, un groupe silyloxy, un groupe acyloxy, un groupe carbamoyloxy, un groupe oxy hétérocyclique, un groupe alkoxycarbonyloxy, un groupe aryloxycarbonyloxy, un groupe amino substitué avec un groupe alkyle, un groupe aryle ou un groupe hétérocyclique, un groupe acylamino, un groupe uréido, un groupe sulfamoylamino, un groupe alkoxycarbonylamino, un groupe aryloxycarbonylamino, un groupe alkylsulfonylamino, un groupe arylsulfonylamino, un groupe nitro, un groupe alkylthio, un groupe arylthio, un groupe alkylsulfonyl, un groupe arylsulfonyle, un groupe alkylsulfinyle, un groupe arylsulfinyle, un groupe sulfamoyle, un groupe thio hétérocylique, ou un groupe hydrophile ionique ; R₁₃ et R₁₁ ou R₁₁ et R₁₂ peuvent être liés entre eux pour former un noyau à 5 chaînons ou à 6 chaînons ; a et e représentent chacun indépendamment un groupe alkyle, un groupe alkoxy, ou un atome d'halogène, et dans un cas où a et e représentent tous deux un groupe alkyle, un nombre total de carbones du groupe alkyle est supérieur ou égal à 3 ; a et b ou e et d peuvent être liés entre eux pour former un anneau ; Q représente un atome d'hydrogène, un groupe alkyle, un groupe alcényle, un groupe alcynyle, un groupe aralkyle, un groupe aromatique, ou un groupe hétérocyclique, où la Formule (M) présente au moins un groupe hydrophile ionique ; 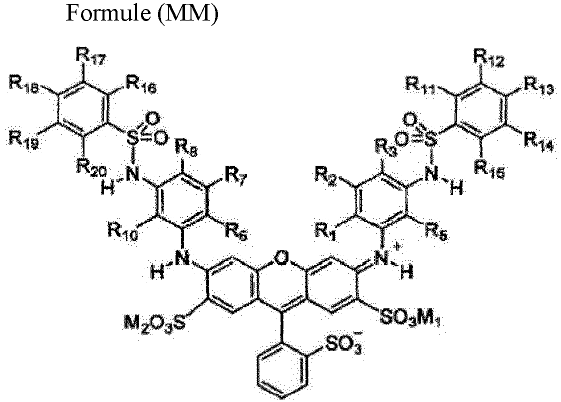
dans la Formule (MM), R₁, R₅, R₆ et R₁₀ représentent chacun indépendamment un groupe alkyle ; R₂, R₃, R₇, R₈, R₁₁, R₁₂, R₁₃, R₁₄, R₁₅, R₁₆, R₁₇, R₁₈, R₁₉ et R₂₀ représentent chacun indépendamment un atome d'hydrogène ou un substituant; M₁ et M₂ représentent chacun indépendamment un atome d'hydrogène, un ion métallique alcalin, ou un ion ammonium ; 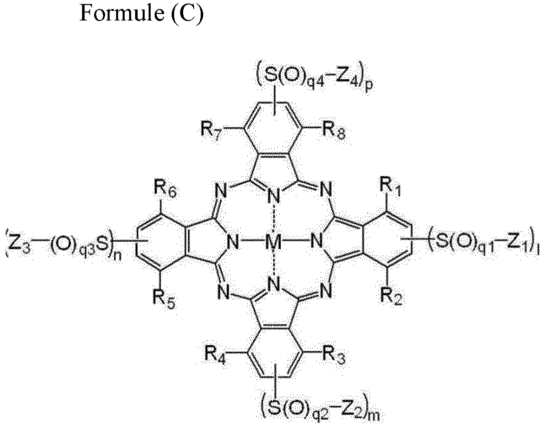
dans la Formule (C), R₁, R₂, R₃, R₄, R₅, R₆, R₇ et R₈ représentent chacun indépendamment un atome d'hydrogène, un atome d'halogène, un groupe alkyle, un group cycloalkyle, un groupe alcényle, un groupe aralkyle, un groupe aryle, un groupe hétérocyclique, un groupe cyano, un groupe hydroxy, un groupe nitro, un groupe amino, un groupe alkylamino, un groupe alkoxy, un groupe aryloxy, un groupe amide, un groupe arylamino, un groupe uréido, un groupe sulfamoylamino, un groupe alkylthio, un groupe arylthio, un groupe alkoxycarbonylamino, un groupe sulfonamide, un groupe carbamoyle, un groupe sulfamoyle, un groupe sulfinyle, un groupe sulfonyle, un groupe alkoxycarbonyle, un groupe oxy hétérocyclique, un groupe azo, un groupe acyloxy, un groupe carbamoyloxy, un groupe silyloxy, un groupe aryloxycarbonyle, un groupe aryloxycarbonylamino, un groupe imide, un groupe thio hétérocyclique, un groupe phosphoryle, un groupe acyle ou un groupe hydrophile ionique ; Z₁, Z₂, Z₃ et Z₄ représentent chacun indépendamment un groupe alkyle, un group cycloalkyle, un groupe alcényle, un groupe alcynyle, un groupe aralkyle, un groupe aryle, un groupe hétérocyclique, et au moins un élément parmi Z₁, Z₂, Z₃ ou Z₄ présente un groupe hydrophile ionique comme substituant; 1, m, n, p, q1, q2, q3 et q4 représentent chacun indépendamment un entier égal à 1 ou 2 ; M représente un ion métal ou un oxyde, un hydroxyde, ou un halogénure d'un métal ; 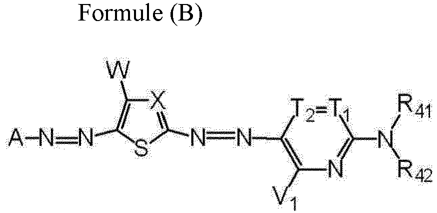
dans la Formule (B), A représente un groupe aromatique ou un groupe hétérocyclique ; X représente un atome d'azote ou =C(W₁)-, et W₁ représente un groupe électroattracteur présentant une valeur de constante de substituant de Hammett σp supérieure ou égale à 0,20 ; T₁ et T₂ représentent chacun =C(R₄₃) et -C(R₄₄)=, ou un parmi T₁ et T₂ représente un atome d'azote, et l'autre représente =C(R₄₃) ou-C(R₄₄)= ; V₁, W, R₄₃ et R₄₄ représentent chacun un atome d'hydrogène, un atome d'halogène, un groupe alkyle, un groupe alcényle, un groupe alcynyle, un groupe aralkyle, un groupe aryle, un groupe hétérocyclique, un groupe cyano, un groupe carboxy, un groupe carbomoyle, ou un groupe alkoxycarbonyle, un groupe aryloxycarbonyle, un groupe oxycarbonyle hétérocyclique, un groupe acyle, un groupe hydroxy, un groupe alkoxy, un groupe aryloxy, un groupe oxy hétérocyclique, un groupe silyloxy, un groupe acyloxy, un groupe carbamoyloxy, un groupe alkoxycarbonyloxy, un groupe aryloxycarbonyloxy, un groupe amino (incluant un groupe alkylamino, un groupe arylamino, et un groupe amino hétérocyclique), un groupe acylamino, un groupe uréido, un groupe sulfamoylamino, un groupe alkoxycarbonylamino, un groupe aryloxycarbonylamino, un groupe alkylsulfonylamino, un groupe arylsulfonylamino, un groupe sulfonylamino hétérocyclique, un groupe nitro, un groupe alkylarylthio, un groupe arylthio, un groupe thio hétérocyclique, un groupe alkylsulfonyle, un groupe arylsulfonyle, un groupe sulfonyle hétérocyclique, un groupe alkylsulfinyle, un groupe arylsulfinyle, un groupe sulfinyle hétérocyclique, un groupe sulfamoyle, ou un groupe sulfo ; R₄₁ et R₄₂ représentent chacun indépendamment un atome d'hydrogène, un groupe alkyle, un groupe alcényle, un groupe alcynyle, un groupe aralkyle, un groupe aryle, un groupe hétérocyclique, un groupe acyle, un groupe alkoxycarbonyle, un groupe aryloxycarbonyle, un groupe carbamoyle, un groupe alkylsulfonyle, un groupe arylsulfonyle, un groupe sulfamoyle ; R₄₁ et R₄₂ ne représentent pas d'atome d'hydrogène en même temps, et R₄₃ et R₄₁ ou R₄₁ et R₄₂ peuvent être liés entre eux pour former un noyau à 5 chaînons ou à 6 chaînons.

2. Jeu d'encres selon la revendication 1,
dans lequel le rapport entre la masse totale de lithium et la masse totale de métaux alcalins inclus dans une encre entière fournie en mélangeant chacune des composition d'encre jaune, composition d'encre magenta, composition d'encre cyan, et composition d'encre noire, pour un rapport de masse égal, est supérieur ou égal à 80 % en masse.

3. Jeu d'encres selon la revendication 1 ou 2,
dans lequel le rapport entre la masse totale de lithium et la masse totale de métaux alcalins inclus dans une encre entière fournie en mélangeant chacune des composition d'encre jaune, composition d'encre magenta, composition d'encre cyan, et composition d'encre noire, pour un rapport de masse égal, est supérieur ou égal à 85 % en masse.

4. Jeu d'encres selon l'une quelconque des revendications 1 à 3,
dans lequel le rapport entre la masse de lithium et la masse totale de métaux alcalins dans chacune des composition d'encre jaune, composition d'encre magenta, composition d'encre cyan, et composition d'encre noire, pour un rapport de masse égal, est supérieur ou égal à 70 % en masse.

5. Jeu d'encres selon l'une quelconque des revendications 1 à 4,
dans lequel la composition d'encre noire contient en outre un composé représenté par la Formule (BA) ; 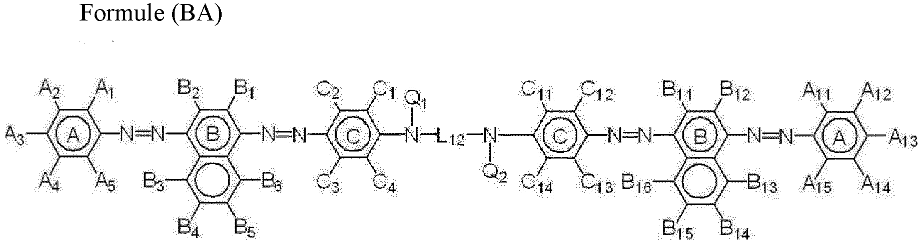
dans la Formule (BA), le noyau A, le noyau B, et le noyau C représentent chacun indépendamment un groupe aryle ou un groupe hétérocyclique ; A₁, A₂, A₃, A₄, A₅, A₁₁, A₁₂, A₁₃, A₁₄, A₁₅, B₁, B₂, B₃, B₄, B₅, B₆, B₁₁, B₁₂, B₁₃, B₁₄, B₁₅, B₁₆, C₁, C₂, C₃, C₄, C₁₁, C₁₂, C₁₃, et C₁₄ représentent chacun indépendamment un atome d'hydrogène ou un substituant ; Q₁ et Q₂ représentent chacun indépendamment un atome d'hydrogène ou un substituant, et L₁₂ représente un groupe de liaison divalent, ou au moins un parmi A₁ à A₅, A₁₁ à A₁₅, B₁ à B₆, B₁₁ à B₁₆, C₁ à C₄, C₁₁ à C₁₄, ou L₁₂ présente au moins un groupe hydrophile ionique.

6. Jeu d'encres selon l'une quelconque des revendications 1 à 5,
dans lequel le solvant A est du 2-éthyl-1,3-hexanediol ; le solvant B est de la 2-pyrrolidone ; le solvant C est du 1,3-butanediol, et le solvant D est de la glycérine.

7. Jeu d'encres selon l'une quelconque des revendications 1 à 6,
dans lequel une teneur en tensio-actif dans chacune des composition d'encre jaune, composition d'encre magenta, composition d'encre cyan, et composition d'encre noire est inférieure à 0,1 % en masse par rapport à la masse totale de chaque composition d'encre.

8. Procédé d'enregistrement à jet d'encre destiné à enregistrer une image à l'aide du jeu d'encres selon l'une quelconque des revendications 1 à 7.
